# EUROPEAN PATENT SPECIFICATION

(11) **EP 2 737 066 B1**
(45) Date of publication and mention of the grant of the patent: **08.11.2017**
(21) Application number: 12778785.1
(22) Date of filing: 20.07.2012
(51) Int. Cl.: C12N 15/66, C12P 19/34

(54) **HIGH THROUGHPUT METHOD FOR ASSEMBLY AND CLONING POLYNUCLEOTIDES COMPRISING HIGHLY SIMILAR POLYNUCLEOTIDIC MODULES**
VERFAHREN MIT HOHEM DURCHSATZ ZUR MONTAGE UND KLONIERUNG VON POLYNUKLEOTIDEN MIT STARK ÄHNLICHEN POLYNUKLEOTIDMODULEN
PROCÉDÉ À HAUT DÉBIT D'ASSEMBLAGE ET DE CLONAGE DE POLYNUCLÉOTIDES COMPRENANT DES MODULES POLYNUCLÉOTIDIQUES TRÈS SIMILAIRES

(30) Priority: 29.07.2011 US 201161513471 P; 22.12.2011 US 201161579494 P
(43) Date of publication of application: 04.06.2014
(73) Proprietor: Cellectis, 75013 Paris (FR)
(72) Inventor: VALTON, Julien, F-94220 Charenton Le Pont (FR); JULLERAT, Alexandre, F-75014 Paris (FR); DUCHATEAU, Philippe, F-91210 Draveil (FR)
(74) Representative: Santarelli
(86) International application number: PCT/IB2012/001861
(87) International publication number: WO 2013/017950

(56) References cited:
- EP-A1- 1 411 122
- WO-A2-2011/072246
- US-A1- 2008 044 862
- MORBITZER ROBERT ET AL: "Assembly of custom TALE-type DNA binding domains by modular cloning", NUCLEIC ACIDS RESEARCH, vol. 39, no. 13, 18 March 2011 (2011-03-18), pages 5790-5799, XP002689132,
- ENGLER C ET AL: "Golden Gate Shuffling: A One-Pot DNA Shuffling Method Based on Type IIs Restriction Enzymes", PLOS ONE, PUBLIC LIBRARY OF SCIENCE, SAN FRANCISCO, CA; US, vol. 4, no. 5, 14 May 2009 (2009-05-14), pages e5553-1, XP002613222, ISSN: 1932-6203, DOI: 10.1371/JOURNAL.PONE.0005553
- MICHELLE CHRISTIAN ET AL: "Targeting DNA Double-Strand Breaks with TAL Effector Nucleases", GENETICS, GENETICS SOCIETY OF AMERICA, AUSTIN, TX, US, vol. 186, no. 2, 1 October 2010 (2010-10-01), pages 757-761, XP002632806, ISSN: 0016-6731, DOI: 10.1534/GENETICS.110.120717 [retrieved on 2010-07-26]
- DIRK HOCKEMEYER ET AL: "Genetic engineering of human pluripotent cells using TALE nucleases", NATURE BIOTECHNOLOGY, vol. 29, no. 8, 7 July 2011 (2011-07-07), pages 731-734, XP55018244, ISSN: 1087-0156, DOI: 10.1038/nbt.1927

## Description

### Field of the Invention

The present invention relates to a method for the assembly and cloning of polynucleotides comprising highly similar polynucleotidic modules, that is highly versatile, does not require intermediate amplification step and can be easily automated for high throughput production of customized polynucleotidic modules.

### Background of the Invention

Recent developments of methods for efficient gene targeting and site specific gene editing have unveiled exciting perspectives for gene therapies (Silva, Poirot et al. 2011). A key requirement of such methods is the ability to produce highly specific and active customized nuclease for unique genome target of interest. Since the past ten years, a lot of efforts have been made to develop customized nucleases with tailored DNA specificity (Carroll 2008; Silva, Poirot et al. 2011; Stoddard 2011; Urnov, Rebar et al. 2011). To date, the majority of customized nucleases used for genome editing are Meganucleases and Zinc finger nucleases. They have been successfully used in edition of numerous targets of interest (Silva, Poirot et al. 2011; Urnov, Rebar et al. 2011). Despite their great promise in the field of genome engineering, a more widespread adoption is still partly hampered by relatively long and/or costly engineering processes.

Engineering such molecule is not straightforward because of the strong context dependency affecting individual protein/base interaction patterns within the DNA binding interface of meganucleases (Grizot, Duclert et al.) and Zinc Finger Nucleases (Ramirez, Foley et al. 2008). Nevertheless, studies describing the making of tailored Zinc Finger proteins and Meganucleases with chosen specificities have been a major contribution to the field of protein engineering. In addition, the impact of these studies reaches far beyond the making of rare cutting endonucleases. Indeed, whereas Zinc Finger Nucleases result from the fusion of a Zinc Finger-based DNA binding domain with the catalytic domain of the bacterial FokI TypeIIS restriction enzyme (Kim, Cha et al. 1996; Smith, Berg et al. 1999; Smith, Bibikova et al. 2000), artifical Zinc Finger proteins have also been used in fusion with other effector domains: transcription activators or inhibitors have been tethered to Zinc Finger domains to activate or repress chosen genes (Choo, Sanchez-Garcia et al. 1994; Isalan, Klug et al. 2001; Pabo, Peisach et al. 2001), and fusions comprising recombinase (Gersbach, Gaj et al. 2010) or transposase domains (Feng, Bednarz et al. 2010) have also been described. With many meganucleases, and especially the meganucleases of the LAGLIDADG family (which have been the ones used in most genome engineering experiments), the catalytic core is embedded into the DNA binding domain (Stoddard 2005; Stoddard 2011). However, the catalytic activity can be inactivated with little impact on DNA binding (Chevalier, Sussman et al. 2004), and one can easily envision fusions between such catalytically inactive mutants and a new effector domain. Thus, developing faster processes to produce new DNA binding domains with chosen specificities (with a potential for automation and scale up) would benefit not only the potential users of rare cutting endonucleases, but also any application requiring to bring a chosen peptide next to a chosen DNA sequence in a living cell.

Transcription activator-like effectors (TALEs), a group of bacterial plant pathogen proteins have recently emerged as new engineerable scaffolds for the making of tailored DNA binding domains with chosen specificities (Boch, Scholze et al. 2009; Moscou and Bogdanove 2009; Boch and Bonas 2010; Christian, Cermak et al. 2010; Li, Huang et al. 2011; Li, Huang et al. 2011). TALE DNA binding domain is composed by a variable number of 33-35 aa repeat modules. These repeat modules are nearly identical to each other except for two variable amino acids located at positions 12 and 13 (Repeat Variable Di residues, RVD). The nature of residues 12 and 13 determines base preferences of individual repeat module. Moscou M. J and Bogdanove A. J and Boch et al. (Boch, Scholze et al. 2009; Moscou and Bogdanove 2009) described the preferential pairing between A, C, G, T and repeat modules harboring respectively NI, HD, NN, and NG at positions 12 and 13. This remarkable simple cipher, consisting in a one-repeat-to-one-base pair code, allowed for prediction of TAL effector binding site and more importantly for construction of custom TAL effector repeat domains that could be tailored to bind DNA sequence of interest. This unprecedented feature unmasked exciting perpectives to develop new molecular tools for targeted genome editing and required development of efficient assembly methods of TALE repeat modules. TALE-derived proteins have been used to specifically activate chosen genes (Morbitzer, Elsaesser et al. 2011; Zhang, Cong et al. 2011). In addition, TALE-based DNA binding domains can also be tethered to various effectors. TALENs (Transcription activator-like effector Nucleases) are formed by fusions of the cleavage domain of FokI and a TALE DNA binding domain (Christian, Cermak et al. 2010; Miller, Tan et al. 2010; Cermak, Doyle et al. 2011; Li, Huang et al. 2011; Li, Huang et al. 2011; Mahfouz, Li et al. 2011).

A major drawback for the large scale assembly of TALE DNA binding domains is that assembly of multiple highly similar repeats is challenging with classical molecular biology methods. In addition, chemical synthesis of such TALE DNA binding domain is prohibitive for large scale production. To tackle this issue, few different research groups have recently developed and reported assembly methods of TALE DNA binding domain (Miller, Tan et al. 2010; Cermak, Doyle et al. 2011; Geissler, Scholze et al. 2011; Li, Huang et al. 2011; Li, Huang et al. 2011; Morbitzer, Elsaesser et al. 2011; Weber, Engler et al. 2011; Zhang, Cong et al. 2011). Those methods all relied on the Golden gate cloning technology that is based on the ability of Type IIS restriction endonucleases to cleave outside of their recognition sequence and produce 4 bp 5' overhang (Spear 2000; Engler, Kandzia et al. 2008). In these methods, TypeIIS recognition sites are placed at the 5' and 3' end of each DNA fragment in inverse orientation. This configuration allows for the seamless ligation of two DNA fragments that have compatible overhang sequences. In addition, as Type IIS restriction sites can be designed to have different overhang sequences, directional assembly of multiple DNA fragments is feasible. More importantly, as TypeIIS restriction sites are removed in the ligation process, restriction and ligation of multiple DNA fragments can be performed at the same time in a "one pot-one step reaction", the hallmark of the Golden gate technology.

While these recently developed assembly methods are fast, inexpensive and clearly advantageous with respect to classical molecular biology techniques, their potential is limited when faced up with high throughput automated production of TALE DNA binding domains. Indeed, they all display different limitations. First, the "one pot-one step reaction" methods described so far are unable to efficiently assemble in one step, a functional DNA binding domains for TALE nuclease. Indeed, to assemble a TALE DNA binding domain, these methods requires mutiple pre-assemblies of TALE repeat subarrays. These TALE repeat subarrays are amplified either by PCR (Zhang, Cong et al. 2011) or by Ecoli transformation (Cermak, Doyle et al. 2011; Geissler, Scholze et al. 2011; Morbitzer, Elsaesser et al. 2011), then recovered and finally coupled together to form the functional TALE DNA binding domain. These amplification steps are prone to errors and/or hard to automate because they require numerous different steps such as plating, colony picking, PCR screening and DNA isolation. Second, the "one pot-one step reaction" leading to assembly of TALE repeats subarrays requires a large number of single repeat plasmid. Indeed, each of the four single TALE repeat (NI, HD, NN, NG and NK) has to be cloned into several flanking typeIIS cleavable sequences to allow for efficient directional assembly of multiple repeats at the same time. In addition, it also requires additional "receiver" plasmids for TALE repeat subarray subcloning, transformation and amplification. All together and depending on the method considered, a large number of plasmids have to be constructed to enable assembly of competent TALE DNA binding domains (Cermak, Doyle et al. 2011; Geissler, Scholze et al. 2011; Zhang, Cong et al. 2011). Third, the Golden Gate cloning plating efficiency (i.e the total amount of positive clones obtained after E coli transformation and plating) decreases with increasing number of incorporated modules (Weber, Engler et al. 2011). Indeed, Weber & al reported that plating efficiency dramatically dropped from 30 000 to 150 positive colonies when the number of incorporated modules increased from 2 to 6 (Weber, Engler et al. 2011). This drawback hampers generation of high diversity libraries of TALE DNA binding domains.

Hence, to overcome the different drawbacks described above, we sought to develop a new method for assembly and cloning polynucleotides comprising highly similar polynucleotidic modules, such as TALE repeated modules. This assembly method is versatile, does not require intermediate amplification steps, and can be easily automated for high throughput production of customized polynucleotidic modules such as the repeated modules of TALE DNA binding domains. It consists in a sequential assembly of repeated modules on a solid phase supported by a 96 well plate format. In this method, a polynucleotide comprising the repeated module is linked to an organic moiety (biotin or digoxygenin) that binds specifically to the solid phase coated with streptavidin or digoxygenin-specific antibodies. Repeated modules are sequentially added via series of consecutive restriction and ligation steps using Type IIS restriction sites and enzymes.

This new method displays several advantages with respect to the methods recently documented in the literature.

Anchoring a DNA fragment onto a solid phase allows for easy removal of excess reactants, byproducts and enzymes and thus theoretically increases the success rate of repeated modules recovery. This method doesn't require any intermediate amplification step such as E. coli transformation/DNA isolation (Cermak, Doyle et al. 2011; Geissler, Scholze et al. 2011; Morbitzer, Elsaesser et al. 2011) and PCR amplification of TALE repeated modules (Zhang, Cong et al. 2011) and it allows for assembly of flexible amounts of repeated modules. In addition, our method requires only one construction per repeat module and one "receiver plasmid". Furthermore, this method can be used on a 96 well plate format and thus allows for simultaneous assembly of a large number of repeated modules. This feature makes it easy to automate with a 96 head pipetting robot.

Finally, this method has high success rates of products recovery and plating efficiency. The large number of positive colonies obtained could enable generation of high diversity libraries of polynucleotides repeated modules such as TALE DNA binding domains.

### Brief summary of the invention

The present invention relates to a method for the assembly and cloning of polynucleotides comprising highly similar polynucleotidic modules, that is highly versatile, does not require intermediate amplification step and can be easily automated for high throughput production of customized polynucleotides comprising an array of multiple polynucleotidic modules. The method of the present invention comprises a sequential assembly of polynucleotidic modules on a solid phase. The method of the present invention is particularly well-suited for assembly of Transcription Activator-Like Effector (TALE) DNA binding repeat modules. The method of the present invention allows to produce libraries of polynucleotides comprising highly similar polynucleotidic modules such as TALE DNA binding domains.

### Brief description of the figures

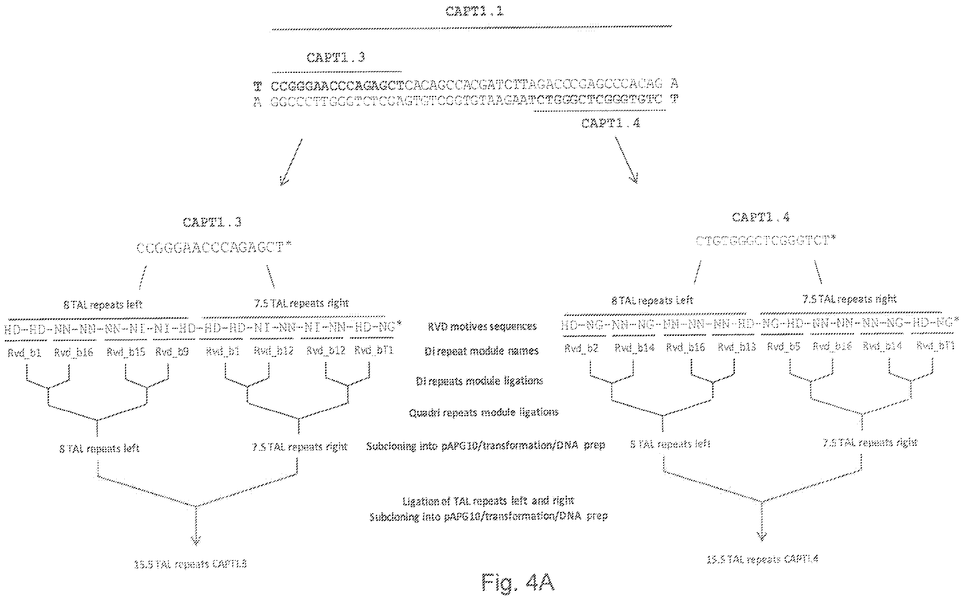

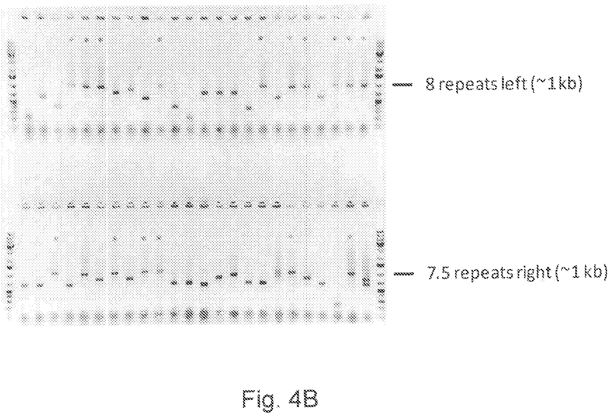

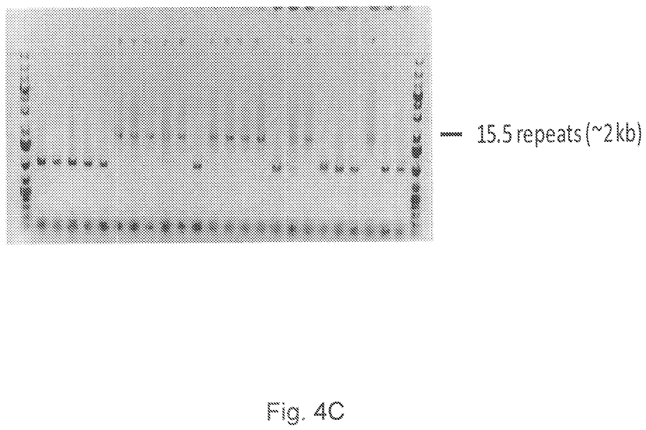

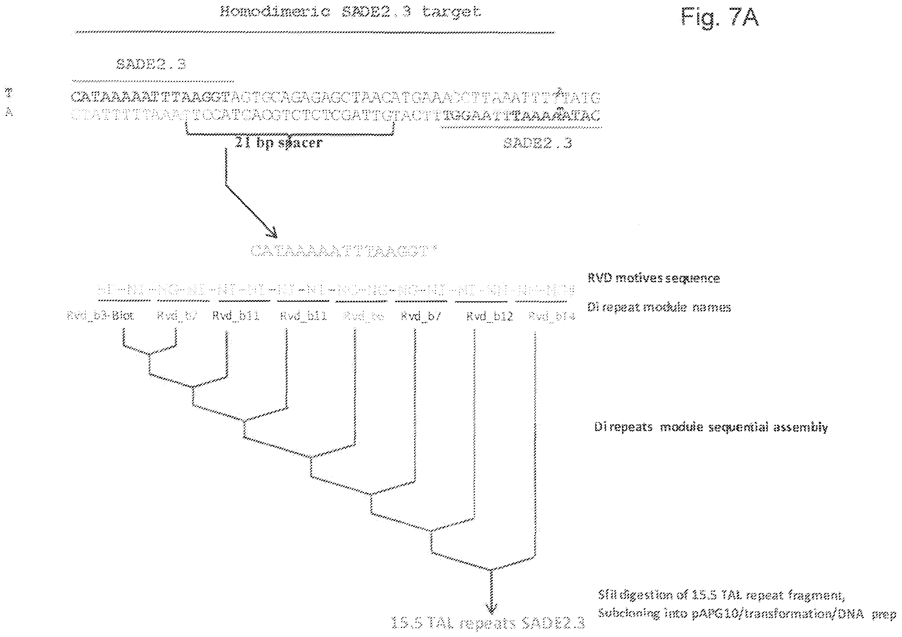

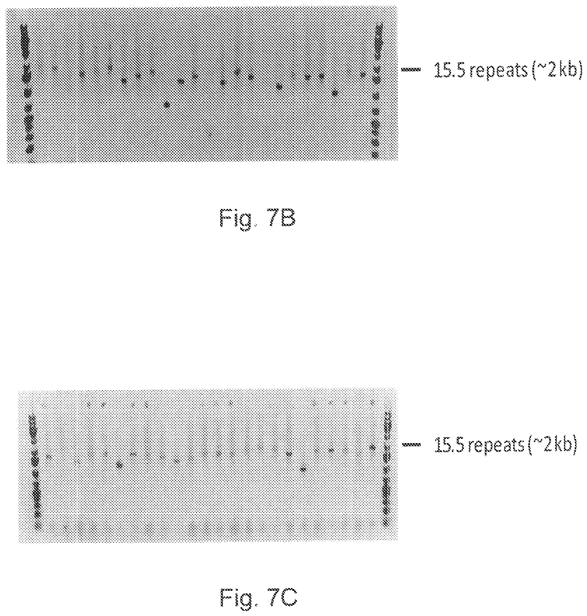

In addition to the preceding features, the invention further comprises other features that will emerge from the description and appended drawings that follow. A more complete appreciation of the invention and many of the attendant advantages thereof will be readily obtained as the same becomes better understood by reference to the following figures in conjunction with the detailed description below.
**Figure 1****:** Repeat polynucleotidic modules cloned into pAPG10 plasmid.
   Binding sites BbvI, SfaNI and SfiI are indicated in red, cyan and green respectively. Overhangs generated by the three enzymes are displayed in dashed lines with the same color code. Figure 1A: mono repeat module. Figure 1B: terminal half repeat module.
**Figure 2****:** Method to assemble two mono repeat polynucleotidic modules.
Figures 2A and 2B: extraction of polynucleotidic modules 1 and 2 from pAPG10 and digestion by SfaNI and BbvI. Figure 2C: ligation of polynucleotidic modules 1 and 2 and sublconing into pAPG10.
**Figure 3****:** Sequential assembly of 15.5 TALE repeats polynucleotidic modules using a sequential parallel process in solution and building blocks comprising di repeat polynucleotidic modules.
   For the sake of clarity, TALE repeat polynucleotidic modules used in the process are displayed with overhangs that are free and ready to react. Figure 3A: steps a-c of the process; Figure 3B: steps e-g of the process; Figure 3C: steps h-i of the process; Figure 3D: steps k-1 of the process.
**Figure 4****:** Assembly of CAPT1.3 and CAPT1.4 TALE repeats polynucleotide modules in solution.
   CAPT1.3 (SEQ ID NO: 1) and CAPT1.4 (SEQ ID NO: 2) targeted sequences and DNA spacer are displayed in green, cyan and red respectively. Figure 4A: Assembly workflow; Figure 4B: Colony PCR screen results of CAPT1.3 7.5 TALE repeats left polynucleotide assembly and 8 TALE repeats right polynucleotide assembly (steps a to g); Figure 4C: Colony PCR screen results of CAPT1.3 15.5 TALE repeats polynucleotide assembly (steps k to 1).* Correspond to clones containing 8 or 7.5 TALE repeats polynucleotides.

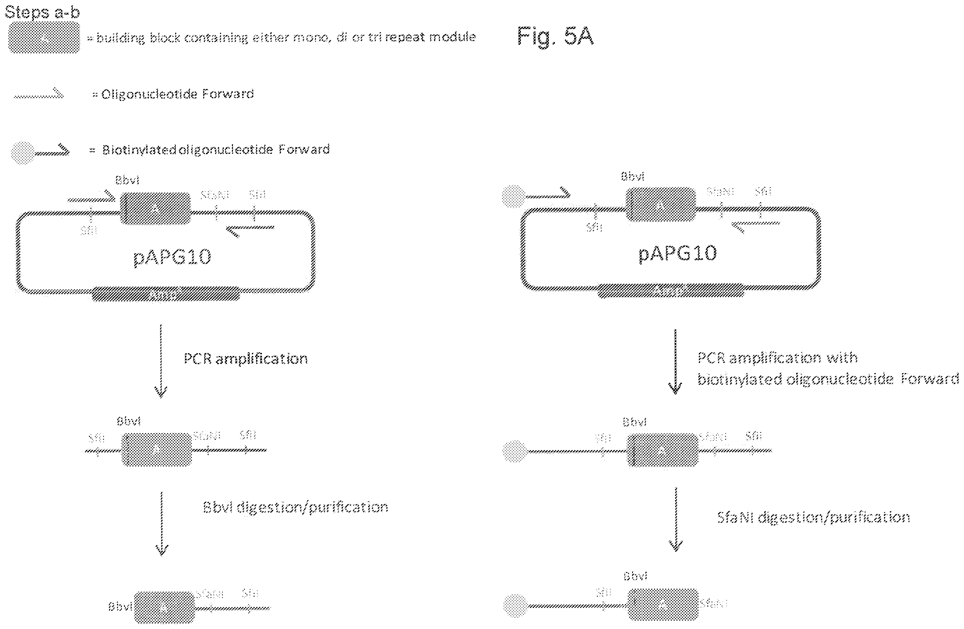

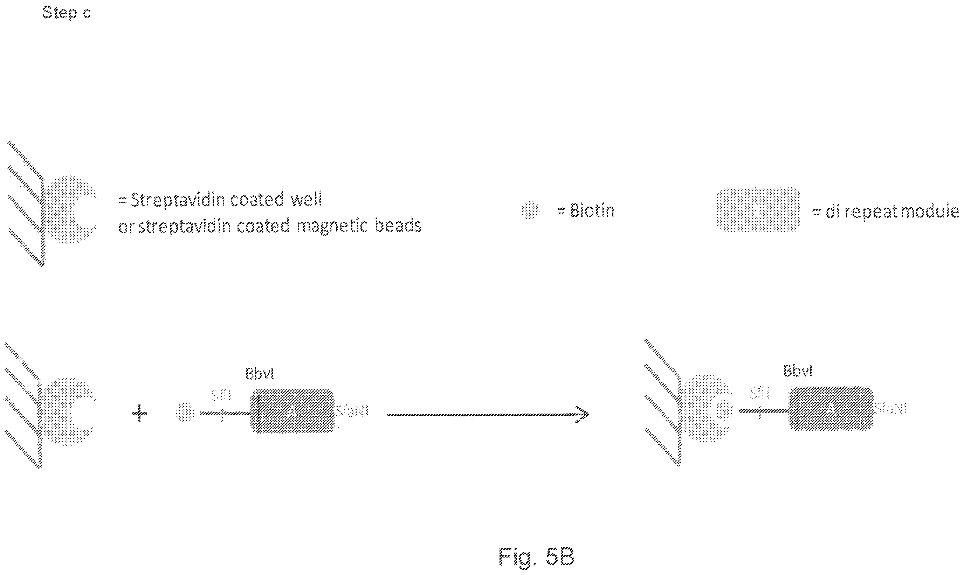

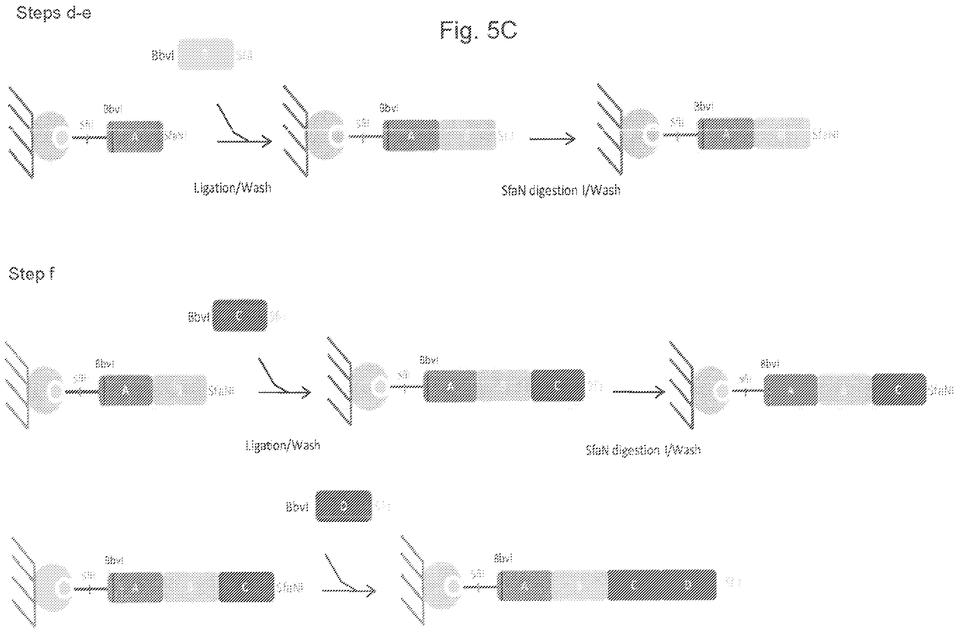

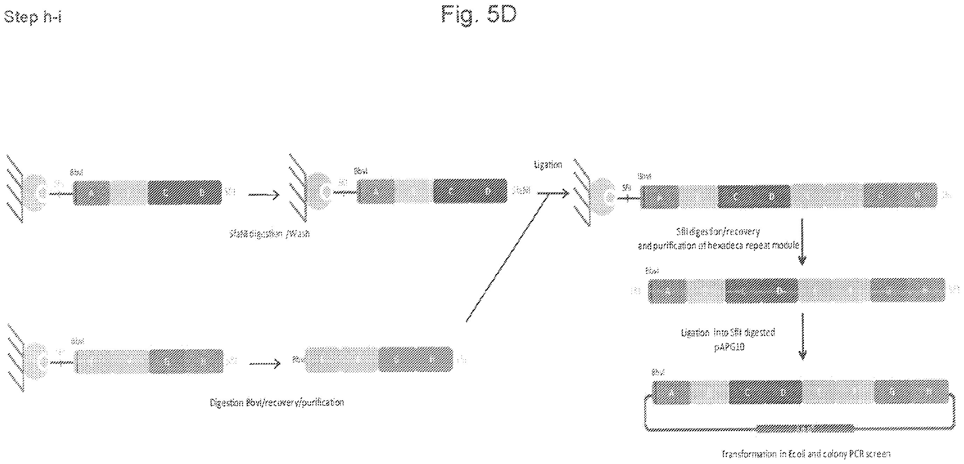

**Figure 5****:** Preparation of building blocks by PCR and BbvI or SfaNI Digestion.
   Solid phase synthesis of TALE repeat stretch using sequential parallel process. For the sake of clarity, repeat polynucleotides modules used in the process are displayed with overhangs that are free and ready to react. Figure 5A: steps a-b of the process; Figure 5B: step c of the process; Figure 5C: steps d-e of the process; Figure 5D: steps h-i of the process.

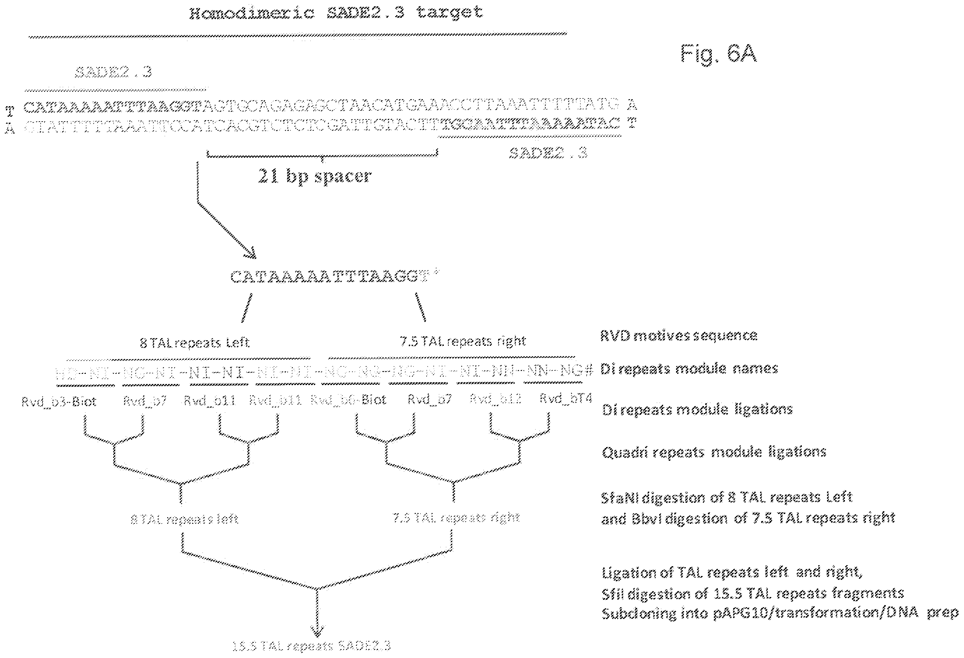

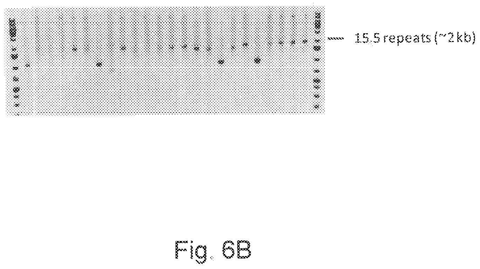

**Figure 6****:** Solid phase assembly of SADE2.3 TALE repeats polynucleotides using a sequential parallel process and building blocks comprising di repeat polynucleotidic modules.
   SADE2.3 target sequences (SEQ ID NO: 3) and DNA spacer are displayed in green and red respectively. The first biotinylated building blocks of TALE repeats left and right are displayed as "RVD_bx-biot". Figure6A: Assembly workflow; Figure6B, colony PCR screen results of SADE2.3 15.5 TALE repeats polynucleotide assembly performed with streptavidin coated magnetic beads as solid phase.* Correspond to clones containing 15.5 TALE repeats
**Figure 7****:** Solid phase assembly of SADE2.3 TALE repeats polynucleotides using a sequential parallel process.
   SADE2.3 target sequences (SEQ ID NO: 3) and DNA spacer are displayed in green and red respectively. The first biotinylated building block is marked as "RVD_b3-biot". Figure7A, Assembly workflow; Figure7B, colony PCR screen results of SADE2.3 15.5 TALE repeats polynucleotide assembly performed with streptavidin coated magnetic beads as solid phase and building blocks comprising di repeats modules; Figure7C, colony PCR screen results of SADE2.3 15.5 TALE repeats polynucleotide assembly performed with streptavidin coated well as solid phase and building blocks comprising di repeats modules.* Correspond to clones containing 15.5 TALE repeats.

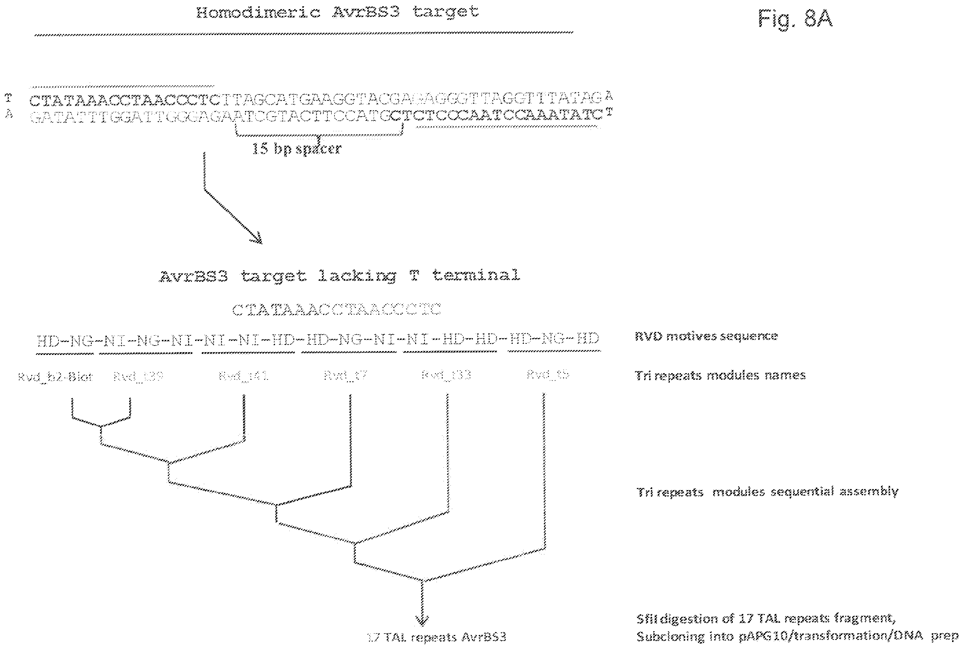

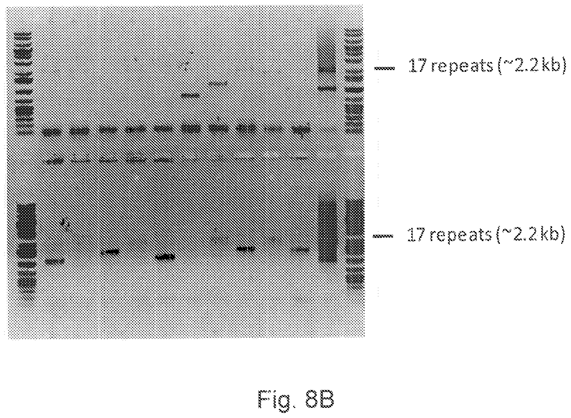

**Figure 8****:** Solid phase assembly of AvrBS3 TALE repeats polynucleotides using a sequential parallel process.
   AvrBS3 target sequence and DNA spacer are displayed in green and red respectively. The first biotinylated building block is displayed as "RVD_b2-biot". Figure8A, Assembly workflow; Figure8B, colony PCR screen results of AvrBS3 17 TALE repeats polynucleotide assembly performed with streptavidin coated well as solid phase, biotinylated first building block comprising di repeats module and additional building blocks comprising tri repeats modules.* Correspond to clones containing 17 TALE repeats.

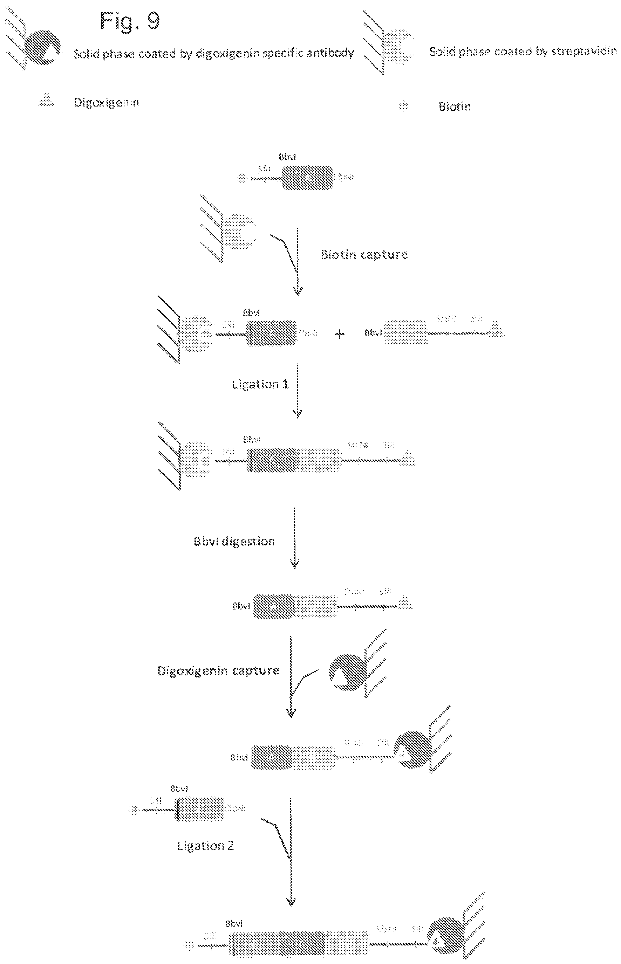

**Figure 9****:** Method for improving TALE repeat polynucleotidic modules assembly success rate.

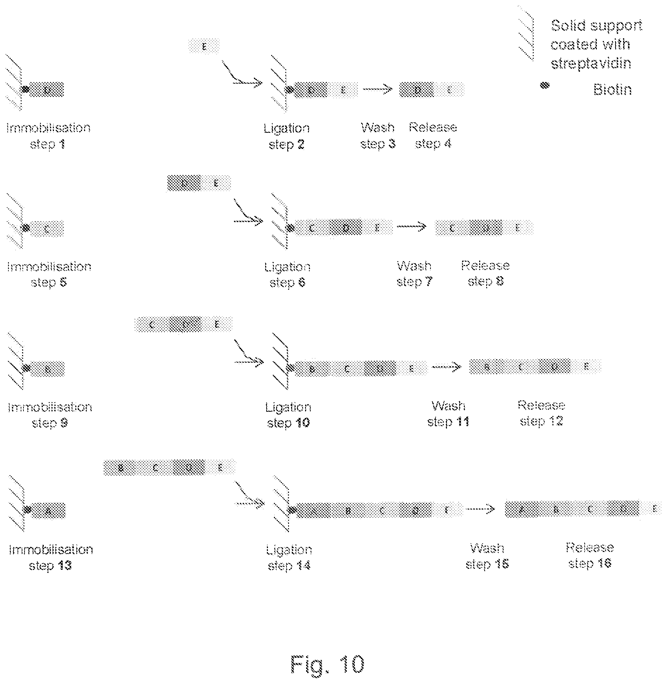

**Figure 10****:** Solid phase assembly of TALE repeat polynucleotides using the reverse elongation method.
   Workflow of steps 1-16 of the process.

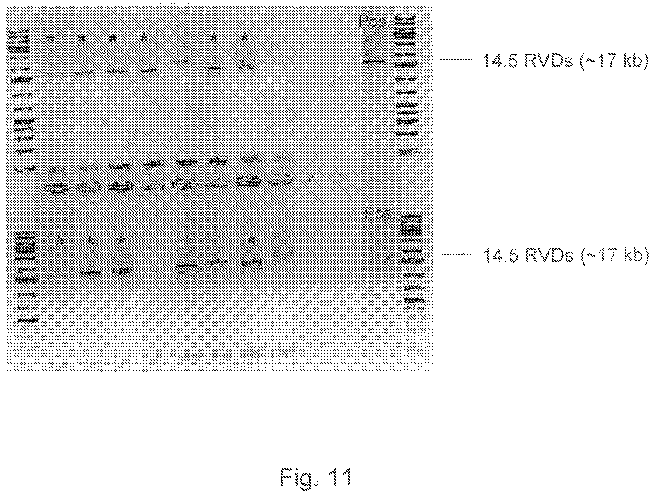

**Figure 11****:** Assembly of 14.5 TALE repeats polynucleotide modules with the reverse elongation method.
   Colony PCR screens results of a 14.5 TALE repeats polynucleotide assembly. * Correspond to clones containing 14.5 repeats polynucleotides.

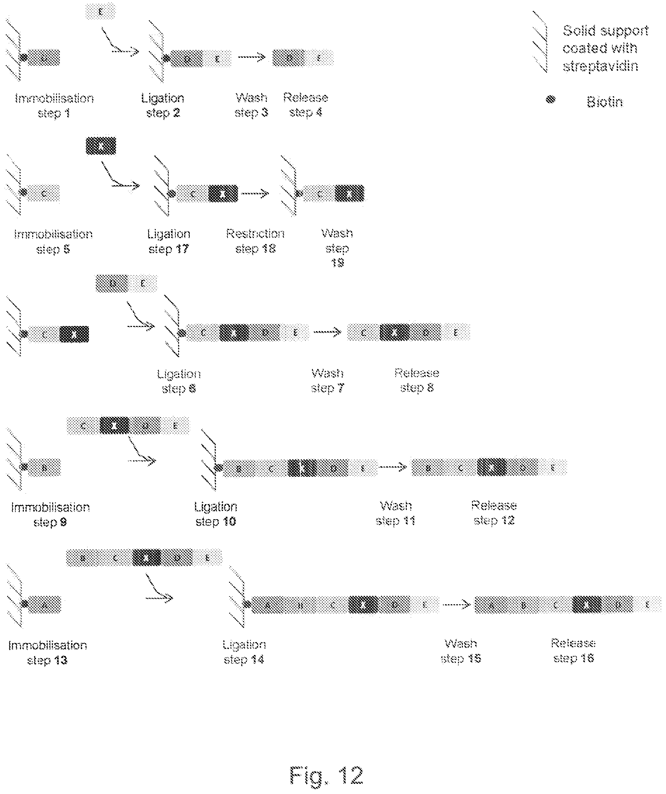

**Figure 12****:** Solid phase assembly of TALE repeat polynucleotides using the reverse elongation method combined with the sequential linear synthesis.

Workflow of steps 1-16 of the reverse elongation process and steps 17-19 of sequential linear synthesis.

The above objects highlight certain aspects of the invention. Additional objects, aspects and embodiments of the invention are found in the following detailed description of the invention.

### Detailed description of the invention

Unless specifically defined herein, all technical and scientific terms used have the same meaning as commonly understood by a skilled artisan in the fields of gene therapy, biochemistry, genetics, and molecular biology.

All methods and materials similar or equivalent to those described herein can be used in the practice or testing of the present invention, with suitable methods and materials being described herein.

In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986) and updated versions.

According to a first aspect of the present disclosure is a method of generating and assembling polynucleotides comprising arrays of at least two highly similar polynucleotidic modules comprising the steps of:
a) generating "n" polynucleotidic building blocks, with n≥1, each polynucleotidic building block comprising at least:
   - one polynucleotidic module;
   - a single cleavage site for a first restriction enzyme A, placed on one side of the polynucleotidic module;
   - a single cleavage site for a second restriction enzyme B; placed on the other side of the polynucleotidic module;
   - wherein A and B can produce compatible cohesive ends;
   - wherein cleavage of said polynucleotidic building blocks with restriction enzyme A results in a polynucleotide comprising a polynucleotidic module flanked on one side by a cohesive ends that can be re-ligated with a polynucleotide building block cleaved by restriction enzyme B without at least restoring a sequence cleavable by restriction enzyme B;
   - wherein cleavage of said polynucleotidic building blocks with restriction enzyme B results in a polynucleotide comprising a polynucleotidic module flanked on one side by a cohesive ends that can be re-ligated with a polynucleotide building block cleaved by restriction enzyme A;
b) generating at least one polynucleotidic building block comprising a single cleavage site for a restriction enzyme A', wherein A' can generate cohesive ends compatible with the cohesive ends produced by B;
c) generating at least one polynucleotide linked to a solid phase comprising:
   - one polynucleotidic module;
   - one end linked to a solid phase;
   - a free end compatible with the cohesive ends resulting from cleavage with restriction enzyme A, and which ligation with a polynucleotidic building block cleaved by restriction enzyme A will not produce at least a sequence cleavable by restriction enzyme B;
   wherein said polynucleotide comprises no sequence cleavable by restriction enzyme B;
d) cutting at least one polynucleotidic building block described in a) with restriction enzyme A;
e) ligating the resulting polynucleotidic module with the free end of the polynucleotide immobilized on the solid phase, thereby producing a new immobilized polynucleotide;
f) cutting the resulting new immobilized polynucleotide with restriction enzyme B, thus producing a new immobilized polynucleotide having a free end compatible with the cohesive ends resulting from cleavage with restriction enzyme A;
g) repeating steps d-f n times, thus producing an immobilized polynucleotide having an array of n+1 polynucleotidic modules;
h) cutting the polynucleotidic building block described in b) with restriction enzyme "A"';
i) ligating the resulting polynucleotidic module with the free end of the polynucleotide immobilized on the solid phase, thereby producing a new immobilized polynucleotide comprising an array of polynucleotidic modules.
In a preferred embodiment, A and A' are identical.

In another embodiment, at least one said polynucleotidic buiding block comprise a pre-assembly of more than one polynucleotidic module. In another embodiment, said polynucleotidic building blocks comprise a pre-assembly of more than one polynucleotidic module. As non-limiting examples, said polynucleotidic building blocks comprise a pre-assembly of 2, 3, 4, 5, 6, 7, 8, 9 or 10 polynucleotide modules. As other non-limiting examples, said polynucleotidic building blocks comprise a pre-assembly of more than 10 polynucleotide modules, i.e., 11, 12, 13, 14, 15, 20, 30, 40, 50 or 100. In another embodiment more than 100 polynucleotides are pre-assembled in said building block In another embodiment, said polynucleotidic building blocks of the present invention can be partially or entirely generated by oligonucleotide synthesis from digital nucleic sequences of said polynucleotidic building blocks and subsequent annealing of the resultant polynucleotidic intermediate; said oligonucleotides being designed and synthetize to produce polynucleotidic building blocks with compatible cohesive ends with or without enzymatic reactions.In another embodiment, some of the building blocks used for the generation of polynucleotides comprising an array of polynucleotidic modules according to the present invention can be generated by oligonucleotide synthesis from digital nucleic sequences, said oligonucleotides being designed and synthetize to produce polynucleotidic building blocks with compatible cohesive ends with or without enzymatic reactions.

In another embodiment, said single cleavage sites respectively for restriction enzymes A and B are two different cleavage sites cleavable by restriction enzymes which produce compatible cohesive overhang ends and wherein said compatible cohesive overhangs remove respective recognition sites for said restriction enzymes A and B upon ligation. In other words, restriction enzymes A and B produce at their respective single cleavage site compatible overhang cohesive ends without restoring a sequence cleavable by restriction enzymes A and B after ligation. In another embodiment, said restriction enzymes A and B belong to subtypes of class II restriction enzymes such as subtypes A, B, C, H and S as listed for example at http://_rebase.neb.com. In another embodiment, said restriction enzymes A and B belong to typeIIS restriction enzymes. In a preferred embodiment said restriction enzymes A and B are BbvI and SfaNI.

In another embodiment, said single cleavage sites respectively for restriction enzymes A and B can be cleavage sites for other enzymes such as nick-creating enzymes (nickases as non-limiting examples) under appropriate use to generate compatible overhang cohesive ends.

In another embodiment, said polynucleotide of c) is the first polynucleotide (considering a 5'-3' reading) comprising a polynucleotide module of the final polynucleotide comprising an array of polynucleotidic modules.

In another embodiment, said first polynucleotide of c) comprises a fragment of building block according to a).

In another embodiment, said polynucleotide of c) is the last polynucleotide (considering a 5'-3' reading) comprising a polynucleotide module of the final polynucleotide comprising an array of polynucleotidic modules.

In another embodiment, said last polynucleotide of c) comprises:
- A polynucleotide sequence not highly similar to a polynucleotide module according to a);
- A fragment of building block according to a).

In another embodiment, said first polypeptide of c) has been generated by:
c1) generating one polynucleotide linked to a solid phase comprising:
   - a polynucleotide sequence not highly similar to a polynucleotide module according to a); wherein said polynucleotide sequence does not comprise a cleavage site for restriction enzyme B;
   - one end linked to a solid phase;
   - a free end compatible with the cohesive ends resulting from cleavage with restriction enzyme A, and which ligation with a polynucleotidic building block cleaved by restriction enzyme A will not produce a sequence cleavable by restriction enzyme B;
c2) cutting a polynucleotidic building block described in a) with restriction enzyme A;
c3) ligating the resulting polynucleotidic module with the free end of the polynucleotide immobilized on the solid phase;
c4) cutting the resulting new immobilized polynucleotide with restriction enzyme B, thus producing a new immobilized polynucleotide comprising:
   - a polynucleotide sequence not highly similar to a polynucleotide module according to a);
   - one polynucleotidic module;
   - one end linked to a solid phase;
   - a free end compatible with the cohesive ends resulting from cleavage with restriction enzyme A, and which ligation with a polynucleotidic building block cleaved by restriction enzyme A will not produce a sequence cleavable by restriction enzyme B;

In another embodiment, the present disclosure is a method of generating and assembling polynucleotides comprising arrays of at least two highly similar polynucleotidic modules comprising the steps of:
a) generating "n" polynucleotidic building blocks, with n≥1, each polynucleotidic building block comprising at least:
   - one polynucleotidic module;
   - a single cleavage site for a first restriction enzyme A, placed on one side of the polynucleotidic module;
   - a single cleavage site for a second restriction enzyme B; placed on the other side of the polynucleotidic module;
   - wherein A and B can produce compatible cohesive ends;
   - wherein cleavage of said polynucleotidic building blocks with restriction enzyme A results in a polynucleotide comprising a polynucleotidic module flanked on one side by a cohesive ends that can be re-ligated with a polynucleotide building block cleaved by restriction enzyme B;
   - wherein cleavage of said polynucleotidic building blocks with restriction enzyme B results in a polynucleotide comprising a polynucleotidic module flanked on one side by a cohesive ends that can be re-ligated with a polynucleotide building block cleaved by restriction enzyme A, without restoring a sequence cleavable by restriction enzyme A;
b) generating at least one polynucleotidic building block comprising a single cleavage site for a restriction enzyme A', generating cohesive ends compatible with the cohesive ends produced by A;
c) generating at least one polynucleotide linked to a solid phase comprising:
   - one polynucleotidic module;
   - one end linked to a solid phase;
   - a free end compatible with the cohesive ends resulting from cleavage with restriction enzyme B, and which ligation with a polynucleotidic building block cleaved by restriction enzyme B will not produce a sequence cleavable by restriction enzyme A;
   wherein said polynucleotide comprises no sequence cleavable by restriction enzyme A;
d) cutting a polynucleotidic building block described in a) with restriction enzyme B;
e) ligating the resulting polynucleotidic module with the free end of the polynucleotide immobilized on the solid phase, thereby producing a new immobilized polynucleotide;
f) cutting the resulting new immobilized polynucleotide with restriction enzyme A, thus producing a new immobilized polynucleotide having a free end compatible with the cohesive ends resulting from cleavage with restriction enzyme B;
g) repeating steps d-f n times, thus producing an immobilized polynucleotide having an array of n+1 polynucleotidic modules;
h) cutting the polynucleotidic building block described in b) with restriction enzyme A';
i) ligating the resulting polynucleotidic module with the free end of the polynucleotide immobilized on the solid phase, thereby producing a new immobilized polynucleotide comprising an array of polynucleotidic modules.

In a preferred embodiment, A and B are identical.

In another embodiment, said polynucleotide sequence not highly similar to a polynucleotide module according to a) is linked to said solid phase. In another embodiment, said polynucleotide sequence not highly similar to a polynucleotide module according to a) encodes a N-terminal polypeptidic sequence of a TALE. In another embodiment, said polynucleotide sequence not highly similar to a polynucleotide module according to a) encodes a C-terminal polypeptidic sequence of a TALE. In another embodiment, said polynucleotide sequence not highly similar to a polynucleotide module according to a) encodes a C-terminal polypeptidic sequence of a TALE and a half Transcription Activator-like Effector (TALE) DNA binding repeat module. In another embodiment, said polynucleotide sequence not highly similar to a polynucleotide module according to a) encodes one protein domain able to process a nucleic acid target sequence adjacent to the nucleic acid sequence bound by a TALE DNA binding domain, thus producing a chimeric protein according to the method of the present invention comprising a set of repeated modules with RVDs to bind a nucleic acid sequence and one protein domain to process a nucleic acid target sequence adjacent to said bound nucleic acid sequence.

In another embodiment, said polynucleotidic building blocks can encode a polynucleotidic module that is not highly similar to the other polynucleotidic modules assembled according to the method of the present invention to produce polynucleotides comprising an array of polynucleotidic modules. As non-limiting example, said polynucleotidic building blocks can encode a protein domain able to process a nucleic acid target sequence adjacent to the nucleic acid sequence bound by the other polynucleotidic modules of the array. Position of said not highly similar module can be anywhere in the array. As a non-limiting example a polynucleotide comprising an array of polynucleotidic modules can comprise a succession of:
7,5 highly similar TALE repeat polynucleotidic modules - a not highly similar polynucleotidic module encoding a protein domain - 7,5 highly similar TALE repeat polynucleotidic modules. In another embodiment, said polynucleotide comprising an array of polynucleotidic modules according to the present invention can comprise more than one not highly similar polynucleotidic module.

In another embodiment, the end of said polynucleotide of c) linked to a solid phase comprises a single cleavage site for a restriction enzyme C, wherein said cleavage of said site with restriction enzyme C allows to unlink said polynucleotide from the solid phase. In another embodiment of this aspect of the invention, said restriction enzyme C is SfiI. In another embodiment, said single cleavage site for a restriction enzyme C is cleavable by said restriction enzyme A or said restriction enzyme B and said final polynucleotide comprising an array of polynucleotidic modules comprises no sequence cleavable by restriction enzymes A or B.

In another embodiment, said method of the present invention further comprises the step of unlinking said final polynucleotide of step i) comprising an array of polynucleotidic modules, by unlinking it with in non enzymatic step such as chemical or ionic treatments well-known in the art.

In another embodiment, said method of the present invention further comprises the step of unlinking said final polynucleotide of step i) comprising an array of polynucleotidic modules, by cutting it with a restriction enzyme. In another embodiment, said restriction enzyme is a restriction enzyme C different than restriction enzymes A and B. In another embodiment, said restriction enzyme is A or B.

In another embodiment, each of said polynucleotidic modules to assemble according to the present invention encodes a Transcription Activator-like Effector (TALE) DNA binding repeat module. Said Transcription Activator-like Effector (TALE) DNA binding repeat module usually comprises between 8 and 30 repeated modules (or repeat modules), more frequently between 8 and 20 repeat modules, again more frequently 15 repeat modules. The assembly of said repeated modules produce a TALE binding domain. Said repeat modules usually encode for 30 to 42 amino acids, more preferably 33-35 amino acids wherein two critical amino acids of each repeat module located at positions 12 and 13 (Repeat Variable Diresidues, RVD), mediate the recognition of one nucleotide of the nucleic acid target sequence targeted by the entire Transcription Activator-like Effector (TALE) DNA binding domain; said polynucleotidic modules can encode for TALE repeat modules comprising equivalent two critical amino acids located at positions other than 12 and 13 specialy in repeat modules taller than 33-35 amino acids long. In another embodiment, said polynucleotidic repeat modules of the present invention can encode for repeat modules-like domains or RVDs-like domains. RVDs-like domains have a sequence different from naturally occurring polynucleotidic repeat modules comprising RVDs (RVDs domains) but have a similar function and / or global structure. As non-limiting examples, said RVDs-like domains are protein domains selected from the group consisting of Puf RNA binding protein or Ankyrin super-family. Non-limiting examples of such proteins from which RVDs-like domain can be derived are given by SEQ ID NO: 4 and SEQ ID NO: 5 respectively corresponding to proteins fem-3 and aRep. Depending on the structural context and binding constraints, said polynucleotidic modules to assemble according to the present invention encodes a Transcription Activator-like Effector (TALE) DNA binding domain that comprises a mix of naturally occurring RVDs structures and RVDs-like domains. In another embodiment, said polynucleotidic TALE repeat modules to assemble according to the present invention encodes a totally artificial Transcription Activator-like Effector (TALE) DNA binding domain *i.e*., without any repeated domains derived from naturally occurring TAL effectors.

In another embodiment, said polynucleotidic building block of b) encodes a half repeat module of a Transcription Activator-like Effector (TALE) DNA binding repeat module. Said half repeat module is equivalent to the truncated repeat module usually made of 20 amino acids that is located at the C-terminus of said Transcription Activator-like Effector (TALE) DNA binding repeat module. In this case, said Transcription Activator-like Effector (TALE) DNA binding repeat module comprises between 8.5 and 30.5 repeat modules, ".5" referring to previously mentioned half repeat module (or terminal repeat module, or half-repeat). More frequently, said Transcription Activator-like Effector (TALE) DNA binding repeat module comprises between 8.5 and 20.5 repeat modules, again more frequently, 15.5 repeat modules.

In another embodiment, said polynucleotidic building block of b) encodes a half TALE repeat module and one TALE mono repeat module (i.e. one and half TALE repeat module). In another embodiment, said polynucleotidic building block of b) encodes a half Transcription Activator-like Effector (TALE) DNA binding repeat module and a C-terminal fragment of a TALE. In another embodiment, said building block of b) encodes a N-terminal fragment of a TALE.

In another embodiment, said polynucleotidic modules to assemble share at least 85% similarity. In another embodiment, said polynucleotidic modules to assemble share 86, 87, 88, 89 or 90% similarity. In another embodiment, said polynucleotidic modules to assemble share 91, 92, 93, 94, 95, 96, 97, 98, 99 or 100% similarity.

BLASTP may also be used to identify an amino acid sequence having at least 80%, 85%, 87.5%, 90%, 92.5%, 95%, 97.5%, 98%, 99% sequence similarity to a reference amino acid sequence using a similarity matrix such as BLOSUM45, BLOSUM62 or BLOSUM80. Unless otherwise indicated a similarity score will be based on use of BLOSUM62. When BLASTP is used, the percent similarity is based on the BLASTP positives score and the percent sequence identity is based on the BLASTP identities score. BLASTP "Identities" shows the number and fraction of total residues in the high scoring sequence pairs which are identical; and BLASTP "Positives" shows the number and fraction of residues for which the alignment scores have positive values and which are similar to each other. Amino acid sequences having these degrees of identity or similarity or any intermediate degree of identity of similarity to the amino acid sequences disclosed herein are contemplated and encompassed by this disclosure.

As previously mentioned, said polynucleotidic modules to assemble according to the present invention can encode Transcription Activator-like Effector (TALE) DNA binding repeat modules. The assembly of such polynucleotidic modules produces a TALE binding domain. Said Transcription Activator-like Effector (TALE) DNA binding domain usually comprises between 8 and 30 repeated modules (or repeat modules, or TALE repeat modules), more frequently between 8 and 20 repeat modules, again more frequently 15 repeat modules. Said repeat modules usually encode for 30 to 42 amino acids, more preferably 33-35 wherein two critical amino acids located at positions 12 and 13 (Repeat Variable Diresidues, RVD) mediate the recognition of one nucleotide of the nucleic acid target sequence targeted by the entire Transcription Activator-like Effector (TALE) DNA binding domain. Preferably, RVDs associated with recognition of the different nucleotides are HD for recognizing C, NG for recognizing T, NI for recognizing A, NN for recognizing G or A, NS for recognizing A, C, G or T, HG for recognizing T, IG for recognizing T, NK for recognizing G, HA for recognizing C, ND for recognizing C, HI for recognizing C, HN for recognizing G, NA for recognizing G, SN for recognizing G or A and YG for recognizing T. More preferably, RVDs associated with recognition of the nucleotides C, T, A, G/A and G respectively are selected from the group consisting of NN or NK for recognizing G, HD for recognizing C, NG for recognizing T and NI for recognizing A. In another embodiment, RVDs associated with recognition of the nucleotide C are selected from the group consisting of N* and RVDs associated with recognition of the nucleotide T are selected from the group consisting of N* and H*, where * denotes a gap in the repeat sequence that corresponds to a lack of amino acid residue at the second position of the RVDs. In another embodiment, critical amino acids 12 and 13 can be mutated towards other amino acid residues in order to modulate their specificity towards nucleotides A, T, C and G and in particular to enhance this specificity. By other amino acid residues is intended any of the twenty natural amino acid residues or unnatural amino acids derivatives.

In another aspect of the present invention is a method wherein said "n" polynucleotidic building blocks are part of a collection encoding polypeptidic repeated modules or polypeptidic repeat modules with Repeat Variable Dipeptide regions (RVDs) comprising a pair of amino acids responsible for recognizing one nucleotide selected from the group consisting of HD for recognizing C, NG for recognizing T, NI for recognizing A, NN for recognizing G or A, NS for recognizing A, C, G or T, HG for recognizing T, IG for recognizing T, NK for recognizing G, HA for recognizing C, ND for recognizing C, HI for recognizing C, HN for recognizing G, NA for recognizing G, SN for recognizing G or A and YG for recognizing T. In a preferred embodiment of this aspect of the invention, is a method wherein said "n" polynucleotidic building blocks are part of a collection encoding polypeptidic repeated modules with Repeat Variable Dipeptide regions (RVDs) comprising a pair of amino acids responsible for recognizing one nucleotide selected from the group consisting of HD for recognizing C, NG for recognizing T, NI for recognizing A, NN and NK for recognizing G.

In another embodiment is a method wherein said "n" polynucleotidic building blocks are part of a collection encoding two polypeptidic repeated modules with Repeat Variable Dipeptide regions (RVDs) for recognizing two nucleotides via two pairs of amino acids (TALE di repeat modules or di repeat modules) selected from the group listed in table 2 of example one below as non-limiting example.

In another embodiment is a method wherein said "n" polynucleotidic building blocks are part of a collection encoding three polypeptidic repeated modules with Repeat Variable Dipeptide regions (RVDs) for recognizing three nucleotides via three pairs of amino acids (TALE tri repeat modules or tri repeat modules) selected from the group listed in table 3 of example one as non-limiting example.

In another embodiment is a method wherein said "n" polynucleotidic building blocks are part of a library encoding "n" polypeptidic repeated modules with Repeat Variable Dipeptide regions (RVDs) for recognizing "n" nucleotides via "n" pairs of amino acids (TALE "n" repeat modules or "n" repeat modules). In another embodiment, "n" is comprised between 1 and 8. In another embodiment, "n" is 1, 2, 3, 4, 5, 6, 7 or 8.

In another embodiment, said polynucleotidic building block brought in previously described steps d) to g) of the method of the present invention is a unique molecular species comprising a precise sequence. In another embodiment, several polynucleotidic building blocks are brought in previously described steps d) to g) of the method of the present invention comprising each a precise sequence. In another embodiment, a library of polynucleotidic building blocks are brought in previously described steps d) to g) of the method of the present invention comprising a library of polynucleotidic modules. In another embodiment said library can be a random mutagenized library of polynucleotidic modules. In another embodiment, a library of polynucleotidic building blocks are used in previously described steps d) to g) of the method of the present invention comprising a library of polynucleotidic modules and encoding a library of TALE repeat polynucleotidic modules. Mutagenesis can concern the entire polynucleotidic module sequence. In another embodiment said library can be a random mutagenized library of TALE repeat polynucleotidic modules. Mutagenesis of said TALE repeat polynucleotidic modules library can focused on critical amino acids such as amino acids located at positions 12 and 13 as non-limiting examples. Mutagenesis of said TALE repeat polynucleotidic modules library can focused on other critical amino acids of said TALE repeat polynucleotidic modules. Mutagenesis of said TALE repeat polynucleotidic modules library may concern the entire TALE repeat module sequence. Said libraries of polynucleotidic modules constituting libraries of polynucleotidic building blocks can be used according to the method of the present invention to introduce diversity into polynucleotides comprising an array of polynucleotidic modules. As non-limiting example, the method according to the present invention is particularly well-suited to introduce diversity into TALE repeat polynucleotidic modules in order to produce high-diversity libraries of TALE DNA binding domains.

According to another aspect of the present disclosure is a method of generating and assembling polynucleotides comprising arrays of at least two highly similar polynucleotidic modules comprising the steps of:
a) generating at least one polynucleotidic building block comprising at least:
   - one polynucleotidic module;
   - a single cleavage site for a first restriction enzyme A, placed on one side of the polynucleotidic module;
   - a single cleavage site for a second restriction enzyme B, placed on the other side of the polynucleotidic module;
   - wherein A and B can produce compatible cohesive ends;
   - wherein cleavage of said polynucleotidic building blocks with restriction enzyme A results in a polynucleotide comprising a polynucleotidic module flanked on one side by a cohesive end that can be re-ligated with a polynucleotide building block cleaved by restriction enzyme B without restoring a sequence cleavable by restriction enzyme A and/or B;
   - wherein cleavage of said polynucleotidic building blocks with restriction enzyme B results in a polynucleotide comprising a polynucleotidic module flanked on one side by a cohesive end that can be re-ligated with a polynucleotide building block cleaved by restriction enzyme A without restoring a sequence cleavable by restriction enzyme A and/or B;
b) generating "n" polynucleotides linked to a solid phase comprising at least:
   - one polynucleotidic module;one end linked to a solid phase;
   - a single cleavage site for a first restriction enzyme A, placed on the side of the polynucleotidic module that is linked to a solid phase;
   - a free end compatible with the cohesive ends resulting from cleavage with restriction enzyme A, and which ligation with a polynucleotidic building block cleaved by restriction enzyme A will not produce a sequence cleavable by restriction enzyme A and/or B;
c) generating one C-terminal polynucleotidic building block comprising at least:
   - one polynucleotidic module;
   - a single cleavage site for a first restriction enzyme A, placed on one side of the polynucleotidic module;
   - a single cleavage site for a second restriction enzyme B, placed on the other side of the polynucleotidic module;
   wherein cleavage of said polynucleotidic building block with restriction enzyme B results in a polynucleotide comprising a polynucleotide module flanked on one side by a cohesive end that cannot be re-ligated with a polynucleotide building block cleaved by restriction enzyme A and/or B;
d) cutting said one C-terminal polynucleotidic building block of c) with restriction enzyme A;
e) ligating the resulting C-terminal polynucleotidic module with the free end of one polynucleotide of b) immobilized on a solid phase, thereby producing a new immobilized polynucleotide comprising one additional polynucleotidic module;
f) cutting the resulting new immobilized polynucleotide with restriction enzyme A, thus producing a new polynucleotide having a free end compatible with the cohesive ends resulting from cleavage with restriction enzyme B;
g) ligating the new polynucleotide with the free end of one polynucleotide of b) immobilized on a solid phase thus producing a new immobilized polynucleotide comprising one additional polynucleotidic module;

In a preferred embodiment, steps e) and g) are respectively followed by washing steps such as three washes with an adapted volume of saline buffer such as PBS well known in the art.

In another embodiment, steps f) and g) are repeated N times to produce an immobilized polynucleotide having an array of n polynucleotidic modules wherein n = N + 3.

In another embodiment, step g) is replaced by the following steps:
g') cutting said at least one polynucleotidic building block of a) with restriction enzyme A;
h) ligating the resulting polynucleotidic module with the free end of one polynucleotide of b) immobilized on a solid phase, thereby producing a new immobilized polynucleotide comprising one additional polynucleotidic module;
i) cutting the resulting new immobilized polynucleotide with restriction enzyme B, thus producing a new immobilized polynucleotide having a free end compatible with the cohesive ends resulting from cleavage with restriction enzyme A;
j) ligating on the new produced immobilized polynucleotide said new polynucleotide resulting from step f) thereby producing a new immobilized polynucleotide comprising "x" additional polynucleotidic modules wherein "x" is equal to the number of polynucleotidic modules present in said new polynucleotide resulting from step f);

In a preferred embodiment, "x" can be any number comprised between 1 and 50, preferably, between 1 and 20, more preferably, 19, 18, 17, 16, 15, 14, 13, 12, 11, 10, 9, 8, 7, 6, 5, 4, 3, 2, or 1.

In a preferred embodiment, steps h) and j) are respectively followed by washing steps such as three washes with an adapted volume of saline buffer such as PBS well known in the art.

In another embodiment, said method of this aspect of the present invention further comprises the steps:
a) Cutting the resulting new immobilized polynucleotide of step j) with restriction enzyme A, thus producing a new polynucleotide having a free end compatible with the cohesive ends resulting from cleavage with restriction enzyme B;
b) Ligating the new polynucleotide with the free end of one polynucleotide of b) immobilized on a solid phase thus producing a new immobilized polynucleotide comprising one additional polynucleotidic module;

In a preferred embodiment, step b) is respectively followed by washing steps such as three washes with an adapted volume of saline buffer such as PBS well known in the art.

In another embodiment, at least one said polynucleotidic building block of a) and / or at least one polynucleotide linked to a solid phase of b) and / or said polynucleotidic building block of c) comprise a pre-assembly of more than one polynucleotidic module.

In another embodiment, at least one said polynucleotidic building block of a) and / or at least one polynucleotide linked to a solid phase of b) and / or said polynucleotidic building block of c) comprise a fragment of building block. In another embodiment, said fragment of building block is a building block deleted at its 3' end. In another embodiment, said fragment of building block is a building block deleted in its center. In another embodiment, said fragment of building block encodes at least a half Transcription Activator-like Effector (TALE) DNA binding repeat module.

In another embodiment, at least one said polynucleotidic building block of a) and / or at least one polynucleotide linked to a solid phase of b) and / or said polynucleotidic building block of c) comprise a building block variant.

In another embodiment, at least one said polynucleotidic building block of a) and / or at least one polynucleotide linked to a solid phase of b) and / or said polynucleotidic building block of c) comprise a polynucleotide sequence not highly similar to a polynucleotide module according to a).

In another embodiment, at least one said polynucleotidic building block of a) and / or at least one polynucleotide linked to a solid phase of b) and / or said polynucleotidic building block of c) further comprises a fragment of building block according to a).

In another embodiment, said one polynucleotide of b) has been generated by:
b1) generating one polynucleotide linked to a solid phase comprising:
   - one polynucleotidic module;
   - one end linked to a solid phase;
   - a single cleavage site for a first restriction enzyme A, placed on the side of the polynucleotidic module that is linked to a solid phase;
   - a single cleavage site for a second restriction enzyme B placed on the other side of the polynucleotide module;
b2) cutting said polynucleotide linked to a solid phase with restriction enzyme B thereby obtaining a polynucleotide with a free end compatible with the cohesive ends resulting from cleavage with restriction enzyme A, and which ligation with a polynucleotidic building block cleaved by restriction enzyme A will not produce a sequence cleavable by restriction enzyme A and/or B;

In another embodiment, said one polynucleotide of b) has been generated by:
b1) generating one polynucleotide linked to a solid phase comprising:
   - one end linked to a solid phase;
   - a polynucleotide sequence not highly similar to a polynucleotide module according to a), wherein said polynucleotide sequence comprises a single cleavage site for a first restriction enzyme A, placed on the side of the polynucleotidic module that is linked to a solid phase;
   - a free end compatible with the cohesive ends resulting from cleavage with restriction enzyme A, and which ligation with a polynucleotidic building block cleaved by restriction enzyme A will not produce a sequence cleavable by restriction enzyme A and/or B;

In another embodiment, said one polynucleotide of b) has been generated by:
b1) generating one polynucleotide linked to a solid phase comprising:
   - one end linked to a solid phase;
   - a polynucleotide sequence not highly similar to a polynucleotide module according to a), wherein said polynucleotide sequence comprises a single cleavage site for a first restriction enzyme A, placed on the side of the polynucleotidic module that is linked to a solid phase and a single cleavage site for a second restriction enzyme B placed on the other side of the polynucleotide module;
b2) cutting said polynucleotide linked to a solid phase with restriction enzyme B thereby obtaining a polynucleotide with a free end compatible with the cohesive ends resulting from cleavage with restriction enzyme A, and which ligation with a polynucleotidic building block cleaved by restriction enzyme A will not produce a sequence cleavable by restriction enzyme A and/or B;

In another embodiment, said one polynucleotide of b) has been generated by:
b1) generating one polynucleotide linked to a solid phase comprising:
   - one end linked to a solid phase;
   - a polynucleotide sequence not highly similar to a polynucleotide module according to a), wherein said polynucleotide sequence comprises a single cleavage site for a first restriction enzyme A, placed on the side of the polynucleotidic module that is linked to a solid phase;
   - a free end compatible with the cohesive ends resulting from cleavage with restriction enzyme A, and which ligation with a polynucleotidic building block cleaved by restriction enzyme A will not produce a sequence cleavable by restriction enzyme A and/or B;
b2) cutting a polynucleotidic building block described in a) with restriction enzyme A;
b3) ligating the resulting polynucleotidic module with the free end of the polynucleotide immobilized on the solid phase;
b4) cutting the resulting new immobilized polynucleotide with restriction enzyme B, thus producing a new immobilized polynucleotide comprising:
   - one end linked to a solid phase;
   - a polynucleotide sequence not highly similar to a polynucleotide module according to a);
   - one polynucleotidic module;
   - a free end compatible with the cohesive ends resulting from cleavage with restriction enzyme A, and which ligation with a polynucleotidic building block cleaved by restriction enzyme A will not produce a sequence cleavable by restriction enzymes A and/or B.

In another embodiment, at least one polynucleotide linked to a solid phase of b) has been generated by a gene synthesis technology wherein said free end compatible with the cohesive ends resulting from cleavage with restriction enzyme A has been obtained by using restriction enzymes or specific annealing and wherein said polynucleotide ligation with a polynucleotidic building block cleaved by restriction enzyme A will not produce at least a sequence cleavable by restriction enzyme A and/or B.

In another embodiment, said at least one polynucleotide linked to a solid phase of b) has been generated by:
- synthesis of a first oligonucleotide linked to a solid phase;
- synthesis of a second oligonucleotide complementary to the first one;
wherein annealing of both oligonucleotides in appropriate conditions generates, without using restriction enzymes, a double stranded polynucleotide with a free end compatible with the cohesive ends resulting from cleavage with restriction enzyme A, and which ligation with a polynucleotidic building block cleaved by restriction enzyme A will not produce a sequence cleavable by restriction enzyme A and/or B.
Parameters for reaching annealing appropriate conditions of oligonucleotides are well known in the art.

In another embodiment, said at least one polynucleotide linked to a solid phase of b) has been generated by:
- synthesis of a first oligonucleotide linked to a solid phase;
- synthesis of a second oligonucleotide complementary to the first one; wherein annealing of both oligonucleotides in appropriate conditions generates a double stranded polynucleotide with a single cleavage site for a second restriction enzyme B placed on the side of the polynucleotide that is not linked to a solid phase;
- Cutting said polynucleotide linked to a solid phase with restriction enzyme B thereby obtaining a polynucleotide with a free end compatible with the cohesive ends resulting from cleavage with restriction enzyme A, and which ligation with a polynucleotidic building block cleaved by restriction enzyme A will not produce a sequence cleavable by restriction enzyme A and/or B;

In another embodiment, said polynucleotide sequence not highly similar to a polynucleotide module according to a) is linked to said solid phase.

In another embodiment, at least one said polynucleotide linked to a solid phase of b) comprises a sequence not highly similar to a polynucleotide module according to a) encoding a N-terminal polypeptidic sequence of a TALE.

In another embodiment, at least one said polynucleotidic building block of a) and / or said polynucleotidic building block of c) comprises a sequence not highly similar to a polynucleotide module according to a) encoding a C-terminal polypeptidic sequence of a TALE.

In another embodiment, at least one said polynucleotidic building block of a) and / or at least one polynucleotide linked to a solid phase of b) and / or said polynucleotidic building block of c) further comprises at least one cleavage site for a restriction enzyme C located outward compared to restriction enzymes A and / or B cleavage sites.

In another embodiment, the last polynucleotide of b) used comprises a single cleavage site for a restriction enzyme C placed on the side of the polynucleotide linked to a solid phase, located outward compared to restriction enzyme A cleavage site, wherein said cleavage with restriction enzyme C allows to unlink said polynucleotide from the solid phase.

In another embodiment of the invention is a method further comprising the step of unlinking said final polynucleotide comprising an array of polynucleotidic modules by cutting it with restriction enzyme C.

In another embodiment of the invention is a method further comprising the steps of:
- unlinking said final polynucleotide comprising an array of polynucleotidic modules of step g) by cutting it with restriction enzyme C;
- subcloning said final polynucleotide comprising an array of polynucleotidic modules into a plasmidic vector.

In another embodiment, said method further comprises the step of subcloning said final polynucleotide comprising an array of polynucleotidic modules from a plasmidic vector into another plasmidic vector by cutting it with restriction enzymes A and B;

In another embodiment of the invention is a method further comprising the steps of:
- unlinking said final polynucleotide comprising an array of polynucleotidic modules of step g) by cutting it with restriction enzymes A and / or B;
- subcloning said final polynucleotide comprising an array of polynucleotidic modules into a plasmidic vector.

In another embodiment of this aspect of the disclosure is a method wherein said polynucleotidic modules to assemble share at least 85% similarity.

In another embodiment of this aspect of the invention is a method wherein each polynucleotidic module encodes a Transcription Activator-like Effector (TALE) DNA binding repeat module.

In another embodiment of this aspect of the disclosure is a method wherein said polynucleotide sequence not highly similar to a polynucleotide module according to a) encodes a C-terminal fragment of a TALE and wherein said fragment of building block encodes at least a half Transcription Activator-like Effector (TALE) DNA binding repeat module.

In another embodiment of this aspect of the invention, said single cleavage sites respectively for restriction enzymes A and B are two different cleavage sites cleavable by restriction enzymes which produce compatible cohesive overhang ends and wherein said compatible cohesive overhangs remove respective recognition sites for said restriction enzymes A and B upon ligation. In other words, restriction enzymes A and B according to the present invention produce at their respective single cleavage site compatible overhang cohesive ends without restoring a sequence cleavable by restriction enzymes A and B after ligation. In another embodiment, said restriction enzymes A and B belong to subtypes of class II restriction enzymes such as subtypes A, B, C, H and S as listed for example at http://_rebase.neb.com. In another embodiment, said restriction enzymes A and B of the present invention belong to typeIIS restriction enzymes. In a preferred embodiment said restriction enzymes A and B of the present invention are BbvI and SfaNI.

In another embodiment, said single cleavage sites respectively for restriction enzymes A and B can be cleavage sites for other enzymes such as nick-creating enzymes (nickases as non-limiting examples) under appropriate use to generate compatible overhang cohesive ends.

In another embodiment of this aspect of the invention, said restriction enzyme C is SfiI.

In another embodiment of this aspect of the invention is a method wherein said at least one polynucleotidic building block of a) and / or said polynucleotides of b) linked to a solid phase and / or said polynucleotidic building block of c) are part of a collection encoding polypeptidic repeated modules with Repeat Variable Dipeptide regions (RVDs) comprising a pair of amino acids responsible for recognizing one nucleotide selected from the group consisting of HD for recognizing C, NG for recognizing T, NI for recognizing A, NN for recognizing G or A, NS for recognizing A, C, G or T, HG for recognizing T, IG for recognizing T, NK for recognizing G, HA for recognizing C, ND for recognizing C, HI for recognizing C, HN for recognizing G, NA for recognizing G, SN for recognizing G or A and YG for recognizing T.

In another embodiment of this aspect of the invention, is a method wherein said at least one polynucleotidic building block of a) and / or said polynucleotides of b) linked to a solid phase and / or said polynucleotidic building block of c) are part of a collection encoding polypeptidic repeated modules with Repeat Variable Dipeptide regions (RVDs) comprising a pair of amino acids responsible for recognizing one nucleotide selected from the group consisting of HD for recognizing C, NG for recognizing T, NI for recognizing A, NN and NK for recognizing G.

In another embodiment of this aspect of the disclosure is a method wherein said at least one polynucleotidic building block of a) and / or said polynucleotides of b) linked to a solid phase and / or said polynucleotidic building block of c) are part of a collection encoding "y" polypeptidic repeated modules with Repeat Variable Dipeptide regions (RVDs) for recognizing "y" nucleotides via "y" pairs of amino acids wherein "y" is comprised between 1 and 8. In another embodiment, "y" equals 1, 2, 3, 4, 5, 6, 7 or 8.

In another embodiment of this aspect of the disclosure is a method wherein said at least one polynucleotidic building block of a) and / or said polynucleotides of b) and / or said polynucleotidic building block of c) linked to a solid phase are part of a collection encoding two polypeptidic repeated modules with Repeat Variable Dipeptide regions (RVDs) for recognizing two nucleotides via two pairs of amino acids selected from the group listed in table 2.

In another embodiment of this aspect of the disclosure is a method wherein said at least one polynucleotidic building block of a) and / or said polynucleotides of b) and / or said polynucleotidic building block of c) linked to a solid phase are part of a collection encoding three polypeptidic repeated modules with Repeat Variable Dipeptide regions (RVDs) for recognizing three nucleotides via three pairs of amino acids selected from the group listed in table 3.

In another embodiment of this aspect of the disclosure is a method wherein said at least one polynucleotidic building block of a) and / or said polynucleotides of b) linked to a solid phase and / or said polynucleotidic building block of c) are part of a library of degenerated building blocks.
In another embodiment of this aspect of the disclosure a library of polynucleotidic building blocks are processed in previously described steps d) to g), g') and h) to j) of the method of the present invention comprising a library of polynucleotidic modules. In another embodiment said library can be a random mutagenized library of polynucleotidic modules. In another embodiment, a library of polynucleotidic building blocks are used in previously described steps d) to g), g') and h) to j) of the method of the present invention comprising a library of polynucleotidic modules and encoding a library of TALE repeat polynucleotidic modules. Mutagenesis can concern the entire polynucleotidic module sequence. In another embodiment said library can be a random mutagenized library of TALE repeat polynucleotidic modules. Mutagenesis of said TALE repeat polynucleotidic modules library can focused on critical amino acids such as amino acids located at positions 12 and 13 as non-limiting examples. Mutagenesis of said TALE repeat polynucleotidic modules library can focused on other critical amino acids of said TALE repeat polynucleotidic modules. Mutagenesis of said TALE repeat polynucleotidic modules library may concern the entire TALE repeat module sequence. Said libraries of polynucleotidic modules constituting libraries of polynucleotidic building blocks can be used according to the method of the present invention to introduce diversity into polynucleotides comprising an array of polynucleotidic modules. As non-limiting example, the method according to the present invention is particularly well-suited to introduce diversity into TALE repeat polynucleotidic modules in order to produce high-diversity libraries of TALE DNA binding domains.

In another aspect of the present invention is a method of conducting a high throughput custom-designed platform of TALE DNA binding domains comprising:
a) receiving a DNA target sequence comprising "n" nucleotides, which TALE DNA binding domain has to bind;
b) generating and assembling polynucleotidic repeated modules, each comprising a pair of amino acids for recognizing each one of the "n" nucleotides of said DNA target sequence according to the present invention, thus releasing a TALE DNA binding domain able to recognize said DNA target sequence;
c) providing said custom-designed TALE DNA binding domains.

In another embodiment "n" is comprised between 1 and 50, preferably 45, more preferably 40, more preferably 35, more preferably 30, more preferably, 29, 28, 27, 26, 25, 24, 23, 22, 21, 20, 19, 18, 17, 16, 15, 14, 13, 12, 11, 10, 9, 8, 7, 6, 5, 4, 3, 2 or 1.

In another aspect of the present disclosure is a method of conducting a high throughput custom-designed platform of chimeric protein derived from a TALE comprising:
a) Receiving a DNA target sequence comprising "n" nucleotides, which a chimeric protein derived from a TALE has to process;
b) Generating and assembling polynucleotidic repeated modules, each comprising a pair of amino acids for recognizing each one of the "n" nucleotides of said DNA target sequence according to the present invention, thus releasing a TALE DNA binding domain able to recognize said DNA target sequence;
c) Fuse said DNA binding domain to a protein domain able to process said DNA target sequence;
d) Providing said custom-designed chimeric protein derived from a TALE.

In another embodiment "n" is comprised between 1 and 50, preferably 45, more preferably 40, more preferably 35, more preferably 30, more preferably, 29, 28, 27, 26, 25, 24, 23, 22, 21, 20, 19, 18, 17, 16, 15, 14, 13, 12, 11, 10, 9, 8, 7, 6, 5, 4, 3, 2 or 1.

In another aspect of the present disclosure is a kit for generating and assembling polynucleotides comprising arrays of at least two highly similar polynucleotidic modules according to the present invention comprising at least a collection of "n" polynucleotidic building blocks encoding "n" polypeptidic repeated modules with Repeat Variable Dipeptide regions (RVDs) for recognizing "n" nucleotides via "n" pairs of amino acids (TALE "n" repeat modules or "n" repeat modules) and instructions to use it.

In another aspect of the present disclosure is a method for producing high diversity libraries of polynucleotides comprising arrays of polynucleotidic modules encoding TALE DNA binding domains comprising:
a) Generating a high diversity library of polynucleotidic building blocks;
b) Assembling polynucleotidic building blocks according to the method of the present invention by using high diversity library of polynucleotidic building blocks of step a) for previously described steps d) to g).

### Definitions

- By "polynucleotidic building block(s)" or "building block(s)" is intended polynucleotidic entities or polynucleotides comprising polynucleotidic modules to assemble according to the present invention. Said building block(s) comprises one or several polynucleotidic modules and allows the assembly of several polynucleotidic modules. In addition to polynucleotidic module(s), said polynucleotidic building block(s) can comprise one single cleavage site for a restriction enzyme or two single cleavage sites for different restriction enzymes and wherein cleavage of said polynucleotidic building blocks with restriction enzyme(s) result in compatible cohesive ends which allow the assembly of several polynucleotidic modules. In addition, said polynucleotidic building block(s) can comprise other polynucleotidic sequences not highly similar to a polynucleotidic module A "fragment of polynucleotidic building block(s)" or "a fragment of building blocks" can be used in the present invention to describe a building block comprising only one single cleavage site for a restriction enzyme A or B or lacking one single cleavage site for a restriction enzyme A or B. A "fragment of polynucleotidic building block" can be deleted at its 3' end, at its 5' end or in its center. More generally, the expression "fragment of polynucleotidic building block" is used to describe a truncated polynucleotide building block.
   By "degenerated building block" is intended a polynucleotidic building block which contains one difference or several differences at specific locations in its sequence compared to a polynucleotidic building block of reference. This particularly applies when diversity has to be introduced in said polynucleotidic building block. Degenerated polynucleotidic building blocks with partial or total diversity introduced at one or several locations of its nucleotidic codon sequence can be used to screen the best interaction of a polynucleotidic building block toward a DNA target sequence; as a non-limiting example, a DNA target sequence of interest can be used as a bait to screen a library of "degenerated building blocks" wherein each "degenerated building block" comprises a similar and related sequence containing diversity at one or several nucleotidic codons (and amino acids encoded by these codons).
- By "polynucleotidic module(s)" or "modules" is intended polynucleotidic entities or polynucleotides to assemble according to the present invention. Said polynucleotidic module(s) can be highly similar modules such as TALE repeat modules as a non-limiting example. Highly similar modules present in multiple copies can be described as "repeated modules".
- "Pre-assembly" means that a polynucleotidic building block used to elongate the polynucleotidic modules assembly process according to the present invention can comprise more than one polynucleotidic module. In this case, said polynucleotidic building block can be described as pre-assembled.
- By a "TALE-nuclease" (TALEN) is intended a fusion protein consisting of a DNA-binding domain derived from a Transcription Activator Like Effector (TALE) and one nuclease catalytic domain to cleave a nucleic acid target sequence. First published TALEN are formed by fusion of the cleavage domain of FokI and a TALE DNA binding domain.
- By "TALE DNA binding domain" is intended part of a Transcription Activator Like Effector (TALE) responsible of DNA binding and composed by a variable number of 33-35 amino acids "repeat modules" or "TALE repeat polynucleotidic modules" or "TALE repeat modules" or "RVDs domains". The nature of residues 12 and 13 determines base preferences of individual repeat module.
- By "chimeric protein" according to the present disclosure is meant any fusion protein comprising a set of repeated modules with RVDs (or with RVDs-like domains) to bind a nucleic acid sequence and one protein domain to process a nucleic acid target sequence adjacent to said bound nucleic acid sequence. Said chimeric protein according to the present disclosure can function as a dimer wherein each monomer constituting said chimeric dimeric protein comprises a set of repeated modules with RVDs (or with RVDs-like domains) to bind a nucleic acid sequence and one protein domain to process a nucleic acid target sequence adjacent to said bound nucleic acid sequence. - "identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of "similarity" or identity between nucleic acid or amino acid sequences is a function of the number of identical or matching nucleotides or amino acids at positions shared by the nucleic acid or polypeptidic sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting. Common software tools used for general sequence alignments taskes include ClustalW for alignment and BLAST or FASTA for database searching.

In sequence alignments of proteins, the degree of similarity between aminoacids occupying a particular position in the sequence can be interpreted as a rough measure of how conserved a particular region or a sequence motif is among lineages. The absence of substitutions in a particular region or sequence or the presence of only very conservative substitutions by amino acids whose side chains have similar biochemical properties, suggest that this region has structural or functional importance.

"Percent identity" or "percent similarity" are used to quantify the similarity between biomolecule sequences. For two naturally occurring sequences, percent identity is a factual measurement, whereas similarity is a hypothesis supported by evidence.
- Amino acid residues in a polypeptide sequence can be designated herein according to the one-letter or three-letter code, in which, for example, Q means Gln or Glutamine residue, R means Arg or Arginine residue and D means Asp or Aspartic acid residue.
- Amino acid substitution means the replacement of one amino acid residue with another, for instance the replacement of an Arginine residue with a Glutamine residue in a peptide sequence is an amino acid substitution.
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- by "DNA target", "DNA target sequence", "target DNA sequence", "nucleic acid target sequence", "target sequence" , or "target" is intended a polynucleotide sequence that is recognized by the DNA binding domain of a protein. These terms refer to a specific DNA location, preferably a genomic location in a cell, but also a portion of genetic material that can exist independently to the main body of genetic material such as plasmids, episomes, virus, transposons or in organelles such as mitochondria or chloroplasts as non-limiting examples. The nucleic acid target sequence is defined by the 5' to 3' sequence of one strand of said target.
- by "generating" a polynucleotide, a polynucleotidic building block or another polynucleotidic entity is meant to synthetize or to make synthetize this entity by one of the gene synthesis methods well-known in the art. It is also encompassed in this definition the generation of said polynucleotidic entity by Polymerase Chain Reaction using appropriate oligonucleotide primers, degenerated or not, linked to a non-polynucleotidic entity, such as Biotin as a non-limiting example, or not.
- by "variant", "polynucleotidic building block variant", "building block variant", "chimeric protein variant" is intended a molecule obtained by replacement of at least one nucleotide, or at least one amino acid residue compared to a polynucleotidic building block, a building block or a chimeric protein, respectively taken as a reference. According to this definition, a variant can result from a truncation, or a mutation or a sequence insertion. Said inserted sequence can possibly be one or several nucleotides, one or several amino acids, one protein motif or one reporter protein.

The above written description of the invention provides a manner and process of making and using it such that any person skilled in this art is enabled to make and use the same, this enablement being provided in particular for the subject matter of the appended claims, which make up a part of the original description.

As used above, the phrases "selected from the group consisting of," "chosen from," and the like include mixtures of the specified materials.

Where a numerical limit or range is stated herein, the endpoints are included. Also, all values and subranges within a numerical limit or range are specifically included as if explicitly written out.

### Examples

### -Example 1: Sequential assembly of Trancription activator-like effector (TALE) epeat polynucleotidic modules in solution

### 1a- Construction of polynucleotidic building blocks comprising TALE repeat polynucleotidic modules

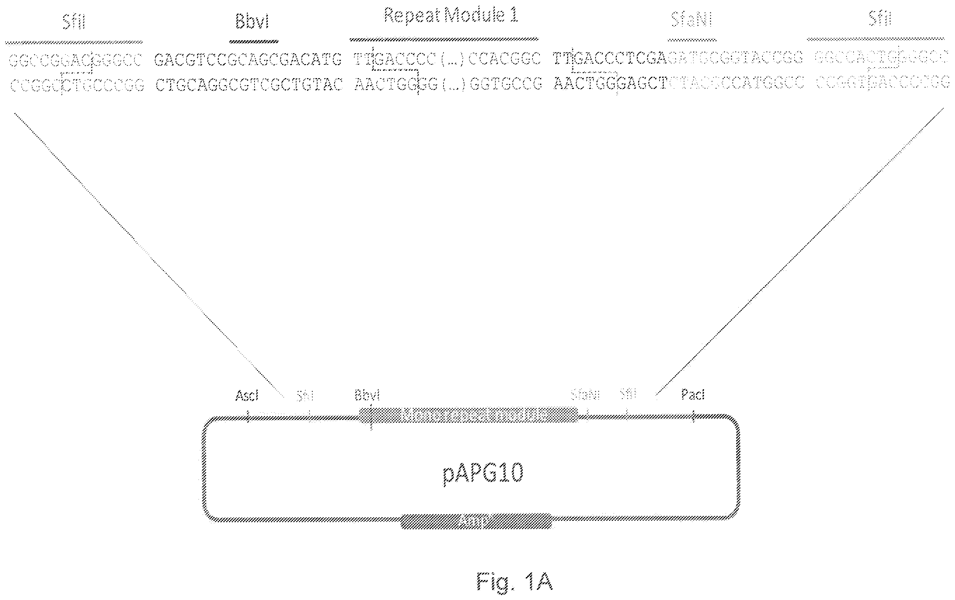

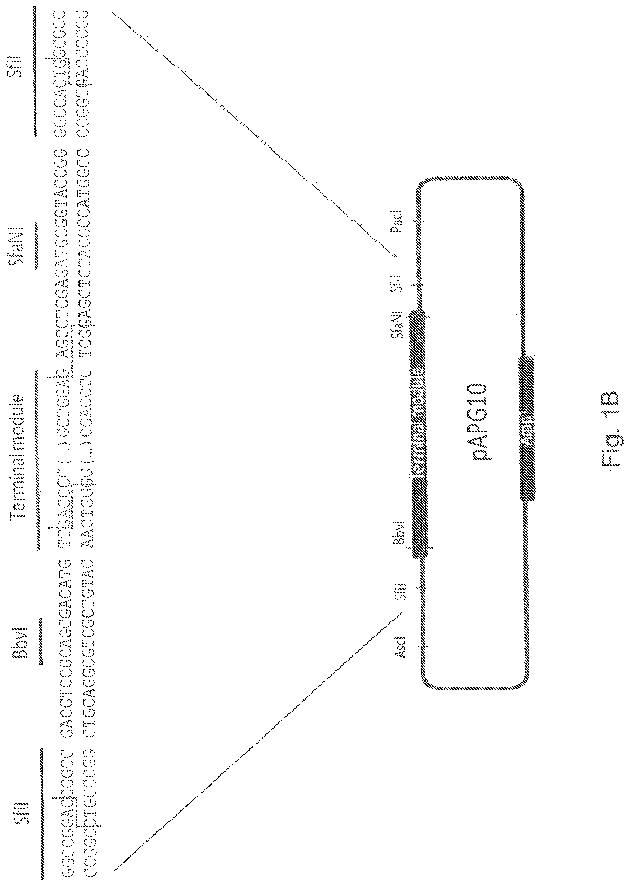

TALE repeat polynucleotidic modules or repeat modules or TALE repeat modules of AvrBS3 from *Xanthomonas* spp. plant pathogen, containing the RVDs NN, NK, NI, HD, NG and the terminal half repeat NG* are synthesized and cloned in the pAPG10 plasmid between restriction sites AscI and PacI (SEQ ID NO: 6-12, figure 1). Within the pAPG10 plasmid, mono repeats are flanked by a 5' end region containing SfiI and BbvI sites and 3' end region containing SfaNI and SfiI sites (figure 1A), i.e a polynucleotidic building block (SEQ ID NO: 13-18). BbvI and SfaNI restriction sites are designed to generate compatible overhangs that allow for coupling two repeat modules together in an oriented fashion. For that purpose, BbvI site is oriented to cut inward into the 5' end of the repeat module sequence such that digestion leaves the appropriate overhang (GACC) to accept a SfaNI digested additional repeat module. Accordingly, SfaNI site was oriented to cut outward into the 3' end flanking sequence of the repeat module such that digestion leaves an appropriate overhang (CTGG) to accept a Bbvl digested repeat module. The terminal half RVD is synthesized and subcloned in pAPG10 according to the same strategy except that the SfaNI site is oriented to cut inward into the 3' end sequence of the repeat module (figure 1B).Example of building block encoding a terminal half RVD is given by SEQ ID NO: 19. SfaNI digestion generates an overhang that is compatible with the one generated by BsmB1 in the final acceptor plasmid (pCLS7183, SEQ ID NO: 20) as will be described below. This design is intended to control the orientation of TALE repeat insertion within pCLS7183. pCLS7183 (SEQ ID NO: 20) is an expression vector comprising the N-terminal and C-terminal domains of the of AvrBs3 protein. A BsmB1 recognition site has been appropriately introduced between the 2 domains. The expression of the chimeric protein is driven by a CMV promoter and can comprise a protein domain able to process a nucleic target sequence adjacent to the nucleic acid sequence bound by the TALE DNA binding domain.

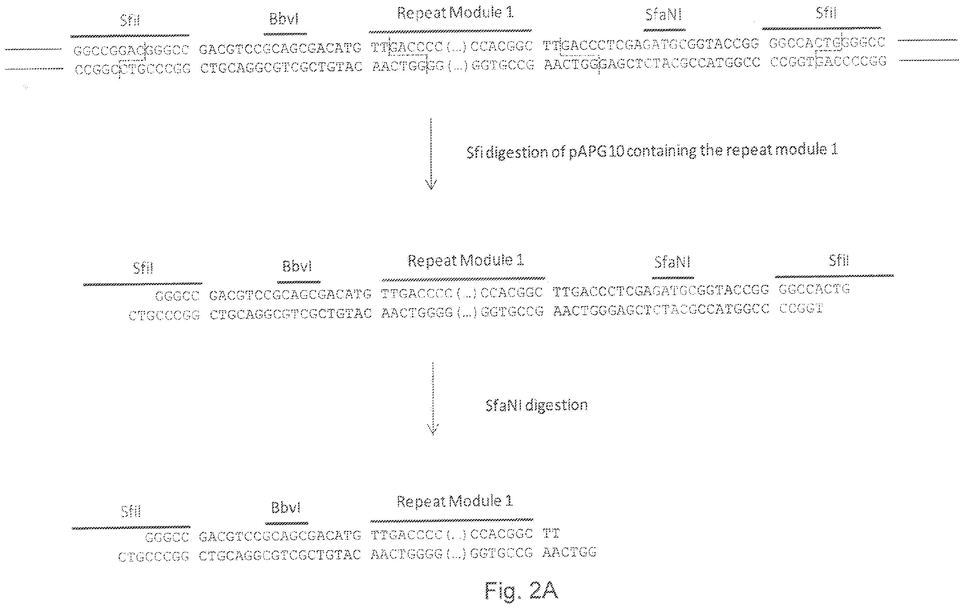

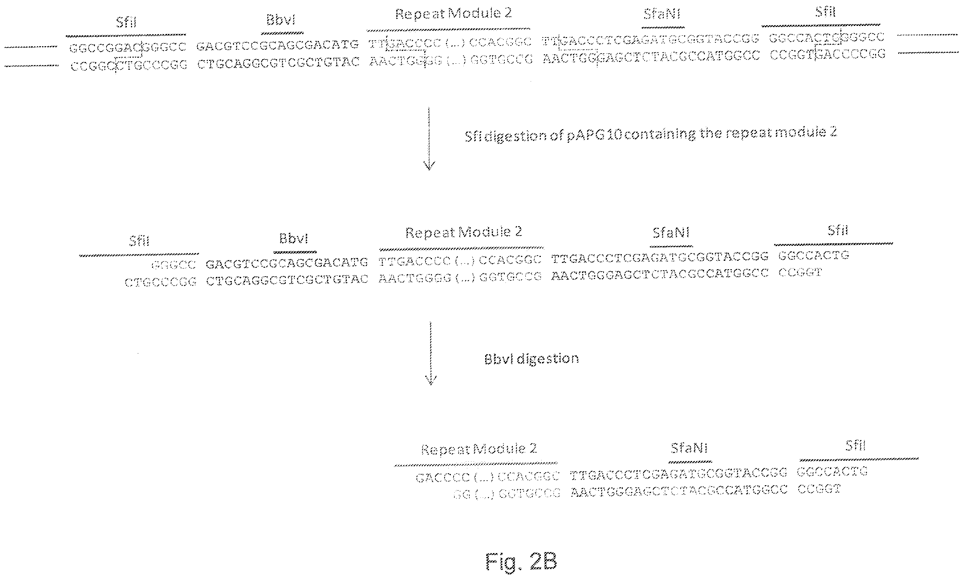

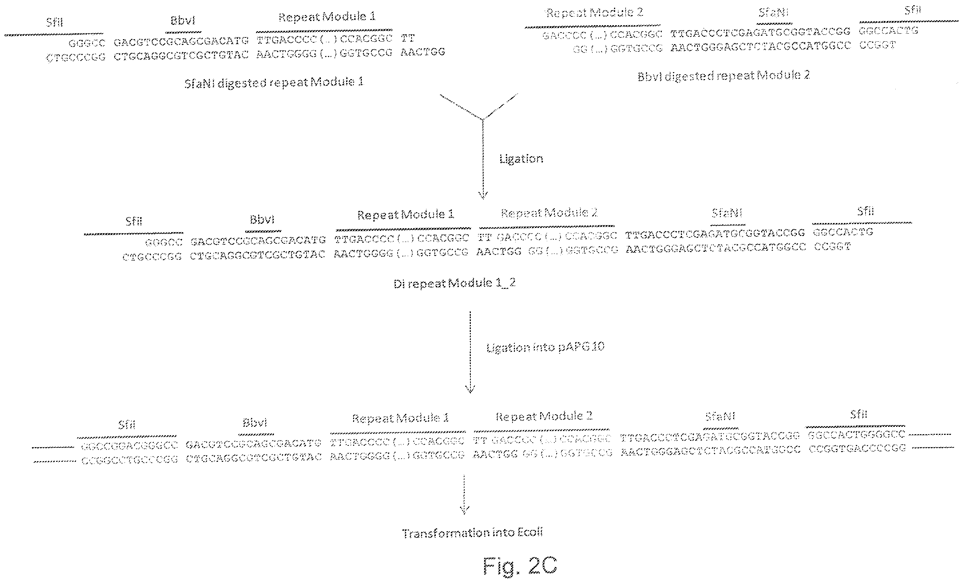

As an illustrative example, to prepare the building block encoding di repeat module 1-2, pAPG10 encoding the repeat modules 1 and 2 [building blocks encoding modules 1 and 2 (SEQ ID NO: 13 and 14)] are first digested by SfiI (Figure 2A and 2B). Extracted building blocks encoding repeat modules 1 and 2 are purified from agarose gel and then digested respectively by SfaNI and BbvI (Figure 2A and 2B) (SEQ ID NO: 21 and 22). Digested fragments are then ligated together to form the building block encoding di repeat module fragment 1-2 in a seamless manner (figure 2C) (SEQ ID NO: 23). Building block encoding di repeat module fragment is then purified on column, subcloned into the SfiI digested pAPG10 and transformed in DH5α competent E. coli (figure 2C, lower part). Clones having building block encoding di repeat module are identified by colony PCR screening using appropriate primers (M13_Forward and M13_Reverse, SEQ ID NO: 24-25 respectively).

### 1b-Sequential assembly of TALE repeat polynucleotidic modules

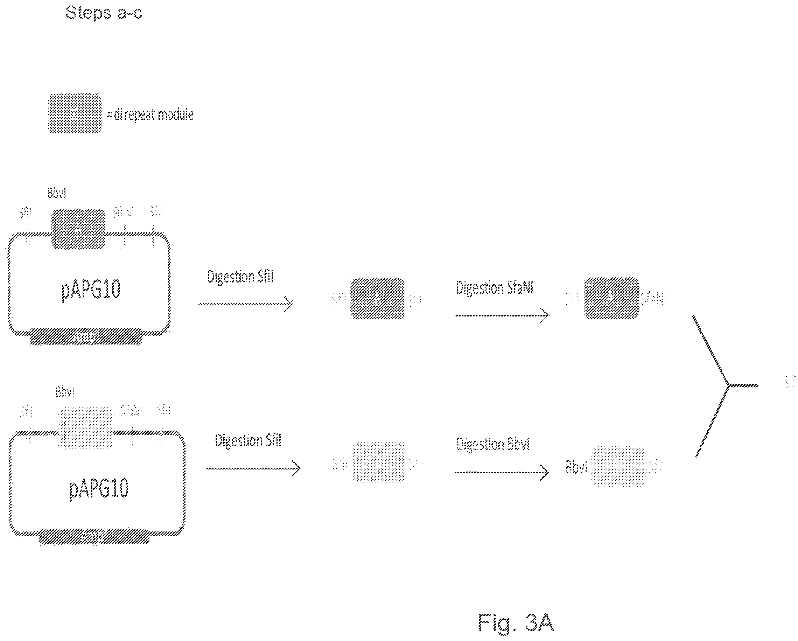

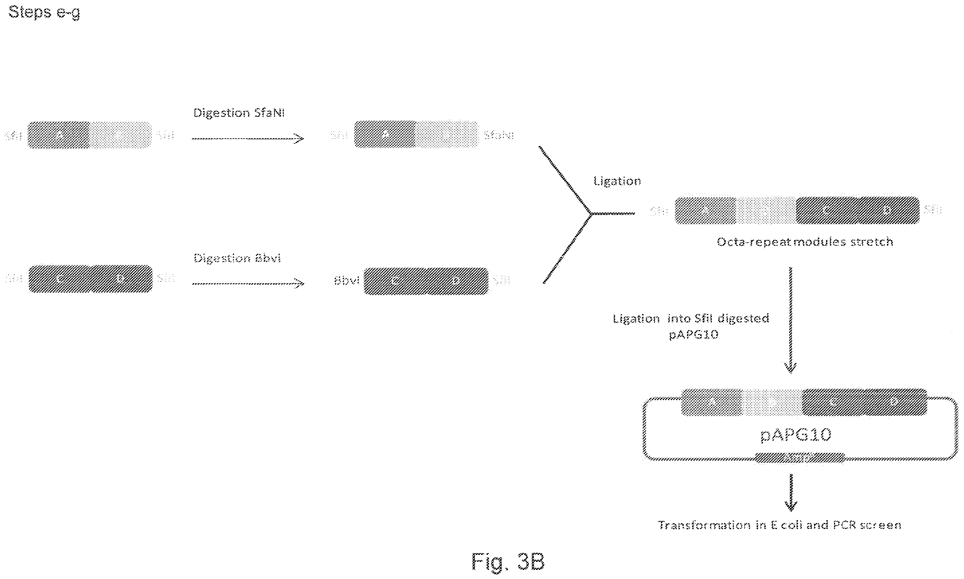

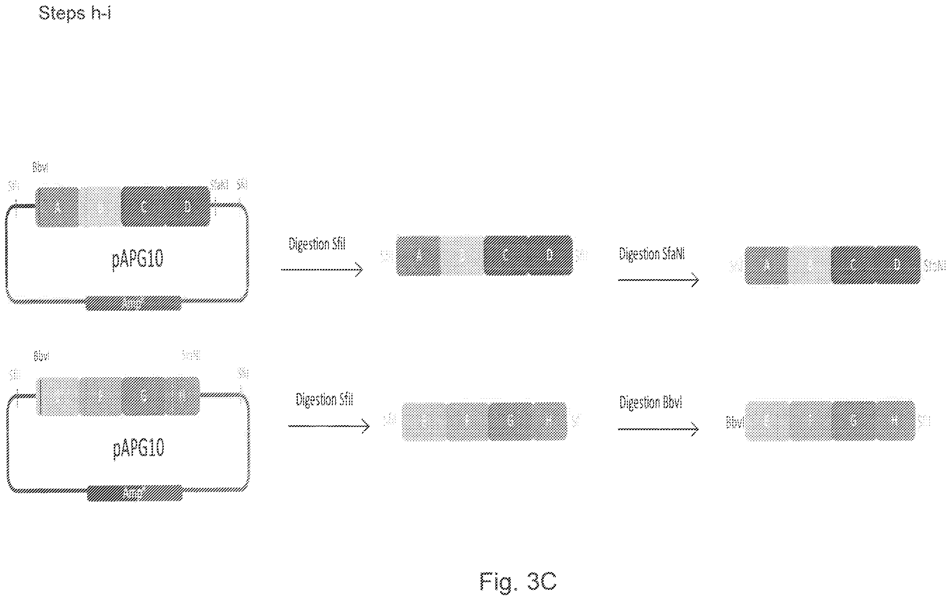

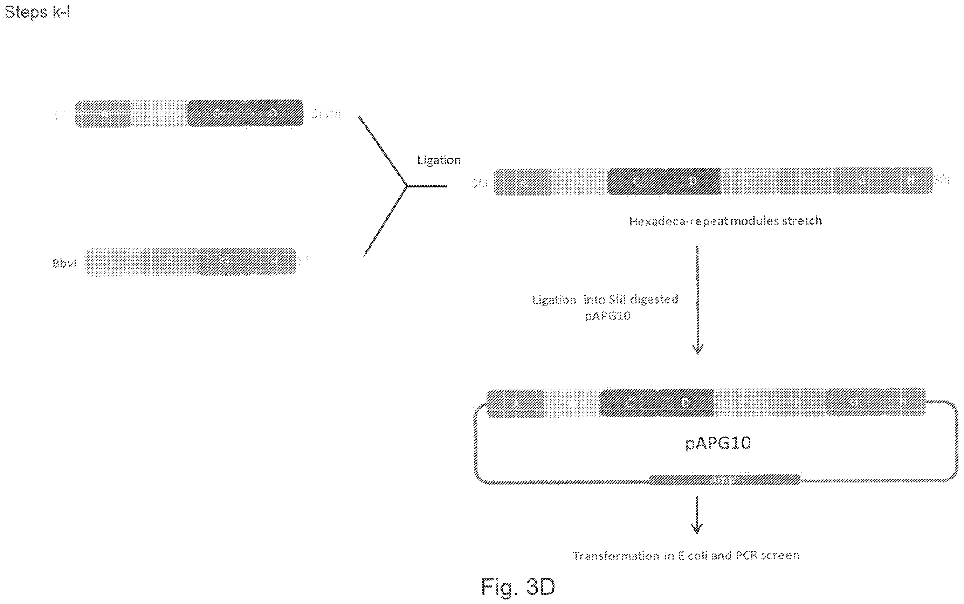

A method to assemble TALE repeat polynucleotidic modules comprised in polynucleotidic building blocks (or TALE building blocks) was designed according to the following protocol. It should be noted that TALE building blocks may contain multiple TALE repeat modules obtained from previous assemblies. As illustrated in figure 3, below is described an example of 15.5 repeat fragment assembly using building blocks encoding TALE di repeat modules.
a-Choose a plasmid containing the first building block encoding the desired TALE di repeat module, extract it with SfiI and SfaNI restriction enzymes (polynucleotidic module A), and purify it on column (As an illustrating non-limiting example a TALE di repeat module HD_NI digested by Sfil and SfaNI is given by SEQ ID NO: 26);
b-Choose the plasmid containing the appropriate second building block encoding the desired second TALE di repeat module, extract it with SfiI and BbvI restriction enzymes (polynucleotidic module B), and purify it on column (As an illustrating non-limiting example a TALE di repeat module NG_NK digested by SfiI and SfaNI is given by SEQ ID NO: 27);
c-Ligate polynucleotidic modules A and B and purify the ligation product (polynucleotide AB) on column (Resulting TALE four repeat module HD_NI_NG_NK is given by SEQ ID NO: 28);
d-In parallel, repeat steps (a)-(c) using the appropriate plasmids in order to generate polynucleotide CD;
e-Digest polynucleotide AB with SfaNI and polynucleotide CD with BbvI and purify both of them on column;
f-Ligate polynucleotides AB with CD together with SfiI digested pAPG10 and transform it in DH5α E. coli;
g-PCR Screen for polynucleotides having the size of ABCD (8 TALE repeats polynucleotide);
h-Extract the 8 TALE repeats polynucleotide with SfiI and SfaNI restriction enzyme (polynucleotide ABCD) and purify it on column;
i-Repeat steps (a)-(g) using the appropriate plasmids to generate polynucleotide EFGH (with polynucleotide H encoding the last half RVD);
j-Extract the 7.5 TALE repeats polynucleotide with SfiI and BbvI restriction enzyme (polynucleotide EFGH) and purify it on column;
k-Ligate polynucleotides ABCD and EFGH together with SfiI digested pAPG10 and transform ligation products in DH5 α E. coli;
l-Colony PCR screen for polynucleotides having the size of ABCDEFGH (15.5 TALE repeats polynucleotide).

### 1c-Materials and methods

### -Preparation of TALE repeat polynucleotidic modules

To prepare TALE repeat polynucleotidic modules for subsequent assembly, pAPG10 bearing TALE repeat polynucleotidic modules were first digested by Sfil. Digestion mixture consisted in 100 µL of a pAPG10 maxiprep containing the desired module (1µg/µL), 5 µL SfiI (75 U, NEB), 12 µL NEB3 buffer (NEB), 1.2 µL BSA 100 X and 1.8 µL H₂O. Digestion was allowed to proceed for 1 hour at 50°C. Digestion products were separated on a 0.8% agarose gel and SfiI digested TALE repeat polynucleotides were extracted from the gel using Nucleospin extract II kit (Macherey-Nagel) and recovered in 35 µL H₂O. 25µL of extracted TALE repeat polynucleotides were then added to either SfaNI or BbvI digestion mixtures. SfaNI and BbvI digestion mixtures contained 2 µL of restriction enzymes (3U), 3µL of NEB3 or NEB2 10 X respectively and 5 µL H₂O. Digestion was allowed to proceed for 1 hour at 37 °C. Digested products were then column purified using Nucleospin extract II kit (Macherey-Nagel) and recovered in 35 µL H₂O. The overall process generated about 5 µg of digested TALE repeat polynucleotidic module ready for assembly.

### -Assembly of 15.5 TALE repeats polynucleotide

To assemble the 15.5 TALE repeats polynucleotide CAPT1.3 described in this example, the 8 first TALE repeats polynucleotide and the 7.5 last TALE repeats polynucleotide (named respectively 8 TALE repeats left and 7.5 TALE repeat right in figure 4) were first assembled separately and then coupled together according to the method described in figure 3.

### - Assembly of 8 TALE repeats left and 7.5 TALE repeats right polynucleotides

Concerning the assembly of the 8 TALE repeats left polynucleotide, 5µL of SfaNI digested first TALE di repeat module Rvd_b1 (20ng/µL), was first ligated with 5 µL of Bbv I digested Rvd_b16 (20ng/µL) in the presence of 1 µL of T4 DNA ligase (3U, promega) and 10 µL of 2X rapid ligation buffer (Promega). Ligation was allowed to proceed for 1 hour at room temperature. Ligation product was then column purified using the Nucleospin extract II kit (Macherey-Nagel) and eluted in 35 µL H₂O. 10 µL of the purified ligation product Rvd_b1_Rvd_b16 was then added to the SfaNI digestion mixture containing 1 µL SfaNI fast digest (Fermentas) and 10 µL fast digest buffer 2X (Fermentas). Digestion was allowed to proceed for 1 hour at 37°C and the digestion product was then purified using Nucleospin extract II kit (Macherey-Nagel) and eluted in 35 µL H20. In parallel, the third and the fourth TALE di repeat polynucleotide Rvd_b15 and Rvd_b9 were ligated together and the ligation product was purified according to the same protocol. 10µL of the purified ligation product Rvd_b15-Rvd_b9 was then added to the BbvI digestion mixture containing 1 µL BbvI (Fermentas) and 10 µL BbvI digestion buffer 2X (Fermentas). Digestion was allowed to proceed for 1 hour at 65°C and the digestion product was purified using Nucleospin extract II kit (Macherey-Nagel) and eluted in 35 µL H₂0.

To complete the assembly of 8 TALE repeats left polynucleotide, 5µL of SfaNI digested Rvd_b1-Rvd_b16 was added to 5 µL of BbvI digested Rvd_b15-Rvd_b9 in the presence of 1µL of T4 DNA ligase (3U, Promega) in a final volume of 20 µL of rapid ligation buffer 1X (Promega). Ligation was allowed to proceed for 45 min at room temperature. To subclone the 8 TALE repeats left polynucleotide into pAPG10, 5 µL of the ligation product was mixed with 1µL of SfiI digested pAPG10 cut vector (20 ng/µL), 5µL of Rapid ligation buffer and 1µL of T4 DNA ligase (Promega). Ligation was allowed to proceed for 30 min at room temperature.

5µL of this ligation mixture was added to 30 µL of E. coli DH5α chimio competent cells (Invitrogen). Cells were transformed according to the manufacturer guidelines and plated on LB AGAR plates supplemented by ampiciline.

Transformants containing pAPG10 bearing the 8 TALE repeats left polynucleotide were identified by colony PCR screening using M13_F and M13_R as PCR primers (SEQ ID NO: 24-25).

The same procedure was used to generate and select the 7.5 TALE repeats left polynucleotide using Rvd_b1, Rvd_b12 and Rvd_T1

### -Coupling of 8 TALE repeats Left and 7.5 TALE repeats right polynucleotides

To generate the final 15.5 TALE repeats polynucleotide CAPT1.3, the 8 TALE repeats left and 7.5 TALE repeat right polynucleotides were first extracted from pAPG10 and then ligated together.

TALE repeats left and right polynucleotides were extracted from pAPG10 by SfiI digestion and gel purification. To do so, 20 µL of pAPG10 DNA preparation containing either TALE repeats left or right polynucleotides was added to a mixture containing 2 µL SfiI (40U, NEB), 3 µL of BSA 10 X, 3 µL of NEB 4 buffer 10 X (NEB), and 2 µL H₂0. The digestion was allowed to proceed 1 hour at 50 °C. Digestion products were run onto 0.8 % agarose gel and extracted using Nucleospin extract II kit (Macherey-Nagel) and 35 µL H₂0 for sample elution.

10 µL of purified 8 TALE repeats left or 7.5 TALE repeat right polynucleotides were then added to digestion mixtures containing SfaNI or BbvI respectively. Digestion mixtures contained 1µL of restriction enzyme (either SfaNI Fast digest or BbvI, Fermentas) in a final volume of 10 µL of SfaNI or BbvI digestion buffer 2X (Fermentas). Digestion was allowed to proceed for 1 hour. Digestion products were purified using Nucleospin extract II kit (Macherey-Nagel) and eluted in 35 µL H₂O.

5µL of SfaNI digested 8 TALE repeats left polynucleotides were added to 5µL of BbvI digested 7.5 TALE repeats right polynucleotides in the presence of 10 µL of Fast Ligase buffer 2X (Promega) and 1 µL of T4 DNA ligase (Promega). The ligation was allowed to proceed for 1 hour at room temperature. 1 µL of SfiI digested pAPG10 cut vector (20 ng/µL) was then added to the ligation mixture and the reaction was allowed to proceed for 30 min at room temperature. 3 µL of this ligation mixture was added to 30 µL of DH5α chimio competent cells (Invitrogen). Cells were transformed according to the manufacturer guidelines and plated on LB AGAR plates supplemented by ampiciline. Transformants containing pAPG10 bearing the 15.5 TALE repeats polynucleotide CAPT1.3 were identified by colony PCR screening using M13_F and M13_R as PCR primers (SEQ ID NO: 24-25).

### 1d-Assembly of TALE repeats polynucleotides CAPT1.3 and CAPT1.4 (15.5 TALE repeats polynucleotides)

The method described above was used to generate 2 different 15.5 TALE repeats polynucleotide s that recognize the heterodimeric target CAPT1.1 (SEQ ID NO: 32) (figure 4, A). This target is located in the coding sequence of Calpain I small subunit, a protein known to be associated with myotonic dystrophy. CAPT1.1 target (SEQ ID NO: 32) is divided into 3 parts, the CAPT1.3 target (SEQ ID NO: 1), a 15 bp spacer (SEQ ID NO: 33) and the CAPT1.4 target (SEQ ID NO: 2) (Figure 4 A, green, red and cyan respectively). To assemble both TALE repeat polynucleotides, CAPT1.3 (SEQ ID NO: 1) and CAPT1.4 (SEQ ID NO: 2) targets were first translated into RVD motif sequences then into polynucleotidic building blocks encoding polynucleotide modules with appropriate sequences. In this example, polynucleotidic building blocks comprises TALE di repeats modules that were obtained as intermediates of previous synthesis see below (section 1e). These building blocks sequences were separated into two parts named 8 TALE repeats left and 7.5 TALE repeats right. Building blocks were then assembled in parallel to generate left 8 TALE repeats polynucleotide and right 7.5 TALE repeats polynucleotide according to the method described above. Both left and right polynucleotides were subcloned into pAPG10 and transformed into E. coli. Transformation resulted in the growth of more than 1000 colonies on LB plate. Among 24 screened colonies, we obtained about 33% of left 8 TALE repeats polynucleotide or right 7.5 TALE repeats polynucleotide (figure 4B). The correct left 8 TALE repeats or right 7.5 TALE repeats polynucleotides were selected, extracted from pAPG10, ligated together and transformed into E. coli. Again, transformation resulted in the growth of more than 1000 colonies on LB plate and among 24 screened colonies, we obtained about 50% of 15.5 TALE repeats polynucleotides (figure 4C).

### 1e-Generation of libraries of TALE di and tri repeat modules

During assembly of TALE DNA binding domains, assembly intermediates containing TALE di or tri repeats were recovered and used to generate libraries of TALE-encoding building blocks. A complete set of pAPG10 plasmids encoding all the 20 possible TALE di repeat modules and all the 64 possible TALE tri repeat modules (including the last terminal half repeat module) was generated. These libraries of TALE-encoding building blocks were used to assemble custom TALE repeats according to the method described in this example and in examples 2 and 3.

Below in tables 1 to 3 are displayed libraries of mono, di and tri repeat modules.

**Table 1: Library of TALE mono repeat modules**

| TAL repeats name | RVD motif | Targeted Bases |
|---|---|---|
| rvd_m1 | HD | C |
| rvd_m2 | NG | T |
| rvd_m3 | NI | A |
| rvd_m4 | NN | G |
| rvd_m5 | NK | G |
| | | |

| TAL repeats name | RVD motif | Targeted Bases |
|---|---|---|
| rvd_mt | NG# | T |

**Table 2: Library of TALE di repeat modules**

| TAL di repeats name | RVD motives | Targeted Bases |
|---|---|---|
| rvd_b1 | HD-HD | CC |
| rvd_b2 | HD-NG | CT |
| rvd_b3 | HD-NI | CA |
| rvd_b4 | HD-NN | CG |
| rvd_b5 | NG-HD | TC |
| rvd_b6 | NG-NG | TT |
| rvd_b7 | NG-NI | TA |
| rvd_b8 | NG-NN | TG |
| rvd_b9 | NI-HD | AC |
| rvd_b10 | NI-NG | AT |
| rvd_b11 | NI-NI | AA |
| rvd_b12 | NI-NN | AG |
| rvd_b13 | NN-HD | GC |
| rvd_b14 | NN-NG | GT |
| rvd_b15 | NN-NI | GA |
| rvd_b16 | NN-NN | GG |
| rvd_b17 | NG-NK | TG |
| rvd_b18 | HD-NK | CG |
| rvd_b19 | NI-NK | AG |
| rvd_b20 | NK-HD | GC |
| rvd_b21 | NK-NI | GA |
| rvd_b22 | NK-NK | GG |
| rvd_b23 | NK-NG | GT |
| | | |

| TAL di repeats name | RVD motives | Targeted Bases |
|---|---|---|
| rvd_bt1 | HD-NG# | CT |
| rvd_bt2 | NG-NG# | TT |
| rvd_bt3 | NI-NG# | AT |
| rvd_bt4 | NN-NG# | GT |

**Table 3: Library of TALE tri repeat modules**

| TAL tri repeats name | RVD motives | Targeted Bases |
|---|---|---|
| rvd_t1 | HD-HD-HD | CCC |
| rvd_t2 | HD-HD-NG | CCT |
| rvd_t3 | HD-HD-NI | CCA |
| rvd_t4 | HD-HD-NN | CCG |
| rvd_t5 | HD-NG-HD | CTC |
| rvd_t6 | HD-NG-NG | CTT |
| rvd_t7 | HD-NG-NI | CTA |
| rvd_t8 | HD-NG-NN | CTG |
| rvd_t9 | HD-NI-HD | CAC |
| rvd_t10 | HD-NI-NG | CAT |
| rvd_t11 | HD-NI-NI | CAA |
| rvd_t12 | HD-NI-NN | CAG |
| rvd_t13 | HD-NN-HD | CGC |
| rvd_t14 | HD-NN-NG | CGT |
| rvd_t15 | HD-NN-NI | CGA |
| rvd_t16 | HD-NN-NN | CGG |
| rvd_t17 | NG-HD-HD | TCC |
| rvd_t18 | NG-HD-NG | TCT |
| rvd_t19 | NG-HD-NI | TCA |
| rvd_t20 | NG-HD-NN | TCG |
| rvd_t21 | NG-NG-HD | TTC |
| rvd_t22 | NG-NG-NG | TTT |
| rvd_t23 | NG-NG-NI | TTA |
| rvd_t24 | NG-NG-NN | TTG |
| rvd_t25 | NG-NI-HD | TAC |
| rvd_t26 | NG-NI-NG | TAT |
| rvd_t27 | NG-NI-NI | TAA |
| rvd_t28 | NG-NI-NN | TAG |
| rvd_t29 | NG-NN-HD | TGC |
| rvd_t30 | NG-NN-NG | TGT |
| rvd_t31 | NG-NN-NI | TGA |
| rvd_t32 | NG-NN-NN | TGG |
| rvd_t33 | NI-HD-HD | ACC |
| rvd_t34 | NI-HD-NG | ACT |
| rvd_t35 | NI-HD-NI | ACA |
| rvd_t36 | NI-HD-NN | ACG |
| rvd_t37 | NI-NG-HD | ATC |
| rvd_t38 | NI-NG-NG | ATT |
| rvd_t39 | NI-NG-NI | ATA |
| rvd_t40 | NI-NG-NN | ATG |
| rvd_t41 | NI-NI-HD | AAC |
| rvd_t42 | NI-NI-NG | AAT |
| rvd_t43 | NI-NI-NI | AAA |
| rvd_t44 | NI-NI-NN | AAG |
| rvd_t45 | NI-NN-HD | AGC |
| rvd_t46 | NI-NN-NG | AGT |
| rvd_t47 | NI-NN-NI | AGA |
| rvd_t48 | NI-NN-NN | AGG |
| rvd_t49 | NN-HD-HD | GCC |
| rvd_t50 | NN-HD-NG | GCT |
| rvd_t51 | NN-HD-NI | GCA |
| rvd_t52 | NN-HD-NN | GCG |
| rvd_t53 | NN-NG-HD | GTC |
| rvd_t54 | NN-NG-NG | GTT |
| rvd_t55 | NN-NG-NI | GTA |
| rvd_t56 | NN-NG-NN | GTG |
| rvd_t57 | NN-NI-HD | GAC |
| rvd_t58 | NN-NI-NG | GAT |
| rvd_t59 | NN-NI-NI | GAA |
| rvd_t60 | NN-NI-NN | GAG |
| rvd_t61 | NN-NN-HD | GGC |
| rvd_t62 | NN-NN-NG | GGT |
| rvd_t63 | NN-NN-NI | GGA |
| rvd_t64 | NN-NN-NN | GGG |
| | | |

| TAL tri repeat name | RVD motives | Targeted Bases |
|---|---|---|
| rvd_tt1 | HD-HD-NG# | CCT |
| rvd_tt2 | HD-NG-NG# | CTT |
| rvd_tt3 | HD-NI-NG# | CAT |
| rvd_tt4 | HD-NN-NG# | CGT |
| rvd_tt5 | NG-HD-NG# | TCT |
| rvd_tt6 | NG-NG-NG# | TTT |
| rvd_tt7 | NG-NI-NG# | TAT |
| rvd_tt8 | NG-NN-NG# | TGT |
| rvd_tt9 | NI-HD-NG# | ACT |
| rvd_tt10 | NI-NG-NG# | ATT |
| rvd_tt11 | NI-NI-NG# | AAT |
| rvd_tt12 | NI-NN-NG# | AGT |
| rvd_tt13 | NN-HD-NG# | GCT |
| rvd_tt14 | NN-NG-NG# | GTT |
| rvd_tt15 | NN-NI-NG# | GAT |
| rvd_tt16 | NN-NN-NG# | GGT |

### -Example 2

Solid phase assembly of TALE repeat polynucleotidic modules using a parallel sequential process.

### 1a-Method for parallel sequential assembly of TALE repeat polynucleotidic modules using a streptavidin coated well and streptavidin coated magnetic beads as solid phases.

An efficient and highly versatile high throughput method for TALE DNA binding domains was implemented. It consists in a sequential assembly of TALE repeat polynucleotidic modules (constituting a TALE DNA binding domain) comprised in polynucleotidic building blocks on a streptavidin coated solid phase supported by a 96 well plate format. In this method, the first polynucleotidic building block of the TALE repeat polynucleotide to assemble is biotinylated on 5'. This first biotinylated polynucleotidic building block is immobilized onto a streptavidin-coated solid phase (either streptavidin-coated well or magnetic beads) and serves as an anchor for TALE repeat polynucleotidic modules assembly. TALE repeat polynucleotidic modules assembly proceeds through a sequential addition of TALE-encoding building blocks according to the method described in example 1.

### 1a-Sequential and parallel assembly process

We used two types of streptavidin coated solid phases:
- streptavidin coated wells (Thermo fisher)
- streptavidin coated magnetic beads (Ademtech)

The handling of both solid phases is essentially the same. However, there are two main differences between the two, the reaction volume (100 µL for streptavidin coated wells and 50 µL for streptavidin coated magnetic beads), and the need of a magnet for streptavidin coated magnetic beads. As illustrated in figure 5, below is described an example of 15.5 TALE repeats polynucleotide synthesis using building blocks encoding TALE di repeat modules.

This method includes the following steps:
a-Choose the shuttle plasmid containing the first building block encoding the desired TALE di repeat module(polynucleotidic module A), PCR amplify it with TAL_Shuttle_Bio_Forward primer and TAL_Shuttle_Reverse (SEQ ID NO: 29 and 30 respectively), digest it with SfaNI and purify it on column, leading to polynucleotidic module A;
b-Choose the pAPG10 containing the appropriate second, third and fourth building blocks (respectively encoding TALE di repeat polynucleotidic modules B, C, D), extract them from pAPG10 using SfiI according to the method described in example 1 or alternatively, PCR amplify them with TAL_Shuttle_short_Forward primer and TAL_Shuttle_Reverse primer (SEQ ID NO: 31 and 30, figure 5A) and purify them. Digest purified fragments with BbvI and purify them on column leading to polynucleotidic modules B, C and D;
c-Immobilisation of the biotinylated first building block on a streptavidin coated magnetic beads (figure 5B);
d-Ligation of polynucleotidic modules A and B followed by a washing step with a saline buffer to remove secondary reaction products, ligation buffer and enzymes (figure 5C);
e-SfaNI digestion of polynucleotide AB followed by a washing step with a saline buffer to remove secondary reaction products, restriction buffer and enzymes (figure 5C);
f-Repeat steps d to e with BbvI digested polynucleotidic modules C and D (figure 5C);
g-In parallel, repeat steps a to f with polynucleotidic modules E, F, G, H (figure 5C);
h-SfaNI digestion of ABCD polynucleotide and BbvI digestion of EFGH polynucleotide (figure 5C).
i-Recovery of EFGH polynucleotide by column purification and ligation with polynucleotide ABCD still attached to the streptavidin coated solid phase (figure 5C).
j-SfiI digestion, recovery and column purification of ABCDEFGH polynucleotide (figure 5C).
k- Subcloning of ABCDEFGH polynucleotide into SfiI digested pAPG10 and transformation into DH5α E coli (figure 5C).
l-Colony PCR screen for polynucleotide having the size of an hexadeca TALE repeat polynucleotide (figure 5C).

### 1b-Materials and methods

### -PCR amplification and digestion of TALE building blocks

Respectively, the first TALE building blocks of left and right TALE repeat polynucleotides were amplified from pAPG10 using TAL_Shuttle_Bio_Forward and TAL_Shuttle_Reverse primers (SEQ ID NO: 29 and 30 respectively). TAL_Shuttle_Bio_Forward contained a biotinylated moiety that binds specifically to the streptavidin coated solid phase. TALE repeat polynucleotidic modules or TALE repeat polynucleotides used for subsequent assembly could be obtained via the method described in section 1a, and could be also obtained by PCR using TAL_Shuttle_short_Forward and TAL_Shuttle_Reverse primers (SEQ ID NO 31 and 30). Conditions for amplification were 5 ng of pAPG10 containing mono or multiple TALE repeat polynucleotidic modules, 250 µM dNTP mix, 200 nM of each oligonucleotide, 1 µL of Herculase II Fusion DNA Polymerase (Agilent) in a final volume of 50 µL of Herculase buffer 1X. PCR was initiated by a 5 min denaturation at 95°C followed by 30 cycles of 30 sec denaturation at 95°C, 30 sec annealing at 48°C and 20 sec elongation at 72°C. This was followed by a 3 min elongation at 72°C. PCR products were column-purified using nucleospin extract II kit (Macherey-Nalgen), recovered in 40 µL H₂0 and digested by 10 U of either BbvI or SfaNI (NEB), in NEB 2 and 3 buffers, respectively, for 2 hours at 37°C. Digested PCR products were then column-purified using the nucleospin extract II kit (Macherey-Nalgen), recovered in 40 µL H₂0 and quantified using a nanodrop device (Thermo scientific). 2µg of purified digested PCR products were typically obtained with this process.

### -Immobilisation of TALE polynucleotidic building blocks on streptavidin coated solid phase

Two types of streptavidin coated solid phases were used for TALE repeat polynucleotides assembly. These are streptavidin coated magnetic beads (Ademtech) and Streptavidin coated plates (Thermo Scientific). Below are described the methods to immobilize biotinylated DNA on both solid phases.

### -Streptavidin coated magnetic beads

To prepare the streptavidin coated magnetic beads for TALE repeat polynucleotides assembly, 10 µL of streptavidin magnetic beads (Ademtech, Masterbeads streptavidin, 500 nm, ref # 03150) were added to 90 µL PBS 1X, pH7.5 (buffer A) in a 1.5 mL tube (Eppendorff, low binding, DNAase and RNAase free). The mixture was washed 3 times with 100 µL buffer A using the magnet provided by the manufacturer (ref # 20105). Beads were then resuspended by pipetting up and down 3 times (off magnet) with 50 µL of biotinylated SfaNI digested di repeat module (prediluted with buffer A to a suitable concentration, see below) and incubated for 30 min at room temperature. The mixture was then placed onto the magnet to remove the supernatant, beads were washed 2 times with 100 µL of PBS 1X, 1M NaCL, pH7.5 (buffer B) to remove nonspecific binding complexes, and then resuspended in 50 µL of buffer A. At this step, the first biotinylated di repeat module was bound to the beads and the system was ready for subsequent additions of TALE repeat modules.

### -Streptavidin coated plates

To prepare the streptavidin coated plate for TALE repeat polynucleotides assembly, each streptavidin coated well was washed 3 times with 150 µL of buffer A and then incubated 1 hour in the presence of 100 µL of the SfaNI digested biotinylated first polynucleotidic building block (prediluted to a suitable concentration, see below). Wells were then washed 2 times with 150 µL of buffer B to remove nonspecific binding complexes and finally equilibrated in 150 µL of buffer A. At this step, the first biotinylated polynucleotidic building block was bound to the wells and the system was ready for subsequent additions of TALE repeat polynucleotidic modules.

### -Assembly of TALE repeats polynucleotidic modules using a series of consecutive digestion and ligation steps

Here, for the sake of clarity, we only described experimental conditions used with streptavidin coated magnetic beads. The experimental conditions used with streptavidin coated plate are essentially the same as the ones described below except for the reaction volume (100 µL instead of 50 µL).

To assemble the 15.5 TALE repeats polynucleotide described in this example (SADE2.3), the 8 first TALE repeats polynucleotide and the 7.5 last TALE repeats polynucleotide (named respectively 8 TALE repeats left, 7.5 TALE repeat right in the figure 6) were first assembled separately and then ligated together according to the method described in figure 6. Concerning the assembly of the 8 TALE repeats left, 100 ng of biotinylated SfaNI digested first TALE di repeat module, Rvd_b3-biot, was first immobilized according to the protocol described above. Buffer A was discarded from the beads and ligation with the second TALE di repeat module, the Bbv I digested Rvd_b7, was performed by addition of the ligation mixture. This ligation mixture contained 100 ng of Rvd_b7, 1 µL of T4 DNA ligase (3U, Promega) in a final volume of 50 µL of Rapid ligation buffer 1X (Promega). Ligation was allowed to proceed for 1 hour at room temperature and was then stopped by pipetting the ligation mixture out of the beads. Beads were then washed 2 times with 150 µL of buffer B to remove byproducts and enzymes and finally reequilibrated with 150 µL of buffer A. Supernatant was then discarded from the beads and ligation product containing the TALE quadri repeats polynucleotide Rvd_b3-Rvd_b7 was digested by SfaNI. SfaNI digestion mixture contained 1 U SfaNI in a final volume of 50 µL of NEB 3 1X. Digestion was allowed to proceed for 1 hour at 37°C and was then stopped by pipetting the digestion mixture out of the beads. Beads were then washed 2 times with 150 µL of buffer B to remove byproducts and enzymes and finally reequilibrated with 150 µL of buffer A. Two additional consecutive digestion and ligation steps were performed with Rvd_b11 to get the complete 8 TALE repeats left polynucleotide. The 7.5 TALE repeats right polynucleotide was assembled in parallel with the same overall protocol but with higher quantities of building blocks, restriction enzymes and ligase. Indeed we used 300 ng of biotinylated first TALE di repeats module, Rvd_b6-biot and 300 ng of TALE di repeats modules (Rvd_b7, Rvd_b12 and Rvd_T4) for the subsequent restriction/ligation steps. Building block quantities and enzyme units used for TALE repeat assembly are summarized in Table 4.

To obtain the 15.5 final TALE repeats polynucleotide, the 7.5 TALE repeats right polynucleotides were first stripped off the beads by BbvI digestion. BbvI digestion mixture contained 3U of BbvI in a final volume of 50 µL of NEB 2 1X. Digestion was allowed to proceed for 1 hour at 37°C. Supernatant was then recovered, column purified using nucleospin extract II kit (Macherey-Nalgen) and finally recovered in a final volume of 35 µL H₂0. In parallel, the 8 TALE repeats left polynucleotides were digested by SfaNI according to the protocol described above. Beads containing the SfaNI digested 8 TALE repeats left polynucleotides were washed 2 times with 150 µL of buffer B to remove byproducts and enzymes and finally reequilibrated with 150 µL of buffer A. Supernatant was then discarded and a ligation mixture containing 30 µL of BbvI digested 7.5 TALE repeats right polynucleotides, 1µL of T4 DNA Ligase (3U), in a final volume of 50 µL of Rapid ligation buffer 1X (Promega) was added to the beads. Ligation of TALE repeats Left and Right polynucleotides was allowed to proceed for 1 hour at room temperature and was then stopped by pipetting the ligation mixture out of the beads. Beads were then washed 2 times with 150 µL of buffer B to remove byproducts and enzymes and finally reequilibrated with 150 µL of buffer A. At this stage of the process, the 15.5 TALE repeats polynucleotides were assembled, but still attached to the streptavidin coated beads.

**Table 4: Building block quantities and enzyme units used for TALE repeat assembly**

| 8 TALE repeats left assembly | | | | |
|---|---|---|---|---|
| Di repeat module name | Rvd_b3-biot | Rvd_b7 | Rvd_b11 | Rvd_b11 |
| Quantity (ng) | 100 | 100 | 100 | 100 |
| Ligase (U) | 3 | 3 | 3 | - |
| SfaNI (U) | - | 1 | 1 | 1 |

| 7.5 TALE repeats right assembly | | | | |
|---|---|---|---|---|
| Di repeat module name | Rvd_b6-biot | Rvd_b7 | Rvd_b12 | Rvd_T4 |
| Quantity (ng) | 300 | 300 | 300 | 300 |
| Ligase (U) | 9 | 9 | 9 | - |
| SfaNI (U) | - | 3 | 3 | - |
| BbvI | - | - | - | 3 |

### -Recovery of TALE repeats fragment and subcloning into pAPG10 shuttle vector

Recovery of the 15.5 TALE repeats polynucleotides was performed by SfiI digestion. SfiI digestion mixture containing 20 U Sfil and BSA 1X in a final volume of 100 µL of NEB buffer 4 1X, was added to the beads. The reaction was allowed to proceed for 1 hour at 50 °C. Supernatant was recovered, column purified using nucleospin extract II kit (Macherey-Nalgen) and finally recovered in a final volume of 35 µL H₂0. To subclone the 15.5 TALE repeats polynucleotides into pAPG10, 5µL of the purified solution was added to 5µL of Rapid ligation buffer 2X (Promega), 1µL of SfiI digested pAPG10 cut vector (20ng) and 1µL of T4 DNA ligase (3U, Promega). Ligation was allowed to proceed for 1 hour at room temperature. 5µL of ligated products were then used to transform 30 µL of DH5α chimio competent cells (Invitrogen) according to the manufacturer protocol.

### 1d- Assembly of TALE repeats polynucleotide SADE2.3 (15.5 TALE repeats polynucleotide) using streptavidin coated magnetic beads as solid phase.

The method described above was used to generate a 15.5 TALE repeats polynucleotide that binds to the SADE2.3 homodimeric target sequence (SEQ ID NO: 3) (figure 6A). SADE2.3 is divided into 3 parts, the SADE2.3 target (SEQ ID NO: 34), a 21 bp spacer (SEQ ID NO: 35) and a second SADE2.3 target (SEQ ID NO: 34) (figure 6A, green, red and green respectively). To assemble the 15.5 TALE repeats polynucleotide, SADE2.3 target was first divided into two parts named 8 TALE repeats left and 7.5 TALE repeats right, translated into RVD motif sequences and then into TALE polynucleotidic building blocks comprising di repeats modules sequences. TALE Di repeats polynucleotidic modules were then assembled in parallel to generate 8 TALE repeats left and 7.5 TALE repeats right according to the method described above (see section 1a). The TALE repeats left and right were then digested by SfaNI and BbvI respectively and eventually coupled together to generate the final 15.5 TALE repeats polynucleotide. This final fragment was then stripped off the solid phase by SfiI digestion, recovered by column purification, subcloned into pAPG10 and transformed into E. coli. Transformation resulted in the growth of more than 1000 colonies on LB plate. Among 24 screened colonies, we obtained around 50% of 15.5 TALE repeats polynucleotides (figure 6B). This success rate was independent on the solid phase used for the assembly (data not shown).

### -Example 3

Solid phase assembly of TALE repeat polynucleotidic modules using a linear sequential process.

### 1a-Method for linear sequential assembly of TALE repeat polynucleotidic modules using a streptavidin coated well and streptavidin coated magnetic beads as solid phases.

Below is described an example of 15.5 TALE repeats polynucleotide assembly using building blocks encoding TALE di repeats modules and streptavidin coated plates as a solid phase. This method includes the following steps:
a-Choose a shuttle plasmid containing the first building block encoding the desire TALE di repeat module (polynucleotidicmodule A), PCR amplify it with TAL_Shuttle_Bio_Forward primer and TAL_Shuttle_Reverse primer (SEQ ID NO: 29 and 30 respectively), SfaNI digestion of the PCR product and purification on column, leading to polynucleotide module A.
b-Choose the shuttle containing the next building blocks needed for assembly (respectively encoding TALE di repeat polynucleotidic modules B, C, D, E, F, G), PCR amplify them with TAL_Shuttle_short_Forward primer and TAL_Shuttle_Reverse primer (SEQ ID NO 31 and 30 respectively), digest them with BbvI and purify them on column. Alternatively, these fragments can be extracted from pAPG10 using Sfil according to the method described in example 1.
c-Immobilisation of the biotinylated first building block on a streptavidin coated well.
d-Ligation of polynucleotidic modules A and B followed by a washing step with a saline buffer to remove secondary reaction products, ligation buffer and enzymes.
e-SfaNI digestion of polynucleotide AB followed by a washing step with a saline buffer to remove secondary reaction products, restriction buffer and enzymes.
f-Repeat steps d to e with BbvI digested polynucleotidic modules C, D, E, F, G and H
g- Sfil digestion of ABCDEFGH polynucleotide.
i-Recovery of ABCDEFGH polynucleotide by column purification, subcloning into SfiI digested pAPG10 and transformation into DH5α E coli.
1-Colony PCR screen for fragment having the size of an 15.5 TALE repeat polynucleotide.

### 1b-Material and methods

### -PCR amplification and digestion of TALE building blocks

The first TALE building block of TALE repeat polynucleotide was amplified from pAPG10 using TAL_Shuttle_Bio_Forward and TAL_Shuttle_Reverse primers (SEQ ID NO: 29 and 30 respectively). TAL_Shuttle_Bio_Forward contains a biotinylated moiety that binds specifically to the streptavidin coated solid phase. TALE repeat polynucleotidic modules or TALE repeat polynucleotides used for subsequent assembly could be obtained via the method described in section 1a, and could be also obtained by PCR using TAL_Shuttle_short_Forward and TAL_Shuttle_Reverse primers (SEQ ID NO 31 and 30). Conditions for amplification were 5 ng of pAPG10 containing mono or multiple TALE repeat polynucleotidic modules, 250 µM dNTP mix, 200 nM of each oligonucleotide, 1 µL of Herculase II Fusion DNA Polymerase (Agilent) in a final volume of 50 µL of Herculase buffer 1X. PCR was initiated by a 5 min denaturation at 95°C followed by 30 cycles of 30 sec denaturation at 95°C, 30 sec annealing at 48°C and 20 sec elongation at 72°C. This was followed by a 3 min elongation at 72°C. PCR products were column purified using nucleospin extract II kit (Macherey-Nalgen), recovered in 40 µL H₂0 and digested by 10 U of either BbvI or SfaNI (NEB), in NEB 2 and 3 buffers respectively, for 2 hours at 37°C. Digested PCR products were then column purified using the nucleospin extract II kit (Macherey-Nalgen), recovered in 40 µL H₂0 and quantified using a nanodrop device (Thermo scientific). 2µg of purified digested PCR products were typically obtained with this process.

### -Immobilisation of TALE polynucleotidic building blocks on streptavidin coated solid phase

Two types of streptavidin coated solid phases were used for TALE repeat polynucleotides assembly. These are streptavidin coated magnetic beads (Ademtech) and Streptavidin coated plates (Thermo Scientific). Below are described the methods to immobilize biotinylated DNA on both solid phases.

### -Streptavidin coated magnetic beads

To prepare the streptavidin coated magnetic beads for TALE repeat polynucleotides assembly, 10 µL of streptavidin magnetic beads (Ademtech, Masterbeads streptavidin, 500 nm, ref # 03150) were added to 90 µL PBS 1X, pH7.5 (buffer A) in a 1.5 mL tube (Eppendorff, low binding, DNAase and RNAase free). The mixture was washed 3 times with 100 µL buffer A using the magnet provided by the manufacturer (ref # 20105). Beads were then resuspended by pipetting up and down 3 times (off magnet) with 50 µL of biotinylated SfaNI digested di repeat module (prediluted with buffer A to a suitable concentration, see below) and incubated for 30 min at room temperature. The mixture was then placed onto the magnet to remove the supernatant, beads were washed 2 times with 100 µL of PBS 1X, 1M NaCL, pH7.5 (buffer B) to remove nonspecific binding complexes, and then resuspended in 50 µL of buffer A. At this step, the first biotinylated di repeat module was bound to the beads and the system was ready for subsequent additions of TALE repeat modules.

### -Streptavidin coated plates

To prepare the streptavidin coated plate for TALE repeat polynucleotides assembly, each streptavidin coated well was washed 3 times with 150 µL of buffer A and then incubated 1 hour in the presence of 100 µL of the SfaNI digested biotinylated first polynucleotidic building block (prediluted to a suitable concentration, see below). Wells were then washed 2 times with 150 µL of buffer B to remove nonspecific binding complexes and finally equilibrated in 150 µL of buffer A. At this step, the first biotinylated polynucleotidic building block encoding di repeat module was bound to the wells and the system was ready for subsequent additions of TALE repeat polynucleotidic modules.

### -Assembly of TALE polynucleotidic repeats modules using a series of consecutive digestion and ligation steps

Here, we only described experimental conditions used with streptavidin coated magnetic beads. The experimental conditions used with streptavidin coated plate are essentially the same as the ones described below except for the reaction volume (100 µL instead of 50 µL).

The 15.5 TALE repeats polynucleotide described in this example (SADE2.3) was assembled sequentially in a linear fashion. To do so, 100 ng of biotinylated SfaNI digested first TALE di repeat module, Rvd_b3-biot, was first immobilized on magnetic beads according to the protocol described above. Buffer A was discarded from the beads and ligation with the second TALE di repeat module, the Bbv I digested Rvd_b7, was performed by addition of the ligation mixture. This ligation mixture contained 100 ng of Rvd_b7, 1 µL of T4 DNA ligase (3U, Promega) in a final volume of 50 µL of Rapid ligation buffer 1X (Promega). Ligation was allowed to proceed for 1 hour at room temperature and was then stopped by pipetting the ligation mixture out of the beads. Beads were then washed 2 times with 150 µL of buffer B to remove byproducts and enzymes and finally reequilibrated with 150 µL of buffer A. Supernatant was then discarded from the beads and ligation product containing the TALE quadri repeats polynucleotide Rvd_b3-Rvd_b7 was digested by SfaNI. SfaNI digestion mixture contained 1 U SfaNI in a final volume of 50 µL of NEB 3 1X. Digestion was allowed to proceed for 1 hour at 37°C and was then stopped by pipetting the digestion mixture out of the beads. Beads were then washed 2 times with 150 µL of buffer B to remove byproducts and enzymes and finally reequilibrated with 150 µL of buffer A. Six additional digestion/ligation steps were performed with Rvd_b11, Rvd_b6, Rvd_b7, Rvd_b12, and Rvd_T4 to get the complete 15.5 TALE repeats polynucleotide. Building block quantities and enzyme units used for repeat assembly are summarized in Table 4.

At this stage of the process, the 15.5 TALE repeats polynucleotides were assembled, but still attached to the streptavidin coated beads.

### -Recovery of TALE repeats fragment and subcloning into pAPG10 shuttle vector

Recovery of the 15.5 TALE repeats polynucleotides was performed by SfiI digestion. SfiI digestion mixture containing 20 U SfiI and BSA 1X in a final volume of 100 µL of NEB buffer 4 1X, was added to the beads. The reaction was allowed to proceed for 1 hour at 50 °C. Supernatant was recovered, column purified using nucleospin extract II kit (Macherey-Nalgen) and finally recovered in a final volume of 35 µL H₂0. To subclone the 15.5 TALE repeats polynucleotides into pAPG10, 5µL of the purified solution was added to 5µL of Rapid ligation buffer 2X (Promega), 1µL of SfiI digested pAPG10 cut vector (20ng) and 1µL of T4 DNA ligase (3U, Promega). Ligation was allowed to proceed for 1 hour at room temperature. 5µL of ligated products were then used to transform 30 µL of DH5α chimio competent cells (Invitrogen) according to the manufacturer protocol.

### 1d- Assembly of TALE repeats polynucleotide SADE2.3 (15.5 TALE repeats polynucleotide) using streptavidin coated plates or streptavidin coated magnetic beads as a solid phase.

The method described above was used to generate a 15.5 TALE repeats TALE repeats polynucleotide that recognizes the homodimeric target SADE2.3 (SEQ ID NO: 3) (figure 7A). SADE2.3 is divided into 3 parts, the SADE2.3 target (SEQ ID NO: 34), a 21 bp spacer (SEQ ID NO: 35) and a second SADE2.3 target (SEQ ID NO: 34) (figure 7A, green, red and green respectively). To assemble TALE repeats polynucleotide targeting SADE2.3, SADE2.3 target was first translated into a RVD motif sequence and then into TALE polynucleotidic building blocks comprising di repeats modules sequence. TALE di repeats polynucleotidic modules were then assembled sequentially to generate a 15.5 TALE repeats polynucleotide according to the method described in section 1a. The 15.5 TALE repeats polynucleotide was subcloned into pAPG10 and transformed into E. coli. Transformation resulted in the growth of more than 1000 colonies on LB plate.

Variable success rates for the assembly of 15.5 TALE repeats polynucleotides were obtained depending on thesolid phase used for assembly (streptavidin coated well or streptavidin coated magnetic beads). Around 20% of 15.5 TALE repeats polynucleotides were obtained with streptavidin coated magnetic beads (figure 7B) and 50% of 15.5 TALE repeats polynucleotide were obtained with streptavidin coated well (figure 7C)

### 1e- Assembly of AvrBs3 TALE repeats polynucleotides variant (17 TALE repeats polynucleotide) using streptavidin coated plates as a solid phase.

The method described above (section 1a) was used to generate a 17 TALE repeats polynucleotide that recognizes the homodimeric target AvrBs3 (SEQ ID NO: 36) (figure 8A). AvrBs3 target is divided into 3 parts, the AvrBs3.3 target (SEQ ID NO: 37), a 15 bp spacer (SEQ ID NO: 38) and a second AvrBs3.3 target (SEQ ID NO: 37) (figure 8A, green, red and green respectively). To assemble TALE repeats polynucleotides targeting AvrBs3, AvrBs3 target was first translated into a RVD motif sequence and then into TALE polynucleotidic building blocks comprising di and tri repeats modules sequence. TALE tri repeats polynucleotidic modules were then sequentially assembled to generate a 17 TALE repeats polynucleotide according to the method described in section 1a. The 17 TALE repeats polynucleotide was subcloned into pAPG10 and transformed into E. coli. Transformation resulted in the growth of more than 1000 colonies on LB plate. Among 20 PCR screened colonies, around 40% of AvrBs3 17 TALE repeats polynucleotides (figure 8B) were obtained.

### -Example 4: Improvement of TALE repeat polynucleotidic modules assembly success rate by dual immobilization procedures

As illustrated in figure 9, below is described a method to increase the success rate of TALE repeat polynucleotidic modules assembly. This method consists in a sequential assembly of TALE polynucleotidic modules comprised in polynucleotidic building blocks, assisted by two different solid phases. The first one is coated by streptavidin and is specific for biotinylated DNA fragments whereas the second one is coated by digoxigenin specific antibodies and is specific for digoxigeninylated DNA fragments. Using a combination of two different solid phases enables enrichment of the proper nascent TALE repeat polynucleotides and depletion of improper ones at each assembly step.

A method to assemble TALE polynucleotidic modules comprised in polynucleotidic building blocks was designed according to the following protocol. It should be noted that each TALE building block may contain multiple TALE repeat modules obtained from previous assemblies. Below is described an assembly example of 3 TALE polynucleotidic modules comprised in building blocks using the dual immobilization procedure. Complete assembly of a TALE DNA binding domain is not described here.
a-Immobilization of the first desired SfaNI digested biotinylated building block on a streptavidin coated well (building block A)
b-Ligation of polynucleotide module A with a second BbvI digested digoxigeninylated building block
c-Wash out the unbound polynucleotidic module B with a washing buffer
d-BbvI digestion of polynucleotide AB, recovery of supernatant and immobilization of digested polynucleotide onto a new well coated by digoxigenin specific antibodies
e-Ligation of BbvI digested polynucleotide AB with a third SfaNI biotinylated building block.

### -Example 5: Method for assembly of polynucleotidic building blocks by reverse elongation

### Example 5a: Preparation of building blocks repeat for assembly by reverse elongation

Using reverse elongation on solid phases enables enrichment of the proper nascent TALE repeat polynucleotides and permit high throughput synthesis (HTS) of these molecules. A method to assemble TALE polynucleotidic modules comprised in polynucleotidic building blocks was designed according to the following protocol. Each TALE building block may contain multiple TALE repeat modules obtained from previous assemblies. Below is described how to prepare TALE building blocks using a reverse elongation procedure.

A TALE building block of TALE repeat polynucleotide was amplified from pAPG10 using TAL_Shuttle_Bio_Forward and TAL_Shuttle_Reverse_short primer (SEQ ID NO: 29 and 39, respectively). TAL_Shuttle_Bio_Forward contains a biotinylated moiety that binds specifically to the streptavidin coated solid phase. The last TALE repeat polynucleotidic modules or TALE repeat polynucleotides used for subsequent assembly could be obtained via the method described in example 1, section 1a, and could be also obtained by PCR using TAL_Shuttle_short_Forward and TAL_Shuttle_Reverse primers (SEQ ID NO 31 and 30). Conditions for amplification were 5 ng of pAPG10 containing mono or multiple TALE repeat polynucleotidic modules, 250 µM dNTP mix, 200 nM of each oligonucleotide, 1 µL of Herculase II Fusion DNA Polymerase (Agilent) in a final volume of 50 µL of Herculase buffer 1X. PCR was initiated by a 5 minutes denaturation at 95°C followed by 30 cycles of 30 sec. denaturation at 95°C, 30 sec. annealing at 48°C and 30-45 sec. elongation at 72°C. This was followed by a 3 min. elongation at 72°C. PCR products were column purified using nucleospin extract II kit (Macherey-Nalgen), recovered in 40 µL H₂O and digested by 10 U of either BbvI or SfaNI (NEB), in Fast digest buffers for 1 hour at 37°C. Digested PCR products were then column purified using the nucleospin extract II kit (Macherey-Nalgen), recovered in 40 uL H₂O and quantified using a nanodrop device (Thermo scientific). 1-2µg of purified digested PCR products were typically obtained with this process by PCR.

### -Example 5b: TALE repeat polynucleotidic modules assembly by reverse elongation

As illustrated in figure 10, below is described a method to assemble TALE repeat polynucleotidic modules. This method consists in a sequential assembly of TALE polynucleotidic modules comprised in polynucleotidic building blocks, assisted by a solid phase. The solid phase is coated by streptavidin and is specific for biotinylated DNA fragments prepared as described in examples 2 and 3. Below is described an assembly example of TALE DNA binding domain using a reverse elongation procedure.

The following example is given for the synthesis of a 13.5 blocks polynucleotidic module, using tri-modules (A, B, C, D) and a terminal di-module (1.5 RVDs) (E), but can be adapted to the synthesis of any repeat polynucleotides using various starting modules (Prior to any assembly of polynucleotidic modules, the solid surfaces (streptavidin-coated wells) are prepared by two washing steps with 200 µl of PBS 1x):
1-Immobilization of the desired SfaNI digested biotinylated building block on a streptavidin-coated well (building block D, 300 ng in PBS 1x, 100 µl final volume) 1 hour at room temperature.
2-Ligation of BbvI digested polynucleotide terminal module E (270 ng) in 1x ligation buffer containing 4 µl of T4 DNA ligase in a final volume of 100 µl. After 30 minutes incubation at room temperature, 0.5 mM final concentration of ATP was added and the reaction was set for an additional 30 minutes at room temperature.
**3**-Wash out the unligated polynucleotidic terminal module E with three times 150 µl PBS 1x, NaCl 1M, followed by two times 170 µl PBS 1x.
**4**-Release of nascent chain (DE) from the solid surface by addition of BbvI (0.5 µl Fast digest BbvI, fermentas #FD2074) in 1x Fast digestion buffer (Fermentas) in a final volume of 100 µl, followed by thermal inactivation (65°C, 20 minutes) of the restriction enzyme. The reaction was subsequently cooled down to room temperature.
**5**-Immobilization of the desired SfaNI digested biotinylated building block on a streptavidin coated well (building block C, 250 ng in PBS 1x, 100 µl final volume) 1 hour at room temperature.
**6**-Ligation of the nascent chain (DE) to the immobilized block C by addition of the reaction mixture (step **4**) complemented with 1x ligation buffer and 4 µl of T4 DNA ligase to the immobilized block C (step **5**). After 30 minutes incubation at room temperature, 0.5 mM final concentration of ATP was added and the reaction was set for an additional 30 minutes at room temperature.
7-Wash out the unligated polynucleotidic module DE with three times 150 µl PBS 1x, NaCl 1M, followed by two times 170 µl PBS 1x.
**8**-Release of nascent chain (CDE) from the solid surface by addition of BbvI (0.5 µl Fast digest BbvI, fermentas #FD2074) in 1x Fast digestion buffer (Fermentas) in a final volume of 100 µl, followed by thermal inactivation (65°C, 20 minutes) of the restriction enzyme. The reaction was subsequently cooled down to room temperature.
**9**-Immobilization of the desired SfaNI digested biotinylated building block on a streptavidin-coated well (building block B, 200 ng in PBS 1x, 100 µl final volume) 1 hour at room temperature.
**10**-Ligation of the nascent chain (CDE) to the immobilized block B by addition of the reaction mixture (step **8**) complemented with 1x ligation buffer and 4 µl of T4 DNA ligase to the immobilized block B (step **9**). After 30 minutes incubation at room temperature, 0.5 mM final concentration of ATP was added and the reaction was set for an additional 30 minutes at room temperature.
**11**-Wash out the unligated polynucleotidic module CDE with three times 150 µl PBS 1x, NaCl 1M, followed by two times 170 µl PBS 1x
**12**-Release of nascent chain (BCDE) from the solid surface by addition of BbvI (0.5 µl Fast digest BbvI, fermentas #FD2074) in 1x Fast digestion buffer (Fermentas) in a final volume of 100 µl, followed by thermal inactivation (65°C, 20 minutes) of the restriction enzyme. The reaction was subsequently cooled down to room temperature.
**13**-Immobilization of the desired SfaNI digested biotinylated building block on a streptavidin coated well (building block A, 150 ng in PBS 1x, 100 µl final volume) 1 hour at room temperature.
**14**-Ligation of the nascent chain (BCDE) to the immobilized block A by addition of the reaction mixture (step **12**) complemented with 1x ligation buffer and 4 µl of T4 DNA ligase to the immobilized block A (step **13**). After 30 minutes incubation at room temperature, 0.5 mM final concentration of ATP was added and the reaction was set for an additional 30 minutes at room temperature.
**15**-Wash out the unligated polynucleotidic module BCDE with three times 150 µl PBS 1x, NaCl 1M, followed by two times 170 µl PBS 1x.
16-Release of the synthesized chain (ABCDE) with either SfiI to allow subcloning in the shuttle (pAPG10) plasmid or by sequential SfaNI, wash, BbvI digestions to allow subcloning in plasmids already containing a TALEN scaffold (e.g. pCLS7183, SEQ ID NO: 20) is followed by transformation in E. coli according to standard molecular biology procedures.

It is understood that washing steps can be done respectively after a given immobilization step of a polynucleotides modules or blocks, according to the state of the art; as a nonlimiting example, three washing steps of an appropriate volume of saline buffer, such as PBS, can be added after a given immobilization step.

Analyses by PCR screening (figure 11) reveal a frequency compatible with high throughput synthesis procedures.

In addition the following steps **17, 18** and **19** can be added after steps **1, 5, 9** and/or **13** to combine advantages of both, reverse elongation and direct linear sequential synthesis.
**17**-Ligation of any BbvI digested polynucleotide module X in 1x ligation buffer containing 4 µl of T4 DNA ligase in a final volume of 100 µl. After 30 minutes incubation at room temperature, 0.5 mM final concentration of ATP was added and the reaction was set for an additional 30 minutes at room temperature.
**18**-Digestion of the bound product using SfaNI (1 µl Fast digest BbvI, fermentas #FD2124) in 1x Fast digestion buffer (Fermentas) in a final volume of 100 µl. **19**-Wash out the restriction enzyme and buffer with three times 150 µl PBS 1x, NaCl 1M, followed by two times 170 µl PBS 1x

The case where steps **17, 18** and **19** are added after steps **5** is represented in figure 12.

### Other embodiments

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

### List of cited references

Boch, J. and U. Bonas (2010). "Xanthomonas AvrBs3 family-type III effectors: discovery and function." Annu Rev Phytopathol 48: 419-36.
Boch, J., H. Scholze, et al. (2009). "Breaking the code of DNA binding specificity of TAL-type III effectors." Science 326(5959): 1509-12.
Carroll, D. (2008). "Progress and prospects: zinc-finger nucleases as gene therapy agents." Gene Ther 15(22): 1463-8.
Cermak, T., E. L. Doyle, et al. (2011). "Efficient design and assembly of custom TALEN and other TAL effector-based constructs for DNA targeting." Nucleic Acids Res.
Chevalier, B., D. Sussman, et al. (2004). "Metal-dependent DNA cleavage mechanism of the I-Crel LAGLIDADG homing endonuclease." Biochemistry 43(44): 14015-26.
Choo, Y., I. Sanchez-Garcia, et al. (1994). "In vivo repression by a site-specific DNA-binding protein designed against an oncogenic sequence." Nature 372(6507): 642-5.
Christian, M., T. Cermak, et al. (2010). "Targeting DNA double-strand breaks with TAL effector nucleases." Genetics 186(2): 757-61.
Engler, C., R. Kandzia, et al. (2008). "A one pot, one step, precision cloning method with high throughput capability." PLoS One 3(11): e3647.
Feng, X., A. L. Bednarz, et al. (2010). "Precise targeted integration by a chimaeric transposase zinc-finger fusion protein." Nucleic Acids Res 38(4): 1204-16.
Geissler, R., H. Scholze, et al. (2011). "Transcriptionnal Activators of Human genes with progammable DNA-specificity." PLoS ONE 6(5): e19509.
Gersbach, C. A., T. Gaj, et al. (2010). "Directed evolution of recombinase specificity by split gene reassembly." Nucleic Acids Res.
Grizot, S., A. Duclert, et al. "Context dependence between subdomains in the DNA binding interface of the I-Crel homing endonuclease." Nucleic Acids Res.
Isalan, M., A. Klug, et al. (2001). "A rapid, generally applicable method to engineer zinc fingers illustrated by targeting the HIV-1 promoter." Nat Biotechnol 19(7): 656-60.
Kim, Y. G., J. Cha, et al. (1996). "Hybrid restriction enzymes: zinc finger fusions to Fok I cleavage domain." Proc Natl Acad Sci USA 93(3): 1156-60.
Li, T., S. Huang, et al. (2011). "TAL nucleases (TALNs): hybrid proteins composed of TAL effectors and Fokl DNA-cleavage domain." Nucleic Acids Res 39(1): 359-72.
Li, T., S. Huang, et al. (2011). "Modularly assembled designer TAL effector nucleases for targeted gene knockout and gene replacement in eukaryotes." Nucleic Acids Res.
Mahfouz, M. M., L. Li, et al. (2011). "De novo-engineered transcription activator-like effector (TALE) hybrid nuclease with novel DNA binding specificity creates double-strand breaks." Proc Natl Acad Sci U S A 108(6): 2623-8.
Miller, J. C., S. Tan, et al. (2010). "A TALE nuclease architecture for efficient genome editing." Nat Biotechnol 29(2): 143-8.
Morbitzer, R., J. Elsaesser, et al. (2011). "Assembly of custom TALE-type DNA binding domains by modular cloning." Nucleic Acids Res.
Moscou, M. J. and A. J. Bogdanove (2009). "A simple cipher governs DNA recognition by TAL effectors." Science 326(5959): 1501.
Pabo, C. O., E. Peisach, et al. (2001). "Design and selection of novel Cys2His2 zinc finger proteins." Annu Rev Biochem 70: 313-40.
Ramirez, C. L., J. E. Foley, et al. (2008). "Unexpected failure rates for modular assembly of engineered zinc fingers." Nat Methods 5(5): 374-5.
Silva, G., L. Poirot, et al. (2011). "Meganucleases and other tools for targeted genome engineering: perspectives and challenges for gene therapy." Curr Gene Ther 11(1): 11-27.
Smith, J., J. M. Berg, et al. (1999). "A detailed study of the substrate specificity of a chimeric restriction enzyme." Nucleic Acids Res 27(2): 674-81.
Smith, J., M. Bibikova, et al. (2000). "Requirements for double-strand cleavage by chimeric restriction enzymes with zinc finger DNA-recognition domains." Nucleic Acids Res 28(17): 3361-9.
Spear, M. A. (2000). "Efficient DNA subcloning through selective restriction endonuclease digestion." Biotechniques 28(4): 660-2, 664, 666 passim.
Stoddard, B. L. (2005). "Homing endonuclease structure and function." Q Rev Biophys 38(1): 49-95.
Stoddard, B. L. (2011). "Homing endonucleases: from microbial genetic invaders to reagents for targeted DNA modification." Structure 19(1): 7-15.
Urnov, F. D., E. J. Rebar, et al. (2011). "Genome editing with engineered zinc finger nucleases." Nat Rev Genet 11(9): 636-46.
Weber, E., C. Engler, et al. (2011). "A modular cloning system for standardized assembly of multigene constructs." PLoS One 6(2): e16765.
Zhang, F., L. Cong, et al. (2011). "Efficient construction of sequence-specific TAL effectors for modulating mammalian transcription." Nat Biotechnol 29(2): 149-53.

## Claims

1. A method of generating and assembling polynucleotides comprising arrays of at least two transcription activator-like effector (TALE) DNA binding repeat polynucleotide modules which each encodes for 30 to 42 amino acids, preferably for 33 to 35 amino acids comprising the steps of:
a) Generating "n" polynucleotide building blocks, each comprising at least:
- one TALE DNA binding repeat polynucleotide module;
- a single cleavage site for a first type IIS restriction enzyme A, placed on one side of the polynucleotide module;
- a single cleavage site for a second type IIS restriction enzyme B, placed on the other side of the polynucleotide module;
- wherein A and B can produce compatible cohesive ends;
- wherein cleavage of each of said polynucleotide building blocks with restriction enzyme A results in a polynucleotide comprising a polynucleotide module flanked on one side by a cohesive end that can be re-ligated with a polynucleotide building block cleaved by restriction enzyme B without restoring a sequence cleavable by restriction enzyme A and/or B;
- wherein cleavage of each of said polynucleotide building blocks with restriction enzyme B results in a polynucleotide comprising a polynucleotide module flanked on one side by a cohesive end that can be re-ligated with a polynucleotide building block cleaved by restriction enzyme A without restoring a sequence cleavable by restriction enzyme A and/or B;
b) Generating "n" polynucleotides linked to a solid phase by: b1) linking each of the polynucleotide building blocks generated according to step a) to a solid phase so that the cleavage site for the first restriction enzyme A is placed on the side of the polynucleotide module that is linked to the solid phase and b2) cleaving said polynucleotide building block with restriction enzyme B;
c) Generating one C-terminal polynucleotide building block comprising at least:
- one polynucleotide module;
- a single cleavage site for a first restriction enzyme A, placed on one side of the polynucleotide module;
- a single cleavage site for a second restriction enzyme B, placed on the other side of the polynucleotide module;
wherein cleavage of said C-terminal polynucleotide building block with restriction enzyme A results in a polynucleotide comprising a polynucleotide module flanked on one side by a cohesive end that can be re-ligated with a polynucleotide building block of step a) cleaved by restriction enzyme B without restoring a sequence cleavable by restriction enzyme A; and
wherein cleavage of said C-terminal polynucleotide building block with restriction enzyme B results in a polynucleotide comprising a polynucleotide module flanked on one side by a cohesive end that cannot be re-ligated with a polynucleotide building block of step a) cleaved by restriction enzyme A and/or B;
d) Cutting said one C-terminal polynucleotide building block of c) with restriction enzyme A;
e) Ligating the resulting C-terminal polynucleotide module with the free end of one polynucleotide of b) immobilized on a solid phase, thereby producing a new immobilized polynucleotide comprising one additional polynucleotide module;
f) Cutting the resulting new immobilized polynucleotide with restriction enzyme A, thus producing a new polynucleotide having a free end compatible with the cohesive ends resulting from cleavage of a polynucleotide building block of step a) with restriction enzyme B;
g) Ligating the new polynucleotide with the free end of one polynucleotide of b) immobilized on a solid phase thus producing a new immobilized polynucleotide comprising one additional polynucleotide module.

2. The method according to claim 1 wherein steps f) and g) are repeated N times to produce an immobilized polynucleotide having an array of n TALE DNA binding repeat polynucleotide modules wherein n = N+3.

3. The method of claim 2 wherein said immobilized polynucleotide has between 8 and 20 TALE DNA binding repeat polynucleotide modules.

4. The method according to any one claims 1 to 3 wherein step g) is replaced by the following steps:
g') Cutting said at least one polynucleotide building block of a) with restriction enzyme A;
h) Ligating the resulting polynucleotide module with the free end of one polynucleotide of b) immobilized on a solid phase, thereby producing a new immobilized polynucleotide comprising one additional polynucleotide module;
i) Cutting the resulting new immobilized polynucleotide with restriction enzyme B, thus producing a new immobilized polynucleotide having a free end compatible with the cohesive ends resulting from cleavage with restriction enzyme A;
j) Ligating on the new produced immobilized polynucleotide said new polynucleotide resulting from step f) thereby producing a new immobilized polynucleotide comprising "x" additional polynucleotide modules wherein "x" is equal to the number of polynucleotide modules present in said new polynucleotide resulting from step f).

5. A method according to claim 4 further comprising the steps:
k) Cutting the resulting new immobilized polynucleotide of step j) with restriction enzyme A, thus producing a new polynucleotide having a free end compatible with the cohesive ends resulting from cleavage of a polynucleotide building block of step a) with restriction enzyme B;
l) Ligating the new polynucleotide with the free end of one polynucleotide of b) immobilized on a solid phase thus producing a new immobilized polynucleotide comprising one additional polynucleotide module.

6. The method according to any one of claims 1 to 5 wherein at least one said polynucleotide building block of a) and / or at least one polynucleotide linked to a solid phase of b) and / or said polynucleotide building block of c) comprise a pre-assembly of more than one TALE DNA binding repeat polynucleotide module.

7. The method according to any one of claims 1 to 6 wherein at least one said polynucleotide building block of a) and / or at least one polynucleotide linked to a solid phase of b) and / or said polynucleotide building block of c) comprises one TALE DNA binding repeat polynucleotide module and a fragment of building block that encodes at least a half TALE DNA binding repeat module.

8. The method according to any one of claims 1 to 7 wherein at least one said polynucleotide building block of a) and / or said polynucleotide building block of c) further comprises at least one cleavage site for a type IIS restriction enzyme C located outward compared to restriction enzymes A and / or B cleavage sites and / or wherein at least one polynucleotide linked to a solid phase of b) further comprises at least one cleavage site for a restriction enzyme C located outward compared to restriction enzymes A cleavage site.

9. The method according to any one of claims 1 to 7 wherein the last polynucleotide of b) used comprises a single cleavage site for a restriction enzyme C placed on the side of the polynucleotide linked to a solid phase, located outward compared to restriction enzyme A cleavage site, wherein said cleavage with restriction enzyme C allows to unlink said polynucleotide from the solid phase.

10. The method according to any one of claims 8 and 9 further comprising the step of unlinking the polynucleotide comprising an array of polynucleotide modules by cutting it with restriction enzyme C.

11. The method according to any one of claims 8 to 10 further comprising the steps of:
- unlinking the polynucleotide comprising an array of polynucleotide modules of step g) by cutting it with restriction enzyme C;
- subcloning said polynucleotide comprising an array of polynucleotide modules into a plasmidic vector.

12. The method according to claim 11 further comprising the step of subcloning said final polynucleotide comprising an array of polynucleotide modules from a plasmidic vector into another plasmidic vector by cutting it with restriction enzymes A and B.

13. The method according to any one of claims 2 to 9 further comprising the steps of:
- unlinking said final polynucleotide comprising an array of polynucleotide modules of step g) by cutting it with restriction enzymes A and / or B;
- subcloning said final polynucleotide comprising an array of polynucleotide modules into a plasmidic vector.

14. The method according to to any one of claims 1 to 13 wherein said restriction enzymes A and B are BbvI and SfaNI.

15. The method according to any one of claims 8 to 14 wherein said restriction enzyme C is Sfil.

16. The method according to any one of claims 1 to 15 wherein said at least one polynucleotide building blocks of a) and / or said polynucleotides of b) linked to a solid phase and / or said polynucleotide building block of c) are part of a collection encoding polypeptidic repeated modules with Repeat Variable Dipeptide regions (RVDs) comprising a pair of amino acids responsible for recognizing one nucleotide selected from the group consisting of HD for recognizing C, NG for recognizing T, NI for recognizing A, NN for recognizing G or A, NS for recognizing A, C, G or T, HG for recognizing T, IG for recognizing T, NK for recognizing G, HA for recognizing C, ND for recognizing C, HI for recognizing C, HN for recognizing G, NA for recognizing G, SN for recognizing G or A and YG for recognizing T.

17. A method of conducting a high throughput custom-designed platform of chimeric protein derived from a TALE comprising:
a) Receiving a DNA target sequence comprising "n" nucleotides, which a chimeric protein derived from a TALE has to process;
b) Generating and assembling polynucleotide repeated modules, each with RVDs comprising a pair of amino acids for recognizing each one of the "n" nucleotides of said DNA target sequence according to the method of any one of claims 1 to 16, thus releasing a TALE binding domain able to recognize said DNA target sequence;
c) Fuse said DNA binding domain to a protein domain able to process said DNA target sequence.

## Patentansprüche

1. Verfahren zum Erzeugen und zum Zusammenfügen von Polynucleotiden, die Arrays von wenigstens zwei Transkriptionsaktivator-artiger Effektor (TALE)-DNA-Bindungs-Repeat-Polynucleotid-Modulen, von denen jedes für 30 bis 42 Aminosäuren, vorzugsweise für 33 bis 35 Aminosäuren, kodiert, umfassen, umfassend die Schritte:
a) Erzeugen von "n" Polynucleotid-Baublöcken, jeweils umfassend wenigstens:
- ein TALE-DNA-Bindungs-Repeat-Polynucleotid-Modul;
- eine einzelne Spaltungsstelle für ein erstes Typ-IIS-Restriktionsenzym A, die sich auf einer Seite des Polynucleotid-Moduls befindet;
- eine einzelne Spaltungsstelle für ein zweites Typ IIS-Restriktionsenzym B, die sich auf der anderen Seite des Polynucleotid-Moduls befindet;
- wobei A und B kompatible kohäsive Enden produzieren können;
- wobei eine Spaltung jedes der Polynucleotid-Baublöcke mit Restriktionsenzym A zu einem Polynucleotid führt, das ein Polynucleotid-Modul umfasst, das auf einer Seite von einem kohäsiven Ende flankiert ist, welches mit einem Polynucleotid-Baublock, der durch Restiktionsenzym B gespalten wurde, neu ligiert werden kann, ohne dass eine Sequenz wieder hergestellt wird, die durch Restriktionsenzym A und/oder B spaltbar ist;
- wobei eine Spaltung jedes der Polynucleotid-Baublöcke mit Restriktionsenzym B zu einem Polynucleotid führt, das ein Polynucleotid-Modul umfasst, welches auf einer Seite von einem kohäsiven Ende flankiert wird, welches mit einem Polynucleotid-Baublock, der durch Restriktionsenzym A gespalten wurde, neu ligiert werden kann, ohne dass eine Sequenz wieder hergestellt wird, die durch Restriktionsenzym A und/oder B spaltbar ist;
b) Generieren von "n" Polynucleotiden, die an eine feste Phase gebunden sind durch: b1) Binden jedes der Polynucleotid-Baublöcke, die gemäß Schritt a) erzeugt wurden, an eine feste Phase derart, dass die Spaltungsstelle für das erste Restriktionsenzym A sich auf der Seite des Polynucleotid-Moduls befindet, die an die feste Phase gebunden wird, und b2) Spalten des Polynucleotid-Baublocks mit Restriktionsenzym B;
c) Erzeugen eines C-terminalen Polynucleotid-Baublocks, umfassend wenigstens:
- ein Polynucleotid-Modul;
- eine einzelne Spaltungsstelle für ein erstes Restriktionsenzym A, die sich auf einer Seite des Polynucleotid-Moduls befindet;
- eine einzelne Spaltungsstelle für ein zweites Restriktionsenzym B, die sich auf der anderen Seite des Polynucleotid-Moduls befindet;
wobei eine Spaltung des C-terminalen Polynucleotid-Baublocks mit Restriktionsenzym A zu einem Polynucleotid führt, das ein Polynucleotid-Modul umfasst, das auf einer Seite von einem kohäsiven Ende flankiert wird, welches mit einem Polynucleotid-Baublock von Schritt a), der durch Restriktionsenzym B gespalten wurde, neu ligiert werden kann, ohne dass eine Sequenz wieder hergestellt wird, die durch Restriktionsenzym A spaltbar ist, und
wobei eine Spaltung des C-terminalen Polynucleotid-Baublocks mit Restriktionsenzym B zu einem Polynucleotid führt, das ein Polynucleotid-Modul umfasst, das auf einer Seite von einem kohäsiven Ende flankiert wird, welches nicht mit einem Polynucleotid-Baublock von Schritt a), der durch Restriktionsenzym A und/oder B gespalten wurde, neu ligiert werden kann;
d) Schneiden des einen C-terminalen Polynucleotid-Baublocks von c) mit Restriktionsenzym A;
e) Ligieren des resultierenden C-terminalen Polynucleotid-Moduls mit dem freien Ende eines Polynucleotids von b), das an einer festen Phase immobilisiert ist, wodurch ein neues immobilisiertes Polynucleotid produziert wird, das ein zusätzliches Polynucleotid-Modul umfasst;
f) Schneiden des resultierenden neuen immobilisierten Polynucleotids mit Restriktionsenzym A, wodurch ein neues Polynucleotid produziert ist, das ein freies Ende hat, das mit den kohäsiven Enden kompatibel ist, welche aus einer Spaltung eines Polynucleotid-Baublocks von Schritt a) mit Restriktionsenzym B resultieren;
g) Ligieren des neuen Polynucleotids mit dem freien Ende eines Polynucleotids von b), das an einer festen Phase immobilisiert ist, wodurch ein neues immobilisiertes Polynucleotid produziert wird, das ein zusätzliches Polynucleotid-Modul umfasst.

2. Verfahren nach Anspruch 1, wobei Schritte f) und g) N-mal wiederholt werden, um ein immobilisiertes Polynucleotid zu produzieren, das einen Array von n TALE-DNA-Bindungs-Repeat-Polynucleotid-Modulen hat, wobei n = N+3.

3. Verfahren nach Anspruch 2, wobei das immobilisierte Polynucleotid zwischen 8 und 20 TALE-DNA-Bindungs-Repeat-Polynucleotid-Module hat.

4. Verfahren nach einem der Ansprüche 1 bis 3, wobei Schritt g) durch die folgenden Schritte ersetzt wird:
g') Schneiden des wenigstens einen Polynucleotid-Baublocks von a) mit Restriktionsenzym A;
h) Ligieren des resultierenden Polynucleotid-Moduls mit dem freien Ende eines Polynucleotids von b), das an einer festen Phase immobilisiert ist, wodurch ein neues immobilisiertes Polynucleotid produziert wird, das ein zusätzliches Polynucleotid-Modul umfasst;
i) Schneiden des resultierenden neuen immobilisierten Polynucleotid mit Restriktionsenzym B, wodurch ein neues immobilisiertes Polynucleotid produziert wird, das ein freies Ende hat, das mit den kohäsiven Enden, die aus Spaltung mit Restriktionsenzym A resultieren, kompatibel ist;
j) Ligieren an das neue produzierte immobilisierte Polynucleotid das neue Polynucleotid, das aus Schritt f) resultiert, wodurch ein neues immobilisiertes Polynucleotid produziert wird, das "x" zusätzliche Polynucleotid-Module umfasst, wobei "x" gleich der Anzahl von Polynucleotid-Modulen, die in dem neuen Polynucleotid, das aus Schritt f) resultiert, vorliegen, ist.

5. Verfahren nach Anspruch 4, das außerdem die folgenden Schritte umfasst:
k) Schneiden des resultierenden neuen immobilisierten Polynucleotids von Schritt j) mit Restriktionsenzym A, wodurch ein neues Polynucleotid produziert wird, das ein freies Ende hat, das mit den kohäsiven Enden, die aus Spaltung eines Polynucleotid-Baublocks von Schritt a) mit Restriktionsenzym B resultieren, kompatibel ist;
I) Ligieren des neuen Polynucleotids mit dem freien Ende eines Polynucleotids von b), das an einer festen Phase immobilisiert ist, wodurch ein neues immobilisiertes Polynucleotid produziert wird, das ein zusätzliches Polynucleotid-Modul umfasst.

6. Verfahren nach einem der Ansprüche 1 bis 5, wobei wenigstens ein Polynucleotid-Baublock von a) und/oder wenigstens ein Polynucleotid, das an eine feste Phase gebunden ist, von b) und/oder der Polynucleotid-Baublock von c) ein Vor-Zusammenfügen von mehr als einem TALE-DNA-Bindungs-Repeat-Polynucleotid-Modul umfassen.

7. Verfahren nach einem der Ansprüche 1 bis 6, wobei wenigstens ein Polynucleotid-Baublock von a) und/oder wenigstens ein Polynucleotid, das an eine feste Phase gebunden ist, von b) und/oder der Polynucleotid-Baublock von c) ein TALE-DNA-Bindungs-Repeat-Polynucleotid-Modul und ein Baublockfragment, das wenigstens ein halbes TALE-DNA-Bindungs-Repeat-Modul kodiert, umfassen.

8. Verfahren nach einem der Ansprüche 1 bis 7, wobei wenigstens ein Polynucleotid-Baublock von a) und/oder der Polynucleotid-Baublock von c) außerdem wenigstens eine Spaltungsstelle für ein Typ-IIS-Restriktionsenzym A umfassen, die sich im Vergleich zu den Spaltungsstellen für Enzym A und/oder B außen befindet, und/oder wobei wenigstens ein Polynucleotid, das an eine feste Phase gebunden ist, von b) außerdem wenigstens eine Spaltungsstelle für ein Restriktionsenzym C umfasst, die sich im Vergleich zu der Spaltungsstelle für Restriktionsenzym A außen befindet.

9. Verfahren nach einem der Ansprüche 1 bis 7, wobei das letzte Polynucleotid von b), das verwendet wird, eine einzelne Spaltungsstelle für ein Restriktionsenzym C umfasst, die sich auf der Seite des Polynucleotids, die an eine feste Phase gebunden ist, befindet, die sich im Vergleich zu der Spaltungsstelle für Restriktionsenzym A außen befindet, wobei die Spaltung mit Restriktionsenzym C die Abkoppelung des Polynucleotids von der festen Phase erlaubt.

10. Verfahren nach einem der Ansprüche 8 und 9, das außerdem den Schritt des Abkoppelns des Polynucleotids, das einen Array von Polynucleotid-Modulen umfasst, durch Schneiden desselben mit Restriktionsenzym C umfasst.

11. Verfahren nach einem der Ansprüche 8 bis 10, das außerdem die folgenden Schritte umfasst:
- Abkoppeln des Polynucleotids, das einen Array von Polynucleotid-Modulen umfasst, von Schritt g) durch Schneiden mit Restriktionsenzym C;
- Subklonieren des Polynucleotids, das einen Array von Polynucleotid-Modulen umfasst, in einen Plasmidvektor.

12. Verfahren nach Anspruch 11, das außerdem den Schritt des Subklonierens des endgültigen Polynucleotids, das einen Array von Polynucleotid-Modulen umfasst, aus einem Plasmidvektor in einen anderen Plasmidvektor durch Schneiden desselben mit Restriktionsenzymen A und B umfasst.

13. Verfahren nach einem der Ansprüche 2 bis 9, das außerdem die folgenden Schritte umfasst:
- Abkoppeln des endgültigen Polynucleotids, das einen Array von Polynucleotid-Modulen umfasst, von Schritt g) durch Schneiden mit Restriktionsenzymen A und/oder B;
- Subklonieren des fertigen Polynucleotids, das einen Array von Polynucleotid-Modulen umfasst, in einen Plasmidvektor.

14. Verfahren nach einem der Ansprüche 1 bis 13, wobei die Restriktionsenzyme A und B Bbvl und SfaNI sind.

15. Verfahren nach einem der Ansprüche 8 bis 14, wobei das Restriktionsenzym C Sfil ist.

16. Verfahren nach einem der Ansprüche 1 bis 15, wobei der wenigstens eine Polynucleotid-Baublock von a) und/oder die Polynucleotide von b), die an eine feste Phase gebunden sind, und/oder der Polynucleotid-Baublock von c) Teil einer Sammlung sind, die polypeptidische Repeat-Module mit Repeat-Variable-Dipeptide-Regionen (RVDs) kodiert, welche ein Paar von Aminosäuren umfassen, die für die Erkennung eines Nucleotids, ausgewählt aus der Gruppe, bestehend aus HD zur Erkennung von C, NG zur Erkennung von T, NI zur Erkennung von A, NN zur Erkennung von G oder A, NS zur Erkennung von A, C, G oder T, HG zur Erkennung von T, IG zur Erkennung von T, NK zur Erkennung von G, HA zur Erkennung von C, ND zur Erkennung von C, HI zur Erkennung von C, HN zur Erkennung von G, NA zur Erkennung von G, SN zur Erkennung von G oder A und YG zur Erkennung von T, verantwortlich sind.

17. Verfahren zum Betreiben einer maßgeschneiderten Hochdurchsatz-Plattform von einem von TALE abgeleiteten chimären Protein, umfassend:
a) Aufnehmen einer DNA-Targetsequenz, die "n" Nucleotide umfasst, welche ein chimäres Protein, das von einem TALE abgeleitet ist, zu prozessieren hat;
b) Erzeugen und Zusammenfügen von Polynucleotid-Repeat-Modulen, jedes mit RVD, die ein Paar von Aminosäuren zur Erkennung jedes einzelnen der "n" Nucleotide der DNA-Targetsequenz umfassen, nach dem Verfahren nach einem der Ansprüche 1 bis 16, wobei eine TALE-Bindungsdomäne freigesetzt wird, die in der Lage ist, die DNA-Targetsequenz zu erkennen;
c) Fusionieren der DNA-Bindungsdomäne mit einer Proteindomäne, die in der Lage ist, die DNA-Targetsequenz zu prozessieren.

## Revendications

1. Procédé de génération et d'assemblage de polynucléotides comprenant des matrices constituées d'au moins deux modules polynucléotidiques à répétitions de liaison à l'ADN d'effecteurs de type activateurs transcriptionnels (TALE) qui codent chacun pour 30 à 42 acides aminés, de préférence pour 33 à 35 acides aminés, comprenant les étapes suivantes :
a) génération de "n" éléments polynucléotidiques constitutifs, comprenant chacun au moins :
- un module polynucléotidique à répétitions de liaison à l'ADN TALE ;
- un site de clivage unique pour une première enzyme de restriction A de type IIS, placé d'un côté du module polynucléotidique ;
- un site de clivage unique pour une seconde enzyme de restriction B de type IIS, placé de l'autre côté du module polynucléotidique ;
- où A et B peuvent générer des extrémités cohésives compatibles ;
- où le clivage de chacun desdits éléments polynucléotidiques constitutifs avec l'enzyme de restriction A a pour résultat un polynucléotide comprenant un module poly-nucléotidique flanqué d'un côté par une extrémité cohésive qui peut être re-ligaturée avec un élément polynucléotidique constitutif clivé par l'enzyme de restriction B sans restauration d'une séquence clivable par l'enzyme de restriction A et/ou B ;
- où le clivage de chacun desdits éléments polynucléotidiques constitutifs avec l'enzyme de restriction B a pour résultat un polynucléotide comprenant un module poly-nucléotidique flanqué d'un côté par une extrémité cohésive qui peut être re-ligaturée avec un élément polynucléotidique constitutif clivé par l'enzyme de restriction A sans restauration d'une séquence clivable par l'enzyme de restriction A et/ou B ;
b) génération de "n" polynucléotides liés à une phase solide par : b1) liaison de chacun des éléments polynucléotidiques constitutifs générés selon l'étape a) à une phase solide de façon que le site de clivage pour la première enzyme de restriction A soit placé du côté du module polynucléotidique qui est lié à la phase solide et b2) clivage dudit élément polynucléotidique constitutif avec l'enzyme de restriction B ;
c) génération d'un élément polynucléotidique constitutif C-terminal comprenant au moins :
- un module polynucléotidique ;
- un site de clivage unique pour une première enzyme de restriction A, placé d'un côté du module polynucléotidique ;
- un site de clivage unique pour une seconde enzyme de restriction B, placé de l'autre côté du module polynucléotidique ;
dans lequel le clivage dudit élément polynucléotidique constitutif C-terminal avec l'enzyme de restriction A a pour résultat un polynucléotide comprenant un module polynucléotidique flanqué d'un côté par une extrémité cohésive qui peut être re-ligaturée avec un élément polynucléotidique constitutif de l'étape a) clivé par l'enzyme de restriction B sans restauration d'une séquence clivable par l'enzyme de restriction A ; et
dans lequel le clivage dudit élément polynucléotidique constitutif C-terminal avec l'enzyme de restriction B a pour résultat un polynucléotide comprenant un module polynucléotidique flanqué d'un côté par une extrémité cohésive qui ne peut pas être re-ligaturée avec un élément polynucléotidique constitutif de l'étape a) clivé par l'enzyme de restriction A et/ou B ;
d) coupure dudit élément polynucléotidique constitutif C-terminal de l'étape c) avec l'enzyme de restriction A ;
e) ligature du module polynucléotidique C-terminal résultant avec l'extrémité libre d'un polynucléotide de l'étape b) immobilisé sur une phase solide, pour produire ainsi un nouveau polynucléotide immobilisé comprenant un module polynucléotidique supplémentaire ;
f) coupure du nouveau polynucléotide immobilisé résultant avec l'enzyme de restriction A, pour produire ainsi un nouveau polynucléotide ayant une extrémité libre compatible avec les extrémités cohésives résultant du clivage d'un élément polynucléotidique constitutif de l'étape a) avec l'enzyme de restriction B ;
g) ligature du nouveau polynucléotide avec l'extrémité libre d'un polynucléotide de l'étape b) immobilisé sur une phase solide, pour produire ainsi un nouveau polynucléotide immobilisé comprenant un module polynucléotidique supplémentaire.

2. Procédé selon la revendication 1, dans lequel les étapes f) et g) sont répétées N fois pour produire un polynucléotide immobilisé comprenant une matrice de n modules polynucléotidiques à répétitions de liaison à l'ADN TALE où n = N + 3.

3. Procédé selon la revendication 2, dans lequel ledit polynucléotide immobilisé comprend entre 8 et 20 modules polynucléotidiques à répétitions de liaison à l'ADN TALE.

4. Procédé selon l'une quelconque des revendications 1 à 3, dans lequel l'étape g) est remplacée par les étapes suivantes :
g') coupure dudit au moins élément polynucléotidique constitutif de l'étape a) avec l'enzyme de restriction A ;
h) ligature du module polynucléotidique résultant avec l'extrémité libre d'un polynucléotide de l'étape b) immobilisé sur une phase solide, pour produire ainsi un nouveau polynucléotide immobilisé comprenant un module polynucléotidique supplémentaire ;
i) coupure du nouveau polynucléotide immobilisé résultant avec l'enzyme de restriction B, pour produire ainsi un nouveau polynucléotide immobilisé ayant une extrémité libre compatible avec les extrémités cohésives résultant du clivage avec l'enzyme de restriction A ;
j) ligature sur le nouveau polynucléotide immobilisé produit dudit nouveau polynucléotide résultant de l'étape f) pour produire ainsi un nouveau polynucléotide immobilisé comprenant "x" modules polynucléotidiques supplémentaires où "x" est égal au nombre de modules polynucléotidiques présents dans ledit nouveau polynucléotide résultant de l'étape f).

5. Procédé selon la revendication 4, comprenant en outre les étapes de :
k) coupure du nouveau polynucléotide immobilisé résultant de l'étape j) avec l'enzyme de restriction A, pour produire ainsi un nouveau polynucléotide ayant une extrémité libre compatible avec les extrémités cohésives résultant du clivage d'un élément polynucléotidique constitutif généré à l'étape a) avec l'enzyme de restriction B ;
l) ligature du nouveau polynucléotide avec l'extrémité libre d'un polynucléotide de b) immobilisé sur une phase solide, pour produire ainsi un nouveau polynucléotide immobilisé comprenant un module polynucléotidique supplémentaire.

6. Procédé selon l'une quelconque des revendications 1 à 5, dans lequel au moins un élément poly-nucléotidique constitutif de a) et/ou au moins un polynucléotide lié à une phase solide de b) et/ou ledit élément polynucléotidique constitutif de c) comprennent un pré-assemblage constitué de plus d'un module poly-nucléotidique à répétitions de liaison à l'ADN TALE.

7. Procédé selon l'une quelconque des revendications 1 à 6, dans lequel au moins un élément poly-nucléotidique constitutif de a) et/ou au moins un polynucléotide lié à une phase solide de b) et/ou ledit élément polynucléotidique constitutif de c) comprennent un module polynucléotidique à répétitions de liaison à l'ADN TALE et un fragment d'élément constitutif qui code pour au moins un demi-module à répétitions de liaison à l'ADN TALE.

8. Procédé selon l'une quelconque des revendications 1 à 7, dans lequel au moins un élément poly-nucléotidique constitutif de a) et/ou élément polynucléotidique constitutif de c) comprend en outre au moins un site de clivage pour une enzyme de restriction C de type IIS situé vers l'extérieur comparativement aux sites de clivage des enzymes de restriction A et/ou B et/ou dans lequel au moins un polynucléotide lié à une phase solide de b) comprend en outre au moins un site de clivage pour une enzyme de restriction C situé vers l'extérieur comparativement au site de clivage de l'enzyme de restriction A.

9. Procédé selon l'une quelconque des revendications 1 à 7, dans lequel le dernier polynucléotide de b) utilisé comprend un seul site de clivage pour une enzyme de restriction C placé du côté du polynucléotide lié à une phase solide, situé vers l'extérieur comparativement au site de clivage de l'enzyme de restriction A, dans lequel ledit clivage avec l'enzyme de restriction C permet de détacher ledit polynucléotide de la phase solide.

10. Procédé selon l'une quelconque des revendications 8 et 9, comprenant en outre l'étape de séparation du polynucléotide comprenant une matrice de modules polynucléotidiques par coupure à l'aide de l'enzyme de restriction C.

11. Procédé selon l'une quelconque des revendications 8 à 10, comprenant en outre les étapes de :
- séparation du polynucléotide comprenant une matrice de modules polynucléotidiques de l'étape g) par coupure à l'aide de l'enzyme de restriction C ;
- sous-clonage dudit polynucléotide comprenant une matrice de modules polynucléotidiques dans un vecteur plasmidique.

12. Procédé selon la revendication 11, comprenant en outre l'étape de sous-clonage dudit polynucléotide final comprenant une matrice de modules polynucléotidiques d'un vecteur plasmidique dans un autre vecteur plasmidique par coupure à l'aide des enzymes de restriction A et B.

13. Procédé selon l'une quelconque des revendications 2 à 9, comprenant en outre les étapes de :
- séparation dudit polynucléotide final comprenant une matrice de modules polynucléotidiques de l'étape g) par coupure à l'aide des enzymes de restriction A et/ou B ;
- sous-clonage dudit polynucléotide final comprenant une matrice de modules polynucléotidiques dans un vecteur plasmidique.

14. Procédé selon l'une quelconque des revendications 1 à 13, dans lequel lesdites enzymes de restriction A et B sont BbvI et SfaNI.

15. Procédé selon l'une quelconque des revendications 8 à 14, dans lequel ladite enzyme de restriction C est SfiI.

16. Procédé selon l'une quelconque des revendications 1 à 15, dans lequel ledit au moins élément polynucléotidique constitutif de a) et/ou lesdits polynucléotides de b) liés à une phase solide et/ou ledit élément polynucléotidique constitutif de c) font partie d'une collection codant pour des modules polypeptidiques répétés comportant des régions dipeptidiques variables à répétition (RVD) comprenant une paire d'acides aminés chargée de reconnaître un nucléotide choisie dans le groupe constitué par HD pour reconnaître C, NG pour reconnaître T, NI pour reconnaître A, NN pour reconnaître G ou A, NS pour reconnaître A, C, G ou T, HG pour reconnaître T, IG pour reconnaître T, NK pour reconnaître G, HA pour reconnaître C, ND pour reconnaître C, HI pour reconnaître C, HN pour reconnaître G, NA pour reconnaître G, SN pour reconnaître G ou A et YG pour reconnaître T.

17. Procédé de mise en oeuvre d'une plateforme haut débit sur mesure de protéines chimères dérivées d'un effecteur TALE comprenant :
a) la réception d'une séquence d'ADN cible comprenant "n" nucléotides destinée à être traitée par une protéine chimère dérivée d'un effecteur TALE ;
b) la génération et l'assemblage de modules polynucléotidiques répétés, comportant chacun des RVD comprenant une paire d'acides aminés pour la reconnaissance de chacun des "n" nucléotides de ladite séquence d'ADN cible par le procédé selon l'une quelconque des revendications 1 à 16, pour libérer ainsi un domaine de liaison TALE capable de reconnaître ladite séquence d'ADN cible ; et
c) la fusion du domaine de liaison à l'ADN à un domaine protéique capable traiter ladite séquence d'ADN cible.


### SEQUENCE LISTING

<110> CELLECTIS
   VALTON JULIEN
   JUILLERAT ALEXANDRE
   DUCHATEAU PHILIPPE
<120> HIGH THROUGHPUT METHOD FOR ASSEMBLY AND CLONING POLYNUCLEOTIDES COMPRISING HIGHLY SIMILAR POLYNUCLEOTIDIC MODULES
<130> 384519WO
<160> 39
<170> PatentIn version 3.5
<210> 1
<211> 17
   <212> DNA
   <213> Artificial sequence
<220>
   <223> CAPT1.3 target
<400> 1
   tccgggaacc cagagct 17
<210> 2
<211> 17
   <212> DNA
   <213> Artificial sequence
<220>
   <223> CAPT1.4 target
<400> 2
   tctgtgggct cgggtct 17
<210> 3
<211> 55
   <212> DNA
   <213> Artificial sequence
<220>
   <223> Full SADE2.3 target
<400> 3
   tcataaaaat ttaaggtagt gcagagagct aacatgaaac cttaaatttt tatga 55
<210> 4
<211> 412
   <212> PRT
   <213> Artificial sequence
<220>
   <223> fem-3 protein
<400> 4
   Ser Asn Asn Val Leu Pro Thr Trp Ser Leu Asp Ser Asn Gly Glu Met
   1               5                   10                  15
   Arg Ser Arg Leu Ser Leu Ser Glu Val Leu Asp Ser Gly Asp Leu Met
               20                  25                  30
   Lys Phe Ala Val Asp Lys Thr Gly Cys Gln Phe Leu Glu Lys Ala Val
           35                  40                  45
   Lys Gly Ser Leu Thr Ser Tyr Gln Lys Phe Gln Leu Phe Glu Gln Val
       50                  55                  60
   Ile Gly Arg Lys Asp Asp Phe Leu Lys Leu Ser Thr Asn Ile Phe Gly
   65                  70                  75                  80
   Asn Tyr Leu Val Gln Ser Val Ile Gly Ile Ser Leu Ala Thr Asn Asp
                   85                  90                  95
   Asp Gly Tyr Thr Lys Arg Gln Glu Lys Leu Lys Asn Phe Ile Ser Ser
               100                 105                 110
   Gln Met Thr Asp Met Cys Leu Asp Lys Phe Ala Cys Arg Val Ile Gln
           115                 120                 125
   Ser Ser Leu Gln Asn Met Asp Leu Ser Leu Ala Cys Lys Leu Val Gln
       130                 135                 140
   Ala Leu Pro Arg Asp Ala Arg Leu Ile Ala Ile Cys Val Asp Gln Asn
   145                 150                 155                 160
   Ala Asn His Val Ile Gln Lys Val Val Ala Val Ile Pro Leu Lys Asn
                   165                 170                 175
   Trp Glu Phe Ile Val Asp Phe Val Ala Thr Pro Glu His Leu Arg Gln
               180                 185                 190
   Ile Cys Ser Asp Lys Tyr Gly Cys Arg Val Val Gln Thr Ile Ile Glu
           195                 200                 205
   Lys Leu Thr Ala Asp Ser Met Asn Val Asp Leu Thr Ser Ala Ala Gln
       210                 215                 220
   Asn Leu Arg Glu Arg Ala Leu Gln Arg Leu Met Thr Ser Val Thr Asn
   225                 230                 235                 240
   Arg Cys Gln Glu Leu Ala Thr Asn Glu Tyr Ala Asn Tyr Ile Ile Gln
   245 250 255
   His Ile Val Ser Asn Asp Asp Leu Ala Val Tyr Arg Glu Cys Ile Ile
   260 265 270
   Glu Lys Cys Leu Met Arg Asn Leu Leu Ser Leu Ser Gln Glu Lys Phe
   275 280 285
   Ala Ser His Val Val Glu Lys Ala Phe Leu His Ala Pro Leu Glu Leu
   290 295 300
   Leu Ala Glu Met Met Asp Glu Ile Phe Asp Gly Tyr Ile Pro His Pro
   305 310 315 320
   Asp Thr Gly Lys Asp Ala Leu Asp Ile Met Met Phe His Gln Phe Gly
   325 330 335
   Asn Tyr Val Val Gln Cys Met Leu Thr Ile Cys Cys Asp Ala Val Ser
   340 345 350
   Gly Arg Arg Gln Thr Lys Glu Gly Gly Tyr Asp His Ala Ile Ser Phe
   355 360 365
   Gln Asp Trp Leu Lys Lys Leu His Ser Arg Val Thr Lys Glu Arg His
   370 375 380
   Arg Leu Ser Arg Phe Ser Ser Gly Lys Lys Met Ile Glu Thr Leu Ala
   385 390 395 400
   Asn Leu Arg Ser Thr His Pro Ile Tyr Glu Leu Gln
   405 410
<210> 5
<211> 201
   <212> PRT
   <213> Artificial sequence
<220>
   <223> aRep
<400> 5
   Met Arg Gly Ser His His His His His His Thr Asp Pro Glu Lys Val
   1               5                   10                  15
   Glu Met Tyr Ile Lys Asn Leu Gln Asp Asp Ser Tyr Tyr Val Arg Arg
               20                  25                  30
   Ala Ala Ala Tyr Ala Leu Gly Lys Ile Gly Asp Glu Arg Ala Val Glu
           35                  40                  45
   Pro Leu Ile Lys Ala Leu Lys Asp Glu Asp Ala Trp Val Arg Arg Ala
       50                  55                  60
   Ala Ala Asp Ala Leu Gly Gln Ile Gly Asp Glu Arg Ala Val Glu Pro
   65                  70                  75                  80
   Leu Ile Lys Ala Leu Lys Asp Glu Asp Gly Trp Val Arg Gln Ser Ala
                   85                  90                  95
   Ala Val Ala Leu Gly Gln Ile Gly Asp Glu Arg Ala Val Glu Pro Leu
               100                 105                 110
   Ile Lys Ala Leu Lys Asp Glu Asp Trp Phe Val Arg Ile Ala Ala Ala
           115                 120                 125
   Phe Ala Leu Gly Glu Ile Gly Asp Glu Arg Ala Val Glu Pro Leu Ile
       130                 135                 140
   Lys Ala Leu Lys Asp Glu Asp Gly Trp Val Arg Gln Ser Ala Ala Asp
   145                 150                 155                 160
   Ala Leu Gly Glu Ile Gly Gly Glu Arg Val Arg Ala Ala Met Glu Lys
                   165                 170                 175
   Leu Ala Glu Thr Gly Thr Gly Phe Ala Arg Lys Val Ala Val Asn Tyr
               180                 185                 190
   Leu Glu Thr His Lys Ser Leu Ile Ser
           195                 200
<210> 6
<211> 102
   <212> DNA
   <213> Artificial sequence
<220>
   <223> TALE monorepeat HD
<400> 6
   ttgaccccgg agcaggtggt ggccatcgcc agccacgatg gcggcaagca ggcgctggag     60
   acggtccagc ggctgttgcc ggtgctgtgc caggcccacg gc                       102
<210> 7
<211> 102
   <212> DNA
   <213> Artificial sequence
<220>
   <223> TALE monorepeat NI
<400> 7
   ttgaccccgg agcaggtggt ggccatcgcc agcaatattg gtggcaagca ggcgctggag     60
   acggtgcagg cgctgttgcc ggtgctgtgc caggcccacg gc                       102
<210> 8
<211> 102
   <212> DNA
   <213> Artificial sequence
<220>
   <223> TALE monorepeat NN
<400> 8
   ttgacccccc agcaggtggt ggccatcgcc agcaataatg gtggcaagca ggcgctggag     60
   acggtccagc ggctgttgcc ggtgctgtgc caggcccacg gc                       102
<210> 9
<211> 102
   <212> DNA
   <213> Artificial sequence
<220>
   <223> TALE monorepeat HG
<400> 9
   ttgaccccgg agcaggtggt ggccatcgcc agccacggcg gcggcaagca ggcgctggag     60
   acggtccagc ggctgttgcc ggtgctgtgc caggcccacg gc                       102
<210> 10
<211> 102
   <212> DNA
   <213> Artificial sequence
<220>
   <223> TALE monorepeat NK
<400> 10
   ttgaccccgg agcaggtggt ggccatcgcc agcaataagg gcggcaagca ggcgctggag      60
   acggtccagc ggctgttgcc ggtgctgtgc caggcccacg gc                        102
<210> 11
<211> 102
   <212> DNA
   <213> Artificial sequence
<220>
   <223> TALE monorepeat NG*
<400> 11
   ttgacccccc agcaggtggt ggccatcgcc agcaatggcg gtggcaagca ggcgctggag      60
   acggtccagc ggctgttgcc ggtgctgtgc caggcccacg gc                        102
<210> 12
<211> 60
   <212> DNA
   <213> Artificial sequence
<220>
   <223> TALE terminal half repeat NG*
<400> 12
   ttgacccctc agcaggtggt ggccatcgcc agcaatggcg gcggcaggcc ggcgctggag 60
<210> 13
<211> 170
   <212> DNA
   <213> Artificial sequence
<220>
   <223> Polynucleotidic building block encoding TALE monorepeat HD
<400> 13
   ggccggacgg gccgacgtcc gcagcgacat gttgaccccg gagcaggtgg tggccatcgc      60
   cagccacgat ggcggcaagc aggcgctgga gacggtccag cggctgttgc cggtgctgtg     120
   ccaggcccac ggcttgaccc tcgagatgcg gtaccggggc cactggggcc                170
<210> 14
<211> 170
   <212> DNA
   <213> Artificial sequence
<220>
   <223> Polynucleotidic building block encoding TALE monorepeat NI
<400> 14
   ggccggacgg gccgacgtcc gcagcgacat gttgaccccg gagcaggtgg tggccatcgc     60
   cagcaatatt ggtggcaagc aggcgctgga gacggtgcag gcgctgttgc cggtgctgtg    120
   ccaggcccac ggcttgaccc tcgagatgcg gtaccggggc cactggggcc               170
<210> 15
<211> 170
   <212> DNA
   <213> Artificial sequence
<220>
   <223> Polynucleotidic building block encoding TALE monorepeat NN
<400> 15
   ggccggacgg gccgacgtcc gcagcgacat gttgaccccc cagcaggtgg tggccatcgc     60
   cagcaataat ggtggcaagc aggcgctgga gacggtccag cggctgttgc cggtgctgtg    120
   ccaggcccac ggcttgaccc tcgagatgcg gtaccggggc cactggggcc               170
<210> 16
<211> 170
   <212> DNA
   <213> Artificial sequence
<220>
   <223> Polynucleotidic building block encoding TALE monorepeat HG
<400> 16
   ggccggacgg gccgacgtcc gcagcgacat gttgaccccg gagcaggtgg tggccatcgc     60
   cagccacggc ggcggcaagc aggcgctgga gacggtccag cggctgttgc cggtgctgtg    120
   ccaggcccac ggcttgaccc tcgagatgcg gtaccggggc cactggggcc               170
<210> 17
<211> 170
   <212> DNA
   <213> Artificial sequence
<220>
   <223> Polynucleotidic building block encoding TALE monorepeat NK
<400> 17
   ggccggacgg gccgacgtcc gcagcgacat gttgaccccg gagcaggtgg tggccatcgc     60
   cagcaataag ggcggcaagc aggcgctgga gacggtccag cggctgttgc cggtgctgtg    120
   ccaggcccac ggcttgaccc tcgagatgcg gtaccggggc cactggggcc               170
<210> 18
<211> 170
   <212> DNA
   <213> Artificial sequence
<220>
   <223> Polynucleotidic building block encoding TALE monorepeat NG*
<400> 18
   ggccggacgg gccgacgtcc gcagcgacat gttgaccccc cagcaggtgg tggccatcgc    60
   cagcaatggc ggtggcaagc aggcgctgga gacggtccag cggctgttgc cggtgctgtg   120
   ccaggcccac ggcttgaccc tcgagatgcg gtaccggggc cactggggcc              170
<210> 19
<211> 125
   <212> DNA
   <213> Artificial sequence
<220>
   <223> Polynucleotidic building block encoding TALE terminal half repeat NG*
<400> 19
   ggccggacgg gccgacgtcc gcagcgacat gttgacccct cagcaggtgg tggccatcgc    60
   cagcaatggc ggcggcaggc cggcgctgga gagcctcgag atgcggtacc ggggccactg   120
   gggcc                                                               125
<210> 20
<211> 2217
   <212> DNA
   <213> Artificial sequence
<220>
   <223> pCLS7183
<400> 20
   atggccgacc ccattcgttc gcgcacacca agtcctgccc gcgagcttct gcccggaccc      60
   caacccgatg gggttcagcc gactgcagat cgtggggtgt ctccgcctgc cggcggcccc     120
   ctggatggct tgccggctcg gcggacgatg tcccggaccc ggctgccatc tccccctgcc     180
   ccctcacctg cgttctcggc gggcagcttc agtgacctgt tacgtcagtt cgatccgtca     240
   ctttttaata catcgctttt tgattcattg cctcccttcg gcgctcacca tacagaggct     300
   gccacaggcg agtgggatga ggtgcaatcg ggtctgcggg cagccgacgc ccccccaccc     360
   accatgcgcg tggctgtcac tgccgcgcgg cccccgcgcg ccaagccggc gccgcgacga     420
   cgtgctgcgc aaccctccga cgcttcgccg gcggcgcagg tggatctacg cacgctcggc     480
   tacagccagc agcaacagga gaagatcaaa ccgaaggttc gttcgacagt ggcgcagcac     540
   cacgaggcac tggtcggcca cgggtttaca cacgcgcaca tcgttgcgtt aagccaacac     600
   ccggcagcgt tagggaccgt cgctgtcaag tatcaggaca tgatcgcagc gttgccagag     660
   gcgacacacg aagcgatcgt tggcgtcggc aaacagtggt ccggcgcacg cgctctggag     720
   gccttgctca cggtggcggg agagttgaga ggtccaccgt tacagttgga cacaggccaa     780
   cttctcaaga ttgcaaaacg tggcggcgtg accgcagtgg aggcagtgca tgcatggcgc     840
   aatgcactga cgggtgcccc gctcaacttg accggagacg cccgggggat caggtcacgt     900
   gcgtctcgga gcattgttgc ccagttatct cgccctgatc cggcgttggc cgcgttgacc     960
   aacgaccacc tcgtcgcctt ggcctgcctc ggcgggcgtc ctgcgctgga tgcagtgaaa    1020
   aagggattgc cgcacgcgcc ggccttgatc aaaagaacca atcgccgtat tcccgaacgc    1080
   acatcccatc gcgttgccga ccacgcgcaa gtggttcgcg tgctgggttt tttccagtgc    1140
   cactcccacc cagcgcaagc atttgatgac gccatgacgc agttcgggat gagcaggcac    1200
   gggttgttac agctctttcg cagagtgggc gtcaccgaac tcgaagcccg cagtggaacg    1260
   ctccccccag ccagtcagcg ttgggaccgt atcctccagg catcagggat gaaaagggcc    1320
   aaaccgtccc ctacttcaac tcaaacgccg gatcaggcgt ctttgcatgc attcgccgat    1380
   tcgctggagc gtgaccttga tgcgcctagc ccaatgcacg agggagatca gacgcgggca    1440
   agtagccgta aacggtcccg atcggatcgt gctgtcaccg gtccctccgc acagcaatcg    1500
   ttcgaggtgc gcgttcccga acagcgcgat gcgctccatt tgcccctctc ctggagggta    1560
   aaacgcccgc gtaccagtat cgggggcggc ctcccggatc ctatcagccg ttcccagctg    1620
   gtgaagtccg agctggagga gaagaaatcc gagttgaggc acaagctgaa gtacgtgccc    1680
   cacgagtaca tcgagctgat cgagatcgcc cggaacagca cccaggaccg tatcctggag    1740
   atgaaggtga tggagttctt catgaaggtg tacggctaca ggggcaagca cctgggcggc    1800
   tccaggaagc ccgacggcgc catctacacc gtgggctccc ccatcgacta cggcgtgatc    1860
   gtggacacca aggcctactc cggcggctac aacctgccca tcggccaggc cgacgaaatg    1920
   cagaggtacg tggaggagaa ccagaccagg aacaagcaca tcaaccccaa cgagtggtgg    1980
   aaggtgtacc cctccagcgt gaccgagttc aagttcctgt tcgtgtccgg ccacttcaag    2040
   ggcaactaca aggcccagct gaccaggctg aaccacatca ccaactgcaa cggcgccgtg    2100
   ctgtccgtgg aggagctcct gatcggcggc gagatgatca aggccggcac cctgaccctg    2160
   gaggaggtga ggaggaagtt caacaacggc gagatcaact tcgcggccga ctgataa       2217
<210> 21
<211> 125
   <212> DNA
   <213> Artificial sequence
<220>
   <223> TALE repeat module HD SFiI and sfaNI digested
<400> 21
   gggccgacgt ccgcagcgac atggaccccg gagcaggtgg tggccatcgc cagccacgat     60
   ggcggcaagc aggcgctgga gacggtccag cggctgttgc cggtgctgtg ccaggcccac    120
   ggctt                                                                125
<210> 22
<211> 132
   <212> DNA
   <213> Artificial sequence
<220>
   <223> TALE repeat module NI SFiI and sfaNI digested
<400> 22
   gaccccggag caggtggtgg ccatcgccag caatattggc ggcaagcagg cgctggagac     60
   ggtccagcgg ctgttgccgg tgctgtgcca ggcccacggc ttgaccctcg agatgcggta    120
   ccggggccac tg                                                        132
<210> 23
<211> 257
   <212> DNA
   <213> Artificial sequence
<220>
   <223> TALE di repeat module HD-NI
<400> 23
   gggccgacgt ccgcagcgac atggaccccg gagcaggtgg tggccatcgc cagccacgat     60
   ggcggcaagc aggcgctgga gacggtccag cggctgttgc cggtgctgtg ccaggcccac    120
   ggcttgaccc cggagcaggt ggtggccatc gccagcaata ttggcggcaa gcaggcgctg    180
   gagacggtcc agcggctgtt gccggtgctg tgccaggccc acggcttgac cctcgagatg    240
   cggtaccggg gccactg                                                   257
<210> 24
<211> 16
   <212> DNA
   <213> Artificial sequence
<220>
   <223> M13_forward primer
<400> 24
   gtaaaacgac ggccag 16
<210> 25
<211> 17
   <212> DNA
   <213> Artificial sequence
<220>
   <223> M13_reverse primer
<400> 25
   caggaaacag ctatgac 17
<210> 26
<211> 227
   <212> DNA
   <213> Artificial sequence
<220>
   <223> TALE di repeat module HD-NI SFiI and sfaNI digested
<400> 26
   gggccgacgt ccgcagcgac atggaccccg gagcaggtgg tggccatcgc cagccacgat      60
   ggcggcaagc aggcgctgga gacggtccag cggctgttgc cggtgctgtg ccaggcccac     120
   ggcttgaccc cggagcaggt ggtggccatc gccagcaata ttggcggcaa gcaggcgctg     180
   gagacggtcc agcggctgtt gccggtgctg tgccaggccc acggctt                   227
<210> 27
<211> 234
   <212> DNA
   <213> Artificial sequence
<220>
   <223> TALE di repeat module NG-NK SFiI and sfaNI digested
<400> 27
   gaccccggag caggtggtgg ccatcgccag caatggcggc ggcaagcagg cgctggagac      60
   ggtccagcgg ctgttgccgg tgctgtgcca ggcccacggc ttgaccccgg agcaggtggt     120
   ggccatcgcc agcaataagg gcggcaagca ggcgctggag acggtccagc ggctgttgcc     180
   ggtgctgtgc caggcccacg gcttgaccct cgagatgcgg taccggggcc actg           234
<210> 28
<211> 461
   <212> DNA
   <213> Artificial sequence
<220>
   <223> TALE four repeat module HD-NI-NG-NK
<400> 28
   gggccgacgt ccgcagcgac atggaccccg gagcaggtgg tggccatcgc cagccacgat      60
   ggcggcaagc aggcgctgga gacggtccag cggctgttgc cggtgctgtg ccaggcccac     120
   ggcttgaccc cggagcaggt ggtggccatc gccagcaata ttggcggcaa gcaggcgctg     180
   gagacggtcc agcggctgtt gccggtgctg tgccaggccc acggcttgac cccggagcag     240
   gtggtggcca tcgccagcaa tggcggcggc aagcaggcgc tggagacggt ccagcggctg     300
   ttgccggtgc tgtgccaggc ccacggcttg accccggagc aggtggtggc catcgccagc     360
   aataagggcg gcaagcaggc gctggagacg gtccagcggc tgttgccggt gctgtgccag     420
   gcccacggct tgaccctcga gatgcggtac cggggccact g                         461
<210> 29
<211> 22
   <212> DNA
   <213> Artificial sequence
<220>
   <223> TAL_Shuttle_Bio_Forward (biotinylated)
<400> 29
   cccagtcacg acgttgtaaa ac 22
<210> 30
<211> 23
   <212> DNA
   <213> Artificial sequence
<220>
   <223> TAL_Shuttle_Reverse
<400> 30
   cacaggaaac agctatgacc atg 23
<210> 31
<211> 23
   <212> DNA
   <213> Artificial sequence
<220>
   <223> TAL_Shuttle_short_Forward
<400> 31
   cctcacaggc cggacgggcc gac 23
<210> 32
<211> 49
   <212> DNA
   <213> Artificial sequence
<220>
   <223> Full CAPT1.1 target
<400> 32
   tccgggaacc cagagctcac agccacgatc ttagacccga gcccacaga 49
<210> 33
<211> 15
   <212> DNA
   <213> Artificial sequence
<220>
   <223> 15 bp spacer of CAPT1.1 target
<400> 33
   cacagccacg atctt 15
<210> 34
<211> 17
   <212> DNA
   <213> Artificial sequence
<220>
   <223> half SADE 2.3 target
<400> 34
   tcataaaaat ttaaggt 17
<210> 35
<211> 21
   <212> DNA
   <213> Artificial sequence
<220>
   <223> 21 bp spacer of SADE 2.3 target
<400> 35
   agtgcagaga gctaacatga a 21
<210> 36
<211> 52
   <212> DNA
   <213> Artificial sequence
<220>
   <223> AvrBS3 target
<400> 36
   tctataaacc taaccctcta gcatgaaggt acgagagggt taggtttata ga 52
<210> 37
<211> 19
   <212> DNA
   <213> Artificial sequence
<220>
   <223> half AvrBS3 target
<400> 37
   tctataaacc taaccctct 19
<210> 38
<211> 15
   <212> DNA
   <213> Artificial sequence
<220>
   <223> 15 bp spacer of AvrBS3 target
<400> 38
   tagcatgaag gtacg 15
<210> 39
<211> 20
   <212> DNA
   <213> Artificial sequence
<220>
   <223> TAL_Shuttle_Reverse_short primer
<400> 39
   cccggtaccg catctcgagg 20